# EUROPEAN PATENT APPLICATION

(11) **EP 3 769 780 A1**
(43) Date of publication of application: **27.01.2021**
(21) Application number: 20176552.6
(22) Date of filing: 31.10.2015
(51) Int. Cl.: A61K 38/57, C07K 14/81

(54) **C1 ESTERASE INHIBITOR FUSION PROTEINS AND USES THEREOF**

(30) Priority: 31.10.2014 US 201462073657 P
(62) Divisional of application: 15795274.8
(71) Applicant: Shire Human Genetic Therapies, Inc., Lexington MA 02421 (US)
(72) Inventor: NORTON, Angela, Lexington, MA Massachusetts 02421 (US); PAN, Clark, Lexington, MA Massachusetts 02421 (US); ISKENDERIAN, Andrea, Lexington, MA Massachusetts 02421 (US); STRACK-LOGUE, Bettina, Lexington, MA Massachusetts 02421 (US)
(74) Representative: Duffield, Stephen

(57) **Abstract**

The present invention provides, among other things, methods and compositions for treating complement mediated disease, in particular, chronic diseases requiring prophylactic and/or maintenance treatment. In one aspect, C1-INH fusion proteins having longer half-life than native plasma-derived C1-INH are provided. In some embodiments, a method according to the present invention includes administering to an individual who is suffering from or susceptible to a complement-mediated disease, an effective amount of a recombinant C1-INH fusion protein such that at least one symptom or feature of said complement-mediated disease is prevented and/or reduced in intensity, severity, or frequency.

## Description

### CROSS-REFERENCE TO RELATED APPLICATIONS

This application claims priority from U.S. provisional patent application serial number 62/073657, filed October 31, 2014, the disclosure of which is hereby incorporated by reference in its entirety.

### BACKGROUND

C1-inhibitor (C1-INH), also known as C1 esterase inhibitor, is the largest member of the serpin protein superfamily. It is a heavily glycosylated serine proteinase inhibitor having the main function of inhibiting the spontaneous activation of the complement system. C1-INH regulates the complement cascade system, plays a key role in the regulation of the contact (kallikrein-kinin) amplification cascade, and participates in the regulation of the coagulation and fibrinolytic systems. Karnaukhova, E., C1-Esterase Inhibitor: Biological Activities and Therapeutic Applications. J Hematol Thromb Dis, 1: 113 (2013).

Dysfunction and/or deficiency of C1-INH in subjects has been correlated with a variety of autoimmune disease due to the failure of C1-INH to inhibit the activation of the complement system. An example of such a disease is hereditary angioedema (HAE), a rare, but potentially life-threatening disorder characterized by unpredictable and recurrent attacks of inflammation. Symptoms of HAE attacks include swelling of the face, mouth and/or airway that occur spontaneously or arc triggered by mild trauma. Such swelling can also occur in any part of the body. In some cases, HAE is associated with low plasma levels of C1-inhibitor, while in other cases the protein circulates in normal or elevated amounts but it is dysfunctional. In addition to the episodes of inflammation, it also can cause more serious or life threatening indications, such as autoimmune diseases or lupus erythematosus.

CINRYZE®, a human plasma derived C1 esterase inhibitor, has been approved for prophylactic use and treatment of acute attacks of HAE. Berinert® (also a plasma-derived human C1-INH, CSL Behring) is indicated for treatment of acute HAE attack. The supply of human plasma derived C1 esterase inhibitor is tied to the availability of blood and plasma donations. Ruconest® (conestat alfa, Pharming N.V.) a recombinant C1-INH expressed in engineered rabbits is indicated for IV administration for treatment of acute HAE attack. However, because Ruconest® is made in rabbits, its glycosylation profile is different from that of human plasma-derived C1-INH. The result is that Ruconest has an extremely short half-life of about 2.4-2.7 hours. See Ruconest® FDA Label and Prescribing Information.

Therefore, it remains a need in the art for improved C1 esterase inhibitor for the treatment of various C1 esterase mediated indication.

### SUMMARY OF THE INVENTION

The present invention provides, among other things, improved long-acting recombinant C1 esterase inhibitors that can be used to effectively treat various complement-mediated disorders and that can be manufactured in a cost-effective matter.

In particular, the present invention provides C1 esterase inhibitor fusion proteins that exhibit longer half-life than Ruconest®. In some embodiments, the C1 inhibitor fusion proteins of the invention exhibit a half-life comparable to or longer than plasma-derived C1-INH. For example, the present inventors have demonstrated that certain exemplary C1 inhibitor fusion proteins according to the present invention have extended serum half-life of at least 4 days. It is contemplated that the long serum half-life of a recombinant C1 inhibitor leads to superior *in vivo* efficacy and permits a preferable dosing regimen and route of administration. In certain embodiments, the C1 inhibitor fusion proteins of the invention may be administered subcutaneously with less frequency than approved C1 inhibitors, while still achieving desired efficacy (e.g., prophylaxis). Moreover, the C1 inhibitor fusion proteins of the invention can be produced recombinantly in host cells such that the disclosed C1 inhibitor fusion proteins are not dependent on blood supply, do not pose a risk of transmission of infectious agents, and are less expensive to manufacture. Since they are recombinantly produced in host cells, they offer more consistency in production and final product than those products purified from human blood, human blood components (e.g. plasma), or animal milk. Moreover, the C1 inhibitor fusion proteins provided herein are not dependent on animal husbandry considerations, including animal age and/or maturity, milk production, animal illness, etc., all of which may affect both quantity and quality (e.g., glycosylation profile, heterogeneity of expressed protein, etc.) of the rabbit expressed C1-INH. Thus, the present invention provides for the cost effective and reliable manufacturing of recombinant C1 esterase inhibitors, and safer, more effective treatment of HAE and other complement-mediated disorders. Other features, objects, and advantages of the present invention are apparent in the detailed description that follows. It should be understood, however, that the detailed description, while indicating embodiments of the present invention, is given by way of illustration only, not limitation. Various changes and modifications within the scope of the invention will become apparent to those skilled in the art from the detailed description.

In one aspect, the present invention provides a fusion protein comprising a human C1-inhibitor polypeptide and an Fc domain. In some embodiments, the human C1-inhibitor polypeptide comprises an amino acid sequence at least at least 50% (e.g., at least 55%, 60%, 65%, 70%, 75%, 80%, 85%, 90%, 95%, 96%, 97%, 98%, 99%, or 100%) identical to the full length human C1-inhibitor having SEQ ID NO:1.

In some embodiments, the fusion protein comprising a human C1-inhibitor polypeptide and an Fc domain includes an amino acid sequence at least 70% identical to the full length human C1-inhibitor protein SEQ ID NO:1. In some embodiments, the fusion protein comprising a human C1-inhibitor polypeptide and an Fc domain includes an amino acid sequence at least 80% identical to the full length human C1-inhibitor protein SEQ ID NO:1. In some embodiments, the fusion protein comprising a human C1-inhibitor polypeptide and an Fc domain includes an amino acid sequence at least 90% identical to the full length human C1-inhibitor protein SEQ ID NO:1. In some embodiments, the fusion protein comprising a human C1-inhibitor polypeptide and an Fc domain includes an amino acid sequence at least 95% identical to the full length human C1-inhibitor protein SEQ ID NO:1. In some embodiments, the fusion protein comprising a human C1-inhibitor polypeptide and an Fc domain includes an amino acid sequence identical to the full length human C1-inhibitor protein SEQ ID NO:1. In some embodiments, the fusion protein comprising a human C1-inhibitor polypeptide and an Fc domain comprises one or more truncations, deletions, mutations or insertions compared to SEQ ID NO:1.

In some embodiments, the fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:2. In some embodiments, the fusion protein comprising a human C1-inhibitor polypeptide and an Fc domain includes an amino acid sequence at least 70% identical to the full length human C1-inhibitor protein SEQ ID NO:2. In some embodiments, the fusion protein comprising a human C1-inhibitor polypeptide and an Fc domain includes an amino acid sequence at least 80% identical to the full length human C1-inhibitor protein SEQ ID NO:2. In some embodiments, the fusion protein comprising a human C1-inhibitor polypeptide and an Fc domain includes an amino acid sequence at least 90% identical to the full length human C1-inhibitor protein SEQ ID NO:2. In some embodiments, the fusion protein comprising a human C1-inhibitor polypeptide and an Fc domain includes an amino acid sequence at least 95% identical to the full length human C1-inhibitor protein SEQ ID NO:2. In some embodiments, the fusion protein comprising a human C1-inhibitor polypeptide and an Fc domain includes an amino acid sequence identical to the full length human C1-inhibitor protein SEQ ID NO:2. In some embodiments, the fusion protein comprising a human C1-inhibitor polypeptide and an Fc domain comprises one or more truncations, deletions, mutations or insertions compared to SEQ ID NO:2.

In some embodiments of the fusion protein comprising a human C1-inhibitor polypeptide and an Fc domain, the Fc domain is attached to the N-terminus of the human C1-inhibitor polypeptide. In some embodiments the Fc domain is a human IgG1 Fc domain. In some embodiments of the fusion protein comprising a human C1-inhibitor polypeptide and an Fc domain, the Fc domain is attached to the N-terminus of the human C1-inhibitor polypeptide. In some embodiments the Fc domain is derived from human IgG1 Fc domain. In some embodiments, an Fc domain suitable for the present invention comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:3. In some embodiments, the fusion protein comprising a human C1-inhibitor polypeptide and an Fc domain includes an amino acid sequence at least 70% identical to the human IgG1 Fc domain SEQ ID NO:3. In some embodiments, the fusion protein comprising a human C1-inhibitor polypeptide and an Fc domain includes an amino acid sequence at least 80% identical to the human TgG1 Fc domain SEQ ID NO:3. In some embodiments, the fusion protein comprising a human C1-inhibitor polypeptide and an Fc domain includes an amino acid sequence at least 90% identical to the human IgG1 Fc domain SEQ ID NO:3. In some embodiments, the fusion protein comprising a human C1-inhibitor polypeptide and an Fc domain includes an amino acid sequence at least 95% identical to the human IgG1 Fc domain SEQ ID NO:3. In some embodiments, the fusion protein comprising a human C1-inhibitor polypeptide and an Fc domain includes an amino acid sequence identical to the human IgG1 Fc domain SEQ ID NO:3. In some embodiments, the fusion protein comprising a human C1-inhibitor polypeptide and an Fc domain comprises one or more truncations, deletions, mutations or insertions compared to SEQ ID NO:3.

In some embodiments, an Fc domain suitable for the present invention comprises a L234A mutation. In some embodiments, an Fc domain suitable for the present invention comprises a L235A mutation. In some embodiments, an Fc domain suitable for the present invention comprises a L234A mutation and a L235A mutation. In some embodiments, an Fc domain suitable for the present invention comprises a L234A mutation or a L235A mutation.

In some embodiments, an Fc domain suitable for the present invention comprises the amino acid sequence of SEQ ID NO:4. In some embodiments, an Fc domain suitable for the present invention comprises one or more mutations that prolong the half-life of the fusion protein comprising a human C1-inhibitor polypeptide and an Fc domain. In some embodiments, the mutation of the Fc domain comprises one or more mutations are selected from one or more positions corresponding to Thr 250, Met 252, Ser 254, Thr 256, Thr 307, Glu 380, Met 428, His 433, and/or Asn 434 of human IgG1.

In some embodiments, an Fc domain suitable for the present invention comprises a H433K mutation. In some embodiments, an Fc domain suitable for the present invention comprises a N434F mutation. In some embodiments, an Fc domain suitable for the present invention comprises a H433K mutation and a N434F mutation. In some embodiments, an Fc domain suitable for the present invention comprises a H433K mutation or a N434F mutation.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:5. In some embodiments, the fusion protein comprising a human C1-inhibitor polypeptide and an Fc domain includes an amino acid sequence at least 70% identical to SEQ ID NO:5. In some embodiments, the fusion protein comprising a human C1-inhibitor polypeptide and an Fc domain includes an amino acid sequence at least 80% identical to SEQ ID NO:5. In some embodiments, the fusion protein comprising a human C1-inhibitor polypeptide and an Fc domain includes an amino acid sequence at least 90% identical to SEQ ID NO:5. In some embodiments, the fusion protein comprising a human C1-inhibitor polypeptide and an Fc domain includes an amino acid sequence at least 95% identical to SEQ ID NO:5. In some embodiments, the fusion protein comprising a human C1-inhibitor polypeptide and an Fc domain includes an amino acid sequence identical to SEQ ID NO:5. In some embodiments, the fusion protein comprising a human C1-inhibitor polypeptide and an Fc domain comprises one or more truncations, deletions, mutations or insertions compared to SEQ ID NO:5.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:6. In some embodiments, the fusion protein comprising a human C1-inhibitor polypeptide and an Fc domain includes an amino acid sequence at least 70% identical to SEQ ID NO:6. In some embodiments, the fusion protein comprising a human C1-inhibitor polypeptide and an Fc domain includes an amino acid sequence at least 80% identical to SEQ ID NO:6. In some embodiments, the fusion protein comprising a human C1-inhibitor polypeptide and an Fc domain includes an amino acid sequence at least 90% identical to SEQ ID NO:6. In some embodiments, the fusion protein comprising a human C1-inhibitor polypeptide and an Fc domain includes an amino acid sequence at least 95% identical to SEQ ID NO:6. In some embodiments, the fusion protein comprising a human C1-inhibitor polypeptide and an Fc domain includes an amino acid sequence identical to SEQ ID NO:6. In some embodiments, the fusion protein comprising a human C1-inhibitor polypeptide and an Fc domain comprises one or more truncations, deletions, mutations or insertions compared to SEQ ID NO:6.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:7. In some embodiments, the fusion protein comprising a human C1-inhibitor polypeptide and an Fc domain includes an amino acid sequence at least 70% identical to SEQ ID NO:7. In some embodiments, the fusion protein comprising a human C1-inhibitor polypeptide and an Fc domain includes an amino acid sequence at least 80% identical to SEQ ID NO:7. In some embodiments, the fusion protein comprising a human C1-inhibitor polypeptide and an Fc domain includes an amino acid sequence at least 90% identical to SEQ ID NO:7. In some embodiments, the fusion protein comprising a human C1-inhibitor polypeptide and an Fc domain includes an amino acid sequence at least 95% identical to SEQ ID NO:7. In some embodiments, the fusion protein comprising a human C1-inhibitor polypeptide and an Fc domain includes an amino acid sequence identical to SEQ ID NO:7. In some embodiments, the fusion protein comprising a human C1-inhibitor polypeptide and an Fc domain comprises one or more truncations, deletions, mutations or insertions compared to SEQ ID NO:7.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:8. In some embodiments, the fusion protein comprising a human C1-inhibitor polypeptide and an Fc domain includes an amino acid sequence at least 70% identical to SEQ ID NO:8. In some embodiments, the fusion protein comprising a human C1-inhibitor polypeptide and an Fc domain includes an amino acid sequence at least 80% identical to SEQ ID NO:8. In some embodiments, the fusion protein comprising a human C1-inhibitor polypeptide and an Fc domain includes an amino acid sequence at least 90% identical to SEQ ID NO:8. In some embodiments, the fusion protein comprising a human C1-inhibitor polypeptide and an Fc domain includes an amino acid sequence at least 95% identical to SEQ ID NO:8. In some embodiments, the fusion protein comprising a human C1-inhibitor polypeptide and an Fc domain includes an amino acid sequence identical to SEQ ID NO:8. In some embodiments, the fusion protein comprising a human C1-inhibitor polypeptide and an Fc domain comprises one or more truncations, deletions, mutations or insertions compared to SEQ ID NO:8. In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to any one of SEQ ID NO:5, SEQ ID NO:6, SEQ ID NO:7, or SEQ ID NO:8.

In some embodiments, the fusion protein comprising a human C1-inhibitor polypeptide and an Fc domain includes an amino acid sequence at least 70% identical to any one of SEQ ID NO:5, SEQ ID NO:6, SEQ ID NO:7, or SEQ ID NO:8. In some embodiments, the fusion protein comprising a human C1-inhibitor polypeptide and an Fc domain includes an amino acid sequence at least 80% identical to any one of SEQ ID NO:5, SEQ ID NO:6, SEQ ID NO:7, or SEQ ID NO:8. In some embodiments, the fusion protein comprising a human C1-inhibitor polypeptide and an Fc domain includes an amino acid sequence at least 90% identical to any one of SEQ ID NO:5, SEQ ID NO:6, SEQ ID NO:7, or SEQ ID NO:8. In some embodiments, the fusion protein comprising a human C1-inhibitor polypeptide and an Fc domain includes an amino acid sequence at least 95% identical to any one of SEQ ID NO:5, SEQ ID NO:6, SEQ ID NO:7, or SEQ ID NO:8. In some embodiments, the fusion protein comprising a human C1-inhibitor polypeptide and an Fc domain includes an amino acid sequence identical to any one of SEQ ID NO:5, SEQ ID NO:6, SEQ ID NO:7, or SEQ ID NO:8. In some embodiments, the fusion protein comprising a human C1-inhibitor polypeptide and an Fc domain comprises one or more truncations, deletions, mutations or insertions compared to SEQ ID NO:5, SEQ ID NO:6, SEQ ID NO:7, or SEQ ID NO:8.

In some embodiments of the fusion protein comprising a human C1-inhibitor polypeptide and an Fc domain, the Fc domain is attached to the N-terminus of the human C1-inhibitor polypeptide. In some embodiments the Fc domain is a human IgG4 Fc domain. In some embodiments of the fusion protein comprising a human C1-inhibitor polypeptide and an Fc domain, the Fc domain is attached to the N-terminus of the human C1-inhibitor polypeptide. In some embodiments the Fc domain is derived from a human IgG4 Fc domain.

In some embodiments, an Fc domain suitable for the present invention comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:9. In some embodiments, the fusion protein comprising a human C1-inhibitor polypeptide and an Fc domain includes an amino acid sequence at least 70% identical to the human IgG4 Fc domain SEQ ID NO:9. In some embodiments, the fusion protein comprising a human C1-inhibitor polypeptide and an Fc domain includes an amino acid sequence at least 80% identical to the human IgG4 Fc domain SEQ ID NO:9. In some embodiments, the fusion protein comprising a human C1-inhibitor polypeptide and an Fc domain includes an amino acid sequence at least 90% identical to the human IgG4 Fc domain SEQ ID NO:9. In some embodiments, the fusion protein comprising a human C1-inhibitor polypeptide and an Fc domain includes an amino acid sequence at least 95% identical to the human IgG4 Fc domain SEQ ID NO:9. In some embodiments, the fusion protein comprising a human C1-inhibitor polypeptide and an Fc domain includes an amino acid sequence identical to the human IgG4 Fc domain SEQ ID NO:9. In some embodiments, the fusion protein comprising a human C1-inhibitor polypeptide and an Fc domain comprises one or more truncations, deletions, mutations or insertions compared to SEQ ID NO:9.

In some embodiments, an Fc domain suitable for the present invention comprises a S241P mutation. In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:10. In some embodiments, the fusion protein comprising a human C1-inhibitor polypeptide and an Fc domain includes an amino acid sequence at least 70% identical to SEQ ID NO:10. In some embodiments, the fusion protein comprising a human C1-inhibitor polypeptide and an Fc domain includes an amino acid sequence at least 80% identical to SEQ ID NO:10. In some embodiments, the fusion protein comprising a human C1-inhibitor polypeptide and an Fc domain includes an amino acid sequence at least 90% identical to SEQ ID NO:10. In some embodiments, the fusion protein comprising a human C1-inhibitor polypeptide and an Fc domain includes an amino acid sequence at least 95% identical to SEQ ID NO:10. In some embodiments, the fusion protein comprising a human C1-inhibitor polypeptide and an Fc domain includes an amino acid sequence identical to SEQ ID NO:10. In some embodiments, the fusion protein comprising a human C1-inhibitor polypeptide and an Fc domain comprises one or more truncations, deletions, mutations or insertions compared to SEQ ID NO:10.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:11. In some embodiments, the fusion protein comprising a human C1-inhibitor polypeptide and an Fc domain includes an amino acid sequence at least 70% identical to SEQ ID NO:11. In some embodiments, the fusion protein comprising a human C1-inhibitor polypeptide and an Fc domain includes an amino acid sequence at least 80% identical to SEQ ID NO:11. In some embodiments, the fusion protein comprising a human C1-inhibitor polypeptide and an Fc domain includes an amino acid sequence at least 90% identical to SEQ ID NO:11. In some embodiments, the fusion protein comprising a human C1-inhibitor polypeptide and an Fc domain includes an amino acid sequence at least 95% identical to SEQ ID NO:11. In some embodiments, the fusion protein comprising a human C1-inhibitor polypeptide and an Fc domain includes an amino acid sequence identical to SEQ ID NO:11. In some embodiments, the fusion protein comprising a human C1-inhibitor polypeptide and an Fc domain comprises one or more truncations, deletions, mutations or insertions compared to SEQ ID NO:11.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:12. In some embodiments, the fusion protein comprising a human C1-inhibitor polypeptide and an Fc domain includes an amino acid sequence at least 70% identical to SEQ ID NO:12. In some embodiments, the fusion protein comprising a human C1-inhibitor polypeptide and an Fc domain includes an amino acid sequence at least 80% identical to SEQ ID NO:12. In some embodiments, the fusion protein comprising a human C1-inhibitor polypeptide and an Fc domain includes an amino acid sequence at least 90% identical to SEQ ID NO:12. In some embodiments, the fusion protein comprising a human C1-inhibitor polypeptide and an Fc domain includes an amino acid sequence at least 95% identical to SEQ ID NO:12. In some embodiments, the fusion protein comprising a human C1-inhibitor polypeptide and an Fc domain includes an amino acid sequence identical to SEQ ID NO:12. In some embodiments, the fusion protein comprising a human C1-inhibitor polypeptide and an Fc domain comprises one or more truncations, deletions, mutations or insertions compared to SEQ ID NO:12.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:13. In some embodiments, the fusion protein comprising a human C1-inhibitor polypeptide and an Fc domain includes an amino acid sequence at least 70% identical to SEQ ID NO:13. In some embodiments, the fusion protein comprising a human C1-inhibitor polypeptide and an Fc domain includes an amino acid sequence at least 80% identical to SEQ ID NO:13. In some embodiments, the fusion protein comprising a human C1-inhibitor polypeptide and an Fc domain includes an amino acid sequence at least 90% identical to SEQ ID NO:13. In some embodiments, the fusion protein comprising a human C1-inhibitor polypeptide and an Fc domain includes an amino acid sequence at least 95% identical to SEQ ID NO:13. In some embodiments, the fusion protein comprising a human C1-inhibitor polypeptide and an Fc domain includes an amino acid sequence identical to SEQ ID NO:13. In some embodiments, the fusion protein comprising a human C1-inhibitor polypeptide and an Fc domain comprises one or more truncations, deletions, mutations or insertions compared to SEQ ID NO:13.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:14. In some embodiments, the fusion protein comprising a human C1-inhibitor polypeptide and an Fc domain includes an amino acid sequence at least 70% identical to SEQ ID NO:14. In some embodiments, the fusion protein comprising a human C1-inhibitor polypeptide and an Fc domain includes an amino acid sequence at least 80% identical to SEQ ID NO:14. In some embodiments, the fusion protein comprising a human C1-inhibitor polypeptide and an Fc domain includes an amino acid sequence at least 90% identical to SEQ ID NO:14. In some embodiments, the fusion protein comprising a human C1-inhibitor polypeptide and an Fc domain includes an amino acid sequence at least 95% identical to SEQ ID NO:14. In some embodiments, the fusion protein comprising a human C1-inhibitor polypeptide and an Fc domain includes an amino acid sequence identical to SEQ ID NO:14. In some embodiments, the fusion protein comprising a human C1-inhibitor polypeptide and an Fc domain comprises one or more truncations, deletions, mutations or insertions compared to SEQ ID NO:14.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:15. In some embodiments, the fusion protein comprising a human C1-inhibitor polypeptide and an Fc domain includes an amino acid sequence at least 70% identical to SEQ ID NO:15. In some embodiments, the fusion protein comprising a human C1-inhibitor polypeptide and an Fc domain includes an amino acid sequence at least 80% identical to SEQ ID NO: 15. In some embodiments, the fusion protein comprising a human C1-inhibitor polypeptide and an Fc domain includes an amino acid sequence at least 90% identical to SEQ ID NO:15. In some embodiments, the fusion protein comprising a human C1-inhibitor polypeptide and an Fc domain includes an amino acid sequence at least 95% identical to SEQ ID NO:15. In some embodiments, the fusion protein comprising a human C1-inhibitor polypeptide and an Fc domain includes an amino acid sequence identical to SEQ ID NO:15. In some embodiments, the fusion protein comprising a human C1-inhibitor polypeptide and an Fc domain comprises one or more truncations, deletions, mutations or insertions compared to SEQ ID NO:15.

In some embodiments, fusion protein comprising a human C1-inhibitor polypeptide and an Fc domain comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:16. In some embodiments, the fusion protein comprising a human C1-inhibitor polypeptide and an Fc domain includes an amino acid sequence at least 70% identical to SEQ ID NO:16. In some embodiments, the fusion protein comprising a human C1-inhibitor polypeptide and an Fc domain includes an amino acid sequence at least 80% identical to SEQ ID NO: 16. In some embodiments, the fusion protein comprising a human C1-inhibitor polypeptide and an Fc domain includes an amino acid sequence at least 90% identical to SEQ ID NO:16. In some embodiments, the fusion protein comprising a human C1-inhibitor polypeptide and an Fc domain includes an amino acid sequence at least 95% identical to SEQ ID NO:16. In some embodiments, the fusion protein comprising a human C1-inhibitor polypeptide and an Fc domain includes an amino acid sequence identical to SEQ ID NO:16. In some embodiments, the fusion protein comprising a human C1-inhibitor polypeptide and an Fc domain comprises one or more truncations, deletions, mutations or insertions compared to SEQ ID NO:16.

In some embodiments of the fusion protein comprising a human C1-inhibitor polypeptide and an Fc domain, the fusion protein comprises a linker between the human C1-inhibitor polypeptide and the Fc domain. In some embodiments, the linker is a peptide comprising 3-100 amino acids.

In some embodiments of the fusion protein comprising a human C1-inhibitor polypeptide and an Fc domain, the fusion protein binds FcRN. In some embodiments of the fusion protein comprising a human C1-inhibitor polypeptide and an Fc domain, the fusion protein inhibits C1 esterase activity.

In some embodiments of the fusion protein comprising a human C1-inhibitor polypeptide and an Fc domain, the Fc domain comprises a mutation that reduces or eliminates ADCC activity. In some embodiments of the fusion protein comprising a human C1-inhibitor polypeptide and an Fc domain, the Fc domain comprises one or more mutations that reduce or eliminate ADCC activity. In some embodiments of the fusion protein comprising a human C1-inhibitor polypeptide and an Fc domain, the Fc domain comprises a mutation that reduces or eliminates CDC activity. In some embodiments of the fusion protein comprising a human C1-inhibitor polypeptide and an Fc domain, the Fc domain comprises one or more mutations that reduce or eliminate CDC activity.

In some embodiments of the fusion protein comprising a human C1-inhibitor polypeptide and an Fc domain, the Fc domain comprises a mutation that reduce or eliminate FcyR binding. In some embodiments of the fusion protein comprising a human C1-inhibitor polypeptide and an Fc domain, the Fc domain comprises one or more mutations that reduce or eliminate FcyR binding. In some embodiments of the fusion protein comprising a human C1-inhibitor polypeptide and an Fc domain, the Fc domain comprises a mutation that reduces or eliminates FcyR effector function. In some embodiments of the fusion protein comprising a human C1-inhibitor polypeptide and an Fc domain, the Fc domain comprises one or more mutations that reduce or eliminate FcyR effector function.

In some embodiments of the fusion protein comprising a human C1-inhibitor polypeptide and an Fc domain, the Fc domain comprises a mutation that reduces or eliminates C1q binding. In some embodiments of the fusion protein comprising a human C1-inhibitor polypeptide and an Fc domain, the Fc domain comprises one or more mutations that reduce or eliminate C1q binding. In some embodiments of the fusion protein comprising a human C1-inhibitor polypeptide and an Fc domain, the mutation that reduces or eliminates C1q binding is in the Fc domain.

In some embodiments of the fusion protein comprising a human C1-inhibitor polypeptide and an Fc domain, the fusion protein inhibits and/or inactivates C1r and/or C1s protease activity. In some embodiments of the fusion protein comprising a human C1-inhibitor polypeptide and an Fc domain, the fusion protein inhibits the lysis of red blood cells *in vitro.*

In some embodiments of the fusion protein comprising a human C1-inhibitor polypeptide and an Fc domain, the fusion protein has a longer half-life than plasma-derived human C1-inhibitor. In some embodiments of the fusion protein comprising a human C1-inhibitor polypeptide and an Fc domain, the fusion protein has a half-life of at least four days. In some embodiments of the fusion protein comprising a human C1-inhibitor polypeptide and an Fc domain, the fusion protein has a half-life of at least five days. In some embodiments of the fusion protein comprising a human C1-inhibitor polypeptide and an Fc domain, the fusion protein has a half-life of at least six days. In some embodiments of the fusion protein comprising a human C1-inhibitor polypeptide and an Fc domain, the fusion protein has a half-life of at least seven days.

In some embodiments of the fusion protein comprising a human C1-inhibitor polypeptide and an Fc domain, the fusion protein has an *in vivo* half-life of or greater than about 12 hours, 18 hours, 24 hours, 36 hours, 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, or 10 days. In some embodiments, a recombinant C1-INH fusion protein has an *in vivo* half-life of between 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

In some embodiments of the fusion protein comprising a human C1-inhibitor polypeptide and an Fc domain, the fusion protein is monovalent. In some embodiments of the fusion protein comprising a human C1-inhibitor polypeptide and an Fc domain, the fusion protein is dimeric.

In one aspect, the present invention provides a fusion protein comprising a human C1-inhibitor polypeptide and an albumin polypeptide. In some embodiments, the human C1-inhibitor polypeptide comprises an amino acid sequence at least at least 50% (e.g., at least 55%, 60%, 65%, 70%, 75%, 80%, 85%, 90%, 95%, 96%, 97%, 98%, 99%, or 100%) identical to the full length human C1-inhibitor having SEQ ID NO:1. In some embodiments, the fusion protein comprising a human C1-inhibitor polypeptide and an albumin polypeptide includes an amino acid sequence at least 70% identical to the full length human C1-inhibitor protein SEQ ID NO:1. In some embodiments, the fusion protein comprising a human C1-inhibitor polypeptide and an albumin polypeptide includes an amino acid sequence at least 80% identical to the full length human C1-inhibitor protein SEQ ID NO:1. In some embodiments, the fusion protein comprising a human C1-inhibitor polypeptide and an albumin polypeptide includes an amino acid sequence at least 90% identical to the full length human C1-inhibitor protein SEQ ID NO:1. In some embodiments, the fusion protein comprising a human C1-inhibitor polypeptide and an albumin polypeptide includes an amino acid sequence at least 95% identical to the full length human C1-inhibitor protein SEQ ID NO:1. In some embodiments, the fusion protein comprising a human C1-inhibitor polypeptide and an albumin polypeptide includes an amino acid sequence identical to the full length human C1-inhibitor protein SEQ ID NO:1. In some embodiments, the fusion protein comprising a human C1-inhibitor polypeptide and an albumin polypeptide comprises one or more truncations, deletions, mutations or insertions compared to SEQ ID NO:1.

In some embodiments, the fusion protein comprising a human C1-inhibitor polypeptide and an albumin polypeptide comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:2. In some embodiments, the fusion protein comprising a human C1-inhibitor polypeptide and an albumin polypeptide includes an amino acid sequence at least 70% identical to the full length human C1-inhibitor protein SEQ ID NO:2. In some embodiments, the fusion protein comprising a human C1-inhibitor polypeptide and an albumin polypeptide includes an amino acid sequence at least 80% identical to the full length human C1-inhibitor protein SEQ ID NO:2. In some embodiments, the fusion protein comprising a human C1-inhibitor polypeptide and an albumin polypeptide includes an amino acid sequence at least 90% identical to the full length human C1-inhibitor protein SEQ ID NO:2. In some embodiments, the fusion protein comprising a human C1-inhibitor polypeptide and an albumin polypeptide includes an amino acid sequence at least 95% identical to the full length human C1-inhibitor protein SEQ ID NO:2. In some embodiments, the fusion protein comprising a human C1-inhibitor polypeptide and an albumin polypeptide includes an amino acid sequence identical to the full length human C1-inhibitor protein SEQ ID NO:2. In some embodiments, the fusion protein comprising a human C1-inhibitor polypeptide and an albumin polypeptide comprises one or more truncations, deletions, mutations or insertions compared to SEQ ID NO:2.

In some embodiments of the fusion protein comprising a human C1-inhibitor polypeptide and an albumin polypeptide, the albumin is attached to the N-terminus of the human C1-inhibitor polypeptide.. In some embodiments the albumin polypeptide comprises one or more domains of human serum albumin. In some embodiments the albumin polypeptide comprises the D3 domain of human serum albumin. In some embodiments the albumin polypeptide is derived from human serum albumin.. In some embodiments the albumin polypeptide is human serum albumin.

In some embodiments, an albumin polypeptide suitable for the present invention comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:20. In some embodiments, the fusion protein comprising a human C1-inhibitor polypeptide and an albumin polypeptide includes an amino acid sequence at least 70% identical to the human albumin polypeptide SEQ ID NO:20. In some embodiments, the fusion protein comprising a human C1-inhibitor polypeptide and an albumin polypeptide includes an amino acid sequence at least 80% identical to the human albumin polypeptide SEQ ID NO:20. In some embodiments, the fusion protein comprising a human C1-inhibitor polypeptide and an albumin polypeptide includes an amino acid sequence at least 90% identical to the human albumin polypeptide SEQ ID NO:20. In some embodiments, the fusion protein comprising a human C1-inhibitor polypeptide and an albumin polypeptide includes an amino acid sequence at least 95% identical to the human albumin polypeptide SEQ ID NO:20. In some embodiments, the fusion protein comprising a human C1-inhibitor polypeptide and an albumin polypeptide includes an amino acid sequence identical to the human albumin polypeptide SEQ ID NO:20. In some embodiments, the fusion protein comprising a human C1-inhibitor polypeptide and an albumin polypeptide comprises one or more truncations, deletions, mutations or insertions compared to SEQ ID NO:20.

In some embodiments, an albumin polypeptide suitable for the present invention comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:17. In some embodiments, the fusion protein comprising a human C1-inhibitor polypeptide and an albumin polypeptide includes an amino acid sequence at least 70% identical to the human albumin polypeptide SEQ ID NO:17. In some embodiments, the fusion protein comprising a human C1-inhibitor polypeptide and an albumin polypeptide includes an amino acid sequence at least 80% identical to the human albumin polypeptide SEQ ID NO:17. In some embodiments, the fusion protein comprising a human C1-inhibitor polypeptide and an albumin polypeptide includes an amino acid sequence at least 90% identical to the human albumin polypeptide SEQ ID NO: 17. In some embodiments, the fusion protein comprising a human C1-inhibitor polypeptide and an albumin polypeptide includes an amino acid sequence at least 95% identical to the human albumin polypeptide SEQ ID NO: 17. In some embodiments, the fusion protein comprising a human C1-inhibitor polypeptide and an albumin polypeptide includes an amino acid sequence identical to the human albumin polypeptide SEQ ID NO:17. In some embodiments, the fusion protein comprising a human C1-inhibitor polypeptide and an albumin polypeptide comprises one or more truncations, deletions, mutations or insertions compared to SEQ ID NO: 17.

In some embodiments of the fusion protein comprising a human C1-inhibitor polypeptide and an albumin polypeptide, the fusion protein comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:18. In some embodiments, the fusion protein comprising a human C1-inhibitor polypeptide and an albumin polypeptide includes an amino acid sequence at least 70% identical to SEQ ID NO:18. In some embodiments, the fusion protein comprising a human C1-inhibitor polypeptide and an albumin polypeptide includes an amino acid sequence at least 80% identical to SEQ ID NO:18. In some embodiments, the fusion protein comprising a human C1-inhibitor polypeptide and an albumin polypeptide includes an amino acid sequence at least 90% identical to SEQ ID NO:18. In some embodiments, the fusion protein comprising a human C1-inhibitor polypeptide and an albumin polypeptide includes an amino acid sequence at least 95% identical to SEQ ID NO:18. In some embodiments, the fusion protein comprising a human C1-inhibitor polypeptide and an albumin polypeptide includes an amino acid sequence identical to SEQ ID NO:18. In some embodiments, the fusion protein comprising a human C1-inhibitor polypeptide and an albumin polypeptide comprises one or more truncations, deletions, mutations or insertions compared to SEQ ID NO:18.

In some embodiments of the fusion protein comprising a human C1-inhibitor polypeptide and an albumin polypeptide, the fusion protein comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:19. In some embodiments, the fusion protein comprising a human C1-inhibitor polypeptide and an albumin polypeptide includes an amino acid sequence at least 70% identical to SEQ ID NO:19. In some embodiments, the fusion protein comprising a human C1-inhibitor polypeptide and an albumin polypeptide includes an amino acid sequence at least 80% identical to SEQ ID NO:19. In some embodiments, the fusion protein comprising a human C1-inhibitor polypeptide and an albumin polypeptide includes an amino acid sequence at least 90% identical to SEQ ID NO:19. In some embodiments, the fusion protein comprising a human C1-inhibitor polypeptide and an albumin polypeptide includes an amino acid sequence at least 95% identical to SEQ ID NO:19. In some embodiments, the fusion protein comprising a human C1-inhibitor polypeptide and an albumin polypeptide includes an amino acid sequence identical to SEQ ID NO:19. In some embodiments, the fusion protein comprising a human C1-inhibitor polypeptide and an albumin polypeptide comprises one or more truncations, deletions, mutations or insertions compared to SEQ ID NO:19.

In some embodiments of the fusion protein comprising a human C1-inhibitor polypeptide and an albumin polypeptide, the fusion protein comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:21. In some embodiments, the fusion protein comprising a human C1-inhibitor polypeptide and an albumin polypeptide includes an amino acid sequence at least 70% identical to SEQ ID NO:21. In some embodiments, the fusion protein comprising a human C1-inhibitor polypeptide and an albumin polypeptide includes an amino acid sequence at least 80% identical to SEQ ID NO:21. In some embodiments, the fusion protein comprising a human C1-inhibitor polypeptide and an albumin polypeptide includes an amino acid sequence at least 90% identical to SEQ ID NO:21. In some embodiments, the fusion protein comprising a human C1-inhibitor polypeptide and an albumin polypeptide includes an amino acid sequence at least 95% identical to SEQ ID NO:21. In some embodiments, the fusion protein comprising a human C1-inhibitor polypeptide and an albumin polypeptide includes an amino acid sequence identical to SEQ ID NO:21. In some embodiments, the fusion protein comprising a human C1-inhibitor polypeptide and an albumin polypeptide comprises one or more truncations, deletions, mutations or insertions compared to SEQ ID NO:21.

In some embodiments of the fusion protein comprising a human C1-inhibitor polypeptide and an albumin polypeptide, the fusion protein comprises an amino acid sequence at least 50% (e.g., at least 55%, 60%, 65%, 70%, 75%, 80%, 85%, 90%, 95%, 96%, 97%, 98%, 99%, or 100%) identical to SEQ ID NO:22. In some embodiments, the fusion protein comprising a human C1-inhibitor polypeptide and an albumin polypeptide includes an amino acid sequence at least 70% identical to SEQ ID NO:22. In some embodiments, the fusion protein comprising a human C1-inhibitor polypeptide and an albumin polypeptide includes an amino acid sequence at least 80% identical to SEQ ID NO:22. In some embodiments, the fusion protein comprising a human C1-inhibitor polypeptide and an albumin polypeptide includes an amino acid sequence at least 90% identical to SEQ ID NO:22. In some embodiments, the fusion protein comprising a human C1-inhibitor polypeptide and an albumin polypeptide includes an amino acid sequence at least 95% identical to SEQ ID NO:22. In some embodiments, the fusion protein comprising a human C1-inhibitor polypeptide and an albumin polypeptide includes an amino acid sequence identical to SEQ ID NO:22. In some embodiments, the fusion protein comprising a human C1-inhibitor polypeptide and an albumin polypeptide comprises one or more truncations, deletions, mutations or insertions compared to SEQ ID NO:22.

In some embodiments, fusion protein comprising a human C1-inhibitor polypeptide and an albumin polypeptide comprises an amino acid sequence at least 50% (e.g., at least 55%, 60%, 65%, 70%, 75%, 80%, 85%, 90%, 95%, 96%, 97%, 98%, 99%, or 100%) identical to any one of SEQ ID NO:17, SEQ ID NO:18, SEQ ID NO:19, SEQ ID NO:21, or SEQ ID NO:22. In some embodiments, the fusion protein comprising a human C1-inhibitor polypeptide and an albumin polypeptide includes an amino acid sequence at least 70% identical to any one of SEQ ID NO:17, SEQ ID NO:18, SEQ ID NO:19, SEQ ID NO:21, or SEQ ID NO:22. In some embodiments, the fusion protein comprising a human Cl-inhibitor polypeptide and an albumin polypeptide includes an amino acid sequence at least 80% identical to any one of SEQ ID NO:17, SEQ ID NO:18, SEQ ID NO:19, SEQ ID NO:21, or SEQ ID NO:22. In some embodiments, the fusion protein comprising a human C1-inhibitor polypeptide and an albumin polypeptide includes an amino acid sequence at least 90% identical to any one of SEQ ID NO:17, SEQ ID NO:18, SEQ ID NO:19, SEQ ID NO:21, or SEQ ID NO:22. In some embodiments, the fusion protein comprising a human C1-inhibitor polypeptide and an albumin polypeptide includes an amino acid sequence at least 95% identical to any one of SEQ ID NO:17, SEQ ID NO:18, SEQ ID NO:19, SEQ ID NO:21, or SEQ ID NO:22. In some embodiments, the fusion protein comprising a human C1-inhibitor polypeptide and an albumin polypeptide includes an amino acid sequence identical to any one of SEQ ID NO:17, SEQ ID NO:18, SEQ ID NO:19, SEQ ID NO:21, or SEQ ID NO:22. In some embodiments, the fusion protein comprising a human C1-inhibitor polypeptide and an albumin polypeptide comprises one or more truncations, deletions, mutations or insertions compared to SEQ ID NO:17, SEQ ID NO:18, SEQ ID NO:19, SEQ ID NO:21, or SEQ ID NO:22.

In some embodiments of the fusion protein comprising a human C1-inhibitor polypeptide and an albumin polypeptide, the fusion protein binds FcRN. In some embodiments of the fusion protein comprising a human C1-inhibitor polypeptide and an albumin polypeptide, the fusion protein inhibits C1 esterase activity. In some embodiments of the fusion protein comprising a human C1-inhibitor polypeptide and an albumin polypeptide, the fusion protein inhibits and/or inactivates C1r and/or C1s protease activity. In some embodiments of the fusion protein comprising a human C1-inhibitor polypeptide and an albumin polypeptide, the fusion protein inhibits the lysis of red blood cells *in vitro.*

In some embodiments of the fusion protein comprising a human C1-inhibitor polypeptide and an albumin polypeptide, the fusion protein comprises a linker between the human C1-inhibitor polypeptide and the albumin polypeptide. In some embodiments, the linker is a peptide comprising 3-100 amino acids. In some embodiments, the linker comprises the sequence GGG. In some embodiments, the linker comprises the sequence of SEQ ID NO:27.

In some embodiments of the fusion protein comprising a human C1-inhibitor polypeptide and an albumin polypeptide, the albumin polypeptide does not comprise one or more mutations selected from the group consisting of 464His, 510 His, 535 His, and/or a combination thereof.

In some embodiments of the fusion protein comprising a human C1-inhibitor polypeptide and an albumin polypeptide, the fusion protein has a longer half-life than plasma-derived human C1-inhibitor. In some embodiments of the fusion protein comprising a human C1-inhibitor polypeptide and an albumin polypeptide, the fusion protein has a half-life of at least four days. In some embodiments of the fusion protein comprising a human C1-inhibitor polypeptide and an albumin polypeptide, the fusion protein has a half-life of at least five days. In some embodiments of the fusion protein comprising a human C1-inhibitor polypeptide and an albumin polypeptide, the fusion protein has a half-life of at least six days. In some embodiments of the fusion protein comprising a human C1-inhibitor polypeptide and an albumin polypeptide, the fusion protein has a half-life of at least seven days.

In some embodiments of the fusion protein comprising a human C1-inhibitor polypeptide and an albumin polypeptide, the fusion protein has an *in vivo* half-life of or greater than about 12 hours, 18 hours, 24 hours, 36 hours, 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, or 10 days. In some embodiments, a recombinant C1-INH fusion protein has an *in vivo* half-life of between 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

In one aspect the present invention provides a nucleic acid encoding a fusion protein of any one of the fusion proteins comprising a human C1-inhibitor polypeptide and an Fc domain disclosed herein. In another aspect the present invention provides a nucleic acid encoding a fusion protein of any one of the fusion proteins comprising a human C1-inhibitor polypeptide and an albumin polypeptide disclosed herein.

In one aspect the present invention provides a cell comprising a nucleic acid encoding a fusion protein of any one of the fusion proteins comprising a human C1-inhibitor polypeptide and an Fc domain disclosed herein. In another aspect the present invention provides a cell comprising a nucleic acid encoding a fusion protein of any one of the fusion proteins comprising a human C1-inhibitor polypeptide and an albumin polypeptide disclosed herein.

In some embodiments, the cell is a mammalian cell. In some embodiments, the mammalian cell is a human cell. In some embodiments, the mammalian cell is a Chinese Hamster Ovary (CHO) cell. In some embodiments, the cell is engineered to modify glycosylation of protein expressed by the cell. In some embodiments, the cell is engineered to modify glycosylation of protein expressed by the cell as compared to the same cell that has not been engineered. In some embodiments, the cell is engineered to enhance, improve, increase and/or humanize glycosylation of protein expressed by the cell. In some embodiments, the cell is engineered to enhance, improve, increase and/or humanize glycosylation of protein expressed by the cell as compared to the same cell that has not been engineered. In some embodiments, the cell is engineered to modify sialylation of protein expressed by the cell. In some embodiments, the cell is engineered to modify sialylation of protein expressed by the cell as compared to the same cell that has not been engineered. In some embodiments, the cell is engineered to enhance, improve, increase and/or humanize sialylation of protein expressed by the cell. In some embodiments, the cell is engineered to enhance, improve, increase and/or humanize sialylation of protein expressed by the cell as compared to the same cell that has not been engineered.

In one aspect, the invention provides a method of producing a fusion protein comprising a step of culturing or cultivating a cell comprising a nucleic acid encoding a fusion protein of any one of the fusion proteins comprising a human C1-inhibitor polypeptide and an Fc domain disclosed herein. In one aspect, the invention provides a method of producing a fusion protein comprising a step of culturing or cultivating a cell comprising a nucleic acid encoding a fusion protein of any one of the fusion proteins comprising a human C1-inhibitor polypeptide and an albumin polypeptide disclosed herein.

In another aspect, the invention provides a fusion protein comprising a human C1-inhibitor polypeptide and an Fc domain and expressed or produced by a cell engineered to modify, enhance, improve, increase and/or humanize glycosylation. In another aspect, the invention provides a fusion protein comprising a human C1-inhibitor polypeptide and an Fc domain and expressed or produced by a cell engineered to modify, enhance, improve, increase and/or humanize sialylation. In another aspect, the invention provides a fusion protein comprising a human C1-inhibitor polypeptide and an albumin polypeptide and expressed or produced by a cell engineered to modify, enhance, improve, increase and/or humanize glycosylation. In another aspect, the invention provides a fusion protein comprising a human C1-inhibitor polypeptide and an albumin polypeptide and expressed or produced by a cell engineered to modify, enhance, improve, increase and/or humanize sialylation.

In another aspect, the invention provides a pharmaceutical composition comprising a fusion protein of any one of the fusion proteins comprising a human C1-inhibitor polypeptide and an Fc domain disclosed herein and a pharmaceutically acceptable carrier. In another aspect, the invention provides a pharmaceutical composition comprising a fusion protein of any one of the fusion proteins comprising a human C1-inhibitor polypeptide and an albumin polypeptide disclosed herein and a pharmaceutically acceptable carrier.

In one aspect, the invention provides a method of treating a complement-mediated disorder comprising administering to a subject in need of treatment a pharmaceutical composition comprising a fusion protein of any one of the fusion proteins comprising a human C1-inhibitor polypeptide and an Fc domain disclosed herein and a pharmaceutically acceptable carrier. In one aspect, the invention provides a method of treating a complement-mediated disorder comprising administering to a subject in need of treatment a pharmaceutical composition comprising a fusion protein of any one of the fusion proteins comprising a human C1-inhibitor polypeptide and an albumin polypeptide disclosed herein and a pharmaceutically acceptable carrier.

In some embodiments, the subject in need of treatment has a complement-mediated disorder. In some embodiments, the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

### BRIEF DESCRIPTION OF THE DRAWINGS

The drawings are for illustration purposes only, not for limitation.

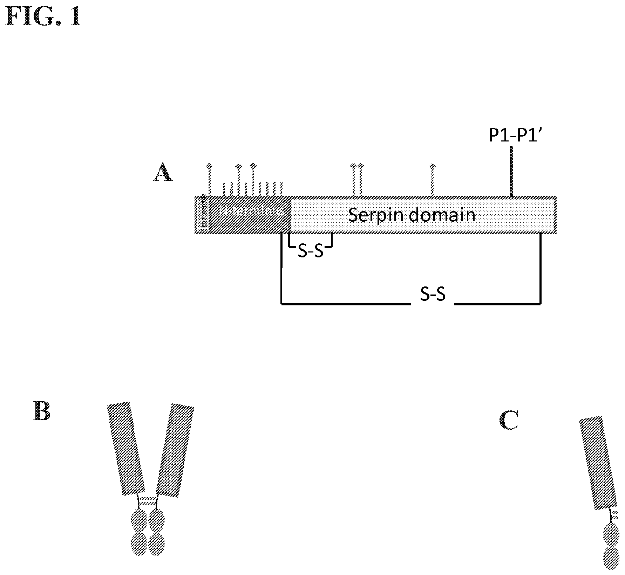

FIG. 1A is a schematic representation of C1-INH. From right to left the three domains are the signal peptide, the N-terminus, also referred to as N-terminal domain, and the serpin domain. N-linked glycans are shown as long vertical lines with diamond heads and O-linked glycans are shown as short vertical lines. FIG. 1B is a schematic representation of an exemplary dimeric C1-INH Fc fusion protein. The C1-INH polypeptide portions are indicated by the rectangles and the Fc portion is indicated by the oval segments. FIG. 1C is a schematic representation of an exemplary monomeric C1-INH Fc fusion protein.

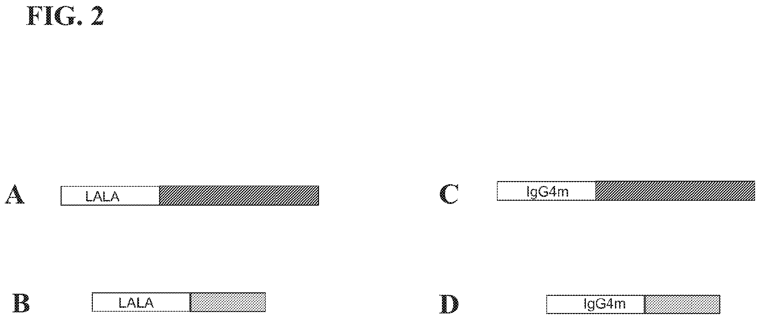

FIG. 2A-2D are a schematic representations of exemplary C1-INH fusion proteins. FIG. 2A is a representation of an IgG1 Fc with LALA mutation fused to a full length C1-INH polypeptide (shown as shaded rectangle). FIG. 2B is a representation of an IgG1 Fc with LALA mutation fused to a truncated C1-INH polypeptide (shown as shaded rectangle). FIG. 2C is a representation of an IgG4 Fc with S241P mutation fused to a full length C1-INH polypeptide (shown as shaded rectangle). FIG. 2D is a representation of an IgG4 Fc with S241P mutation fused to a truncated C1-INH polypeptide (shown as shaded rectangle).

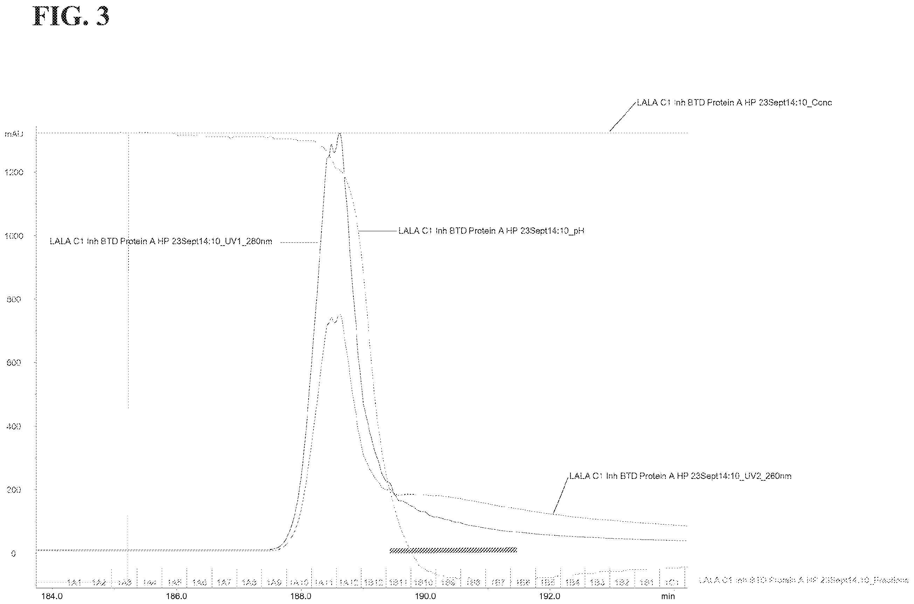

FIG. 3 shows the results of an exemplary protein A purification of full length C1-INH human Fc (hFc) IgG1 LALA fusion protein. The graph shows the results of UV 280 nm, UV 260 nm, concentration, and pH over time during the capture and clution of the fusion protein during the purification process.

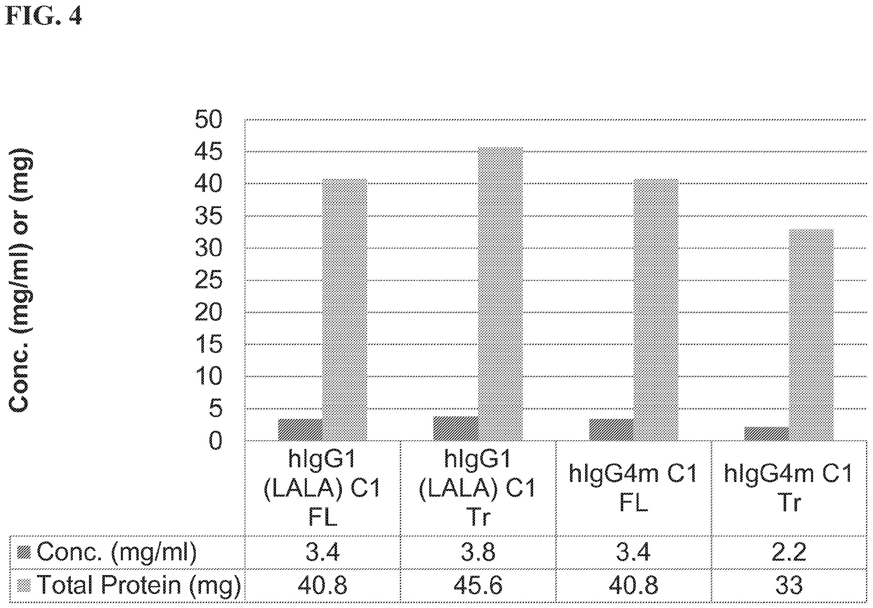

FIG. 4 is a chart of the concentration and total protein collected during prufication of four exemplary Fc fusion constructs: full length (FL) C1-INH hFc IgG1 LALA fusion protein; truncated (Tr) C1-INH hFc IgG1 LALA fusion protein; full length (FL) C1-INH hFc IgG4m (IgG4 S241P) fusion protein; truncated (Tr) C1-INH hFc IgG4m (IgG4 S241P) fusion protein.

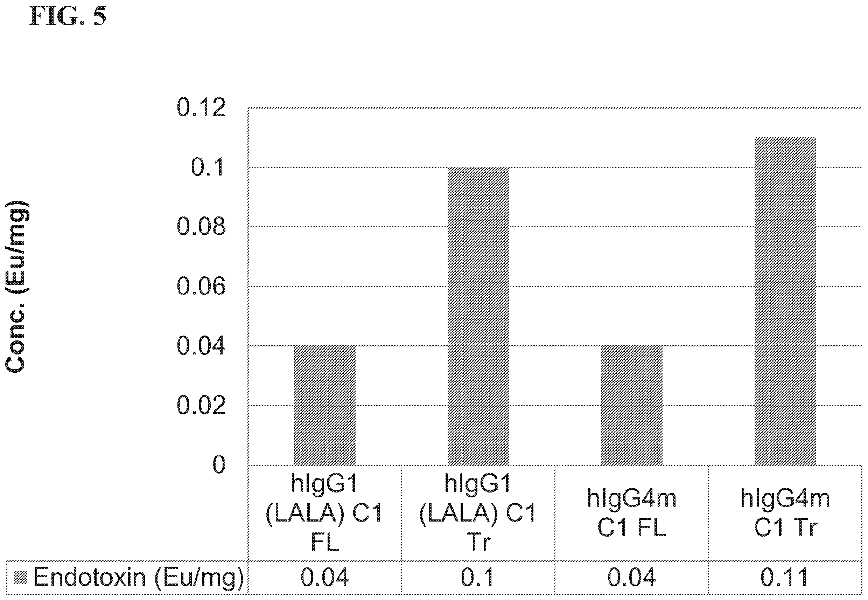

FIG. 5 is a chart depicting the amount of endotoxin detected in the purified samples of full length (FL) C1-INH hFc IgG1 LALA fusion protein; truncated (Tr) C1-INH hFc IgG1 LALA fusion protein; full length (FL) C1-INH hFc IgG4m (IgG4 S241P) fusion protein; truncated (Tr) C1-INH hFc IgG4m (IgG4 S241P) fusion protein.

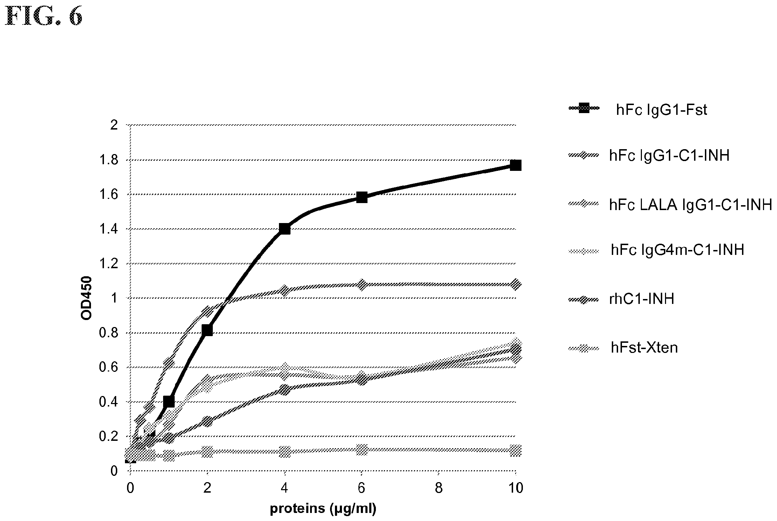

FIG. 6 shows the results of an exemplary C1q binding ELISA for the hFc IgG1-C1-INH, hFc LALA IgG1-C1-INH, and hFc IgG4m-C1-INH fusions, recombinant human C1-INH (rhC1-INH) (expressed in 1080 cells), and IgG1 Fc-human follistatin(hFc IgG1-hFst-XTEN) fusion as a positive control and human follistatin-Xten (hFst-XTEN) fusion as a negative control.

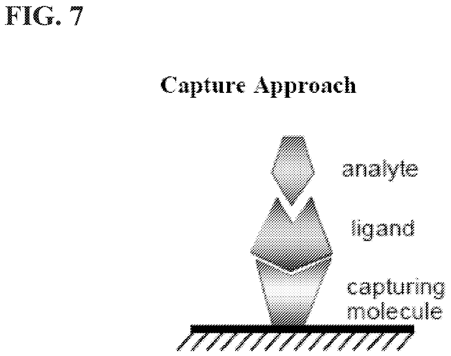

FIG. 7 is a schematic of the surface plasmon resonance (SPR) Biacore capture approach utilized to measure binding of the exemplary fusion constructs to the extracellular domain of the FcγR1.

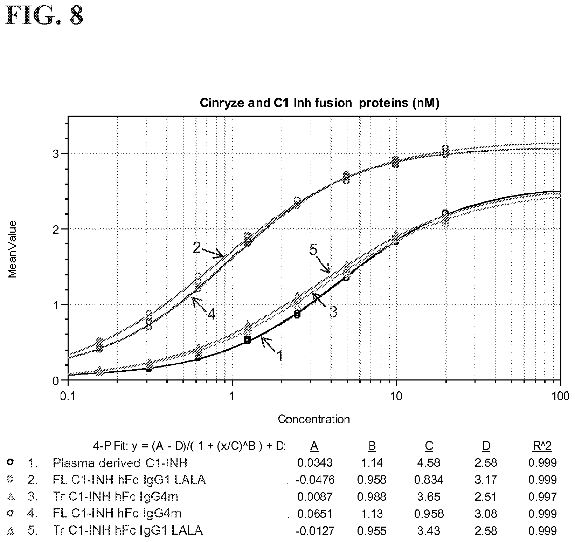

FIG. 8 shows the results of SPR analysis of exemplary effector dead Fc fusion constructs: full length (FL) C1-INH hFc IgG1 LALA fusion protein; truncated (Tr) C1-INH hFc IgG1 LALA fusion protein; full length (FL) C1-INH hFc IgG4m (IgG4 S241P) fusion protein; truncated (Tr) C1-INH hFc IgG4m (IgG4 S241P) fusion protein. Plasma-derived C1-INH was included as a positive control.

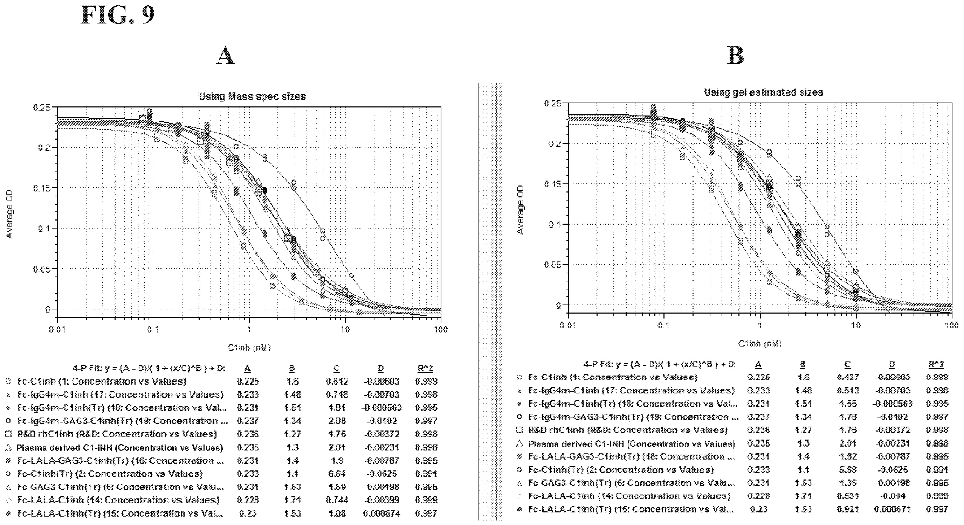

FIGs. 9A and 9B present the results of an assay to measure the ability of the effector dead constructs to inhibit C1s cleavage of a colorimetric peptide. FIG. 9A shows the titration curve for each of the exemplary effector dead constructs tested using the mass spectrometry calculated molecular weights for each of the constructs. FIG. 9B shows the titration curve for each of the exemplary effector dead constructs tested using the gel estimated molecular weights for each of the constructs.

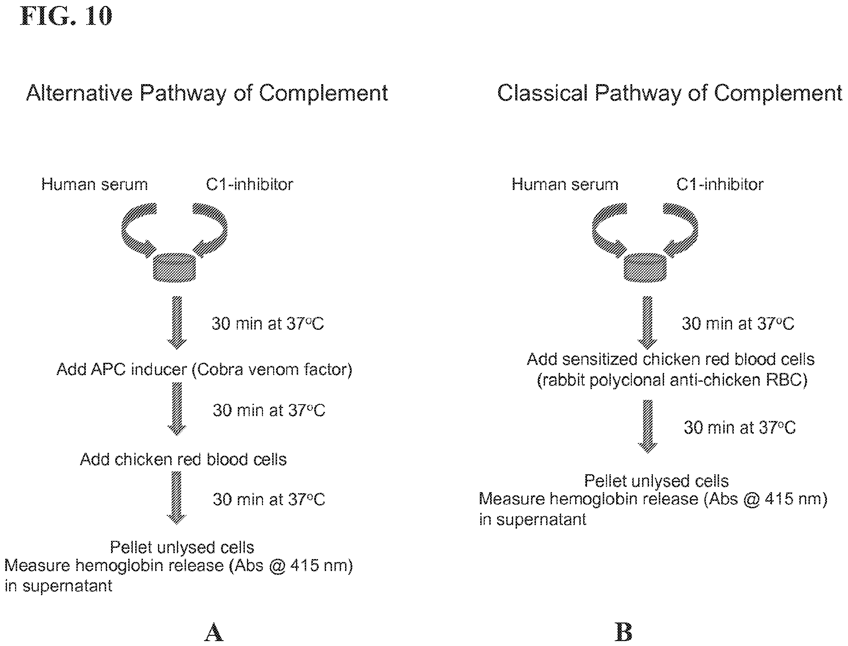

FIG. 10A depicts a schematic overview of a hemolysis assay to measure the activation of the alternative pathway of complement (APC). FIG. 10B depicts a schematic overview of a hemolysis assay to measure the activation of the classical pathway of complement.

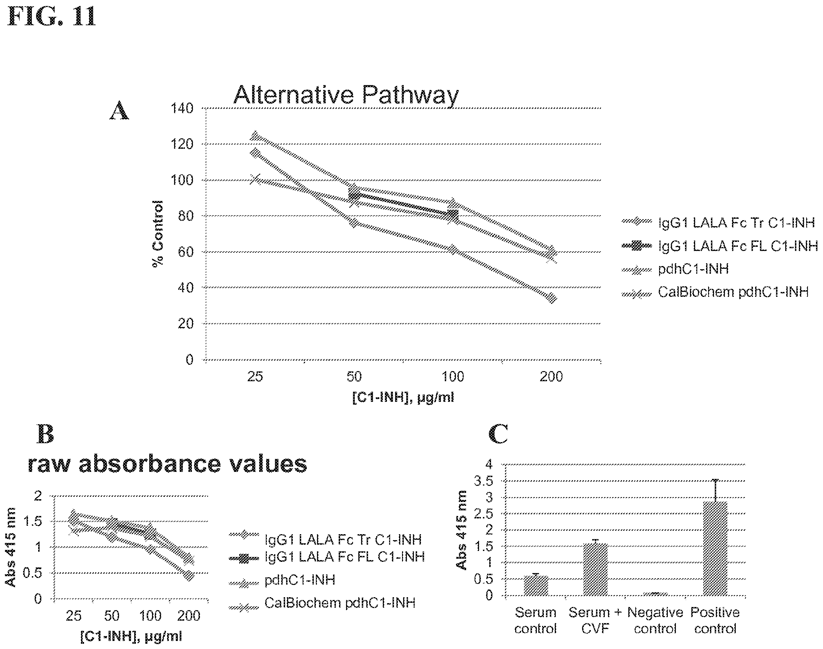

FIGs. 11A, 11B, and 11C show the results of a hemolysis assay to measure the activation of the alternative pathway of complement. FIG. 11A shows the results comparing truncated (Tr) C1-INH hFc IgG1 LALA fusion protein, full length (FL) C1-INH hFc IgG1 LALA fusion protein , a plasma-derived C1-INH preparation, and CalBiochem commercially available plasma-derived C1-INH. The absorbance readings are plotted as a percentage of the control by the concentration of the tested proteins. FIG. 11B shows the plot of raw data collected for each of the samples. FIG. 11C shows the plot of the controls run in the assay.

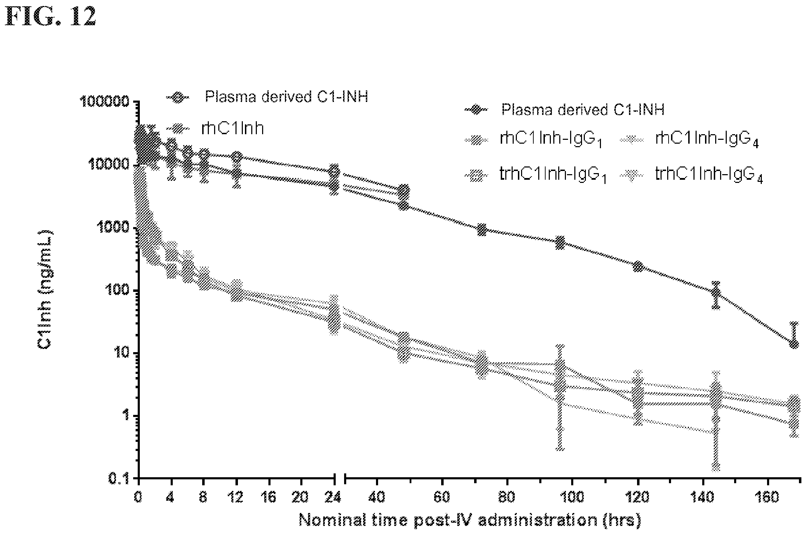

FIG. 12 shows exemplary results of a Rabbit PK study of the effector dead fusion constructs compared with two preparations of plasma-derived C1-inhibitor and HT1080 expressed recombinant C1-INH. IV administration of plasma-derived C1-INH exhibits a monophasic serum concentration-time profile.

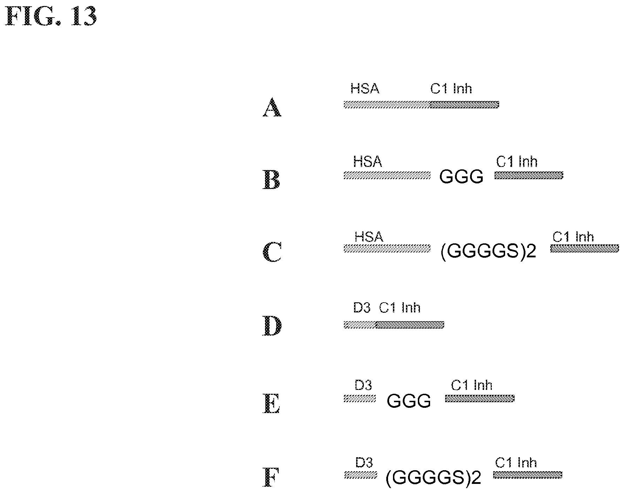

FIGs. 13A-13F are schematic representations of exemplary albumin fusion constructs generated. FIG. 13A is a schematic representation of a human serum albumin (HSA) fused to C1-INH. FIG. 13B is a schematic representation of HSA joined to C1-INH via a GGG linker. FIG. 13C is a schematic representation of HSA joined to C1-INH via a (GGGGS)2 linker. FIG. 13D is a schematic representation of the D3 domain of HSA fused to C1-INH. FIG. 13E is a schematic representation of the D3 domain of HSA joined to C1-INH via a GGG linker. FIG. 13F is a schematic representation of the D3 domain of HSA joined to C1-INH via a (GGGGS)2 linker.

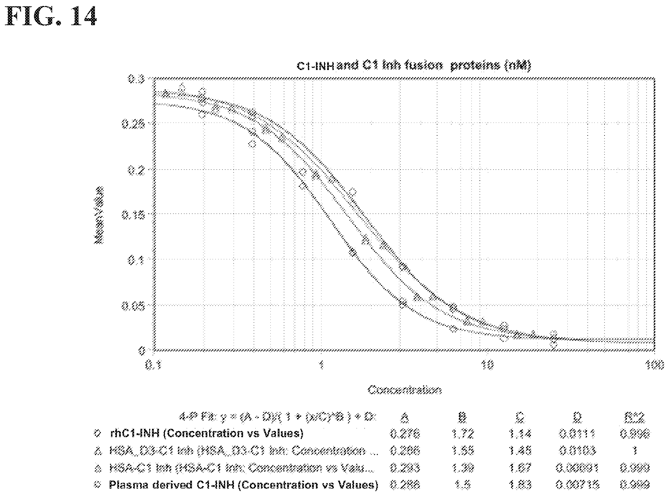

FIG. 14 present the results of an assay to measure the ability of some exemplary albumin C1-INH fusion constructs to inhibit C1s cleavage of a colorimetric peptide, including a plasma-derived C1-INH and HT1080 expressed recombinant C1-INH for comparison.

### DEFINITIONS

In order for the present invention to be more readily understood, certain terms are first defined below. Additional definitions for the following terms and other terms are set forth throughout the specification.
*Animal:* As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e.g.,* a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.
*Approximately or about:* As used in this application, the terms "about" and "approximately" are used as equivalents. Any numerals used in this application with or without about/approximately are meant to cover any normal fluctuations appreciated by one of ordinary skill in the relevant art. As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).
*Bioavailability*: As used herein, the term "bioavailability" generally refers to the percentage of the administered dose that reaches the blood stream of a subject.
*Biologically active:* As used herein, the phrase "biologically active" refers to a characteristic of any agent that has activity in a biological system, and particularly in an organism. For instance, an agent that, when administered to an organism, has a biological effect on that organism, is considered to be biologically active. In particular embodiments, where a peptide is biologically active, a portion of that peptide that shares at least one biological activity of the peptide is typically referred to as a "biologically active" portion.
*Carrier or diluent:* As used herein, the terms "carrier" and "diluent" refers to a pharmaceutically acceptable (e.g., safe and non-toxic for administration to a human) carrier or diluting substance useful for the preparation of a pharmaceutical formulation. Exemplary diluents include sterile water, bacteriostatic water for injection (BWFI), a pH buffered solution (e.g. phosphate-buffered saline), sterile saline solution, Ringer's solution or dextrose solution.
*C1-inhibitor or C1 esterase inhibitor or C1-INH:* As used herein, the term "C1-inhibitor" or "C1 esterase inhibitor" or "C1-INH" can all be used interchangeably and refer to any wild-type or modified C1-INH polypeptides (e.g., C1-INH proteins with amino acid mutations, deletions, insertions, and/or fusion proteins) that retain substantial C1-INH biological activity unless otherwise specified. C1-INH may be recombinantly expressed in cells. In certain embodiments, the C1-INH is expressed in mammalian cells, preferably CHO cells or human cells.
*Functional equivalent or derivative:* As used herein, the term "functional equivalent" or "functional derivative" denotes, in the context of a functional derivative of an amino acid sequence, a molecule that retains a biological activity (either function or structural) that is substantially similar to that of the original sequence. A functional derivative or equivalent may be a natural derivative or is prepared synthetically. Exemplary functional derivatives include amino acid sequences having substitutions, deletions, or additions of one or more amino acids, provided that the biological activity of the protein is conserved. The substituting amino acid desirably has chemico-physical properties which are similar to that of the substituted amino acid. Desirable similar chemico-physical properties include, similarities in charge, bulkiness, hydrophobicity, hydrophilicity, and the like.
*Fusion protein:* As used herein, the term "fusion protein" or "chimeric protein" refers to a protein created through the joining of two or more originally separate proteins, or portions thereof. In some embodiments, a linker or spacer will be present between each protein.
*Half-Life:* As used herein, the term "half-life" is the time required for a quantity such as protein concentration or activity to fall to half of its value as measured at the beginning of a time period.
*Hereditary angioedema or HAE:* As used herein, the term "hereditary angioedema" or "HAE" refers to a blood disorder characterized by unpredictable and recurrent attacks of inflammation. HAE is typically associated with C1-INH deficiency, which may be the result of low levels of C1-INH or C1-INH with impaired or decreased activity. Symptoms include, but are not limited to, swelling that can occur in any part of the body, such as the face, extremities, genitals, gastrointestinal tract and upper airways.
*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.
*In Vitro:* As used herein, the term *"in vitro"* refers to events that occur in an artificial environment, *e.g.,* in a test tube or reaction vessel, in cell culture, *etc.,* rather than within a multi-cellular organism.
*In Vivo*: As used herein, the term *"in vivo"* refers to events that occur within a multi-cellular organism, such as a human and a non-human animal. In the context of cell-based systems, the term may be used to refer to events that occur within a living cell (as opposed to, for example, *in vitro* systems).
*Linker:* As used herein, the term "linker" refers to, in a fusion protein, an amino acid sequence other than that appearing at a particular position in the natural protein and is generally designed to be flexible or to interpose a structure, such as an α-helix, between two protein moieties. A linker is also referred to as a spacer. A linker or a spacer typically does not have biological function on its own.
*Polypeptide:* The term "polypeptide" as used herein refers to a sequential chain of amino acids linked together via peptide bonds. The term is used to refer to an amino acid chain of any length, but one of ordinary skill in the art will understand that the term is not limited to lengthy chains and can refer to a minimal chain comprising two amino acids linked together via a peptide bond. As is known to those skilled in the art, polypeptides may be processed and/or modified. As used herein, the terms "polypeptide" and "peptide" are used inter-changeably.
*Prevent:* As used herein, the term "prevent" or "prevention", when used in connection with the occurrence of a disease, disorder, and/or condition, refers to reducing the risk of developing the disease, disorder and/or condition. See the definition of "risk."
*Protein:* The term "protein" as used herein refers to one or more polypeptides that function as a discrete unit. If a single polypeptide is the discrete functioning unit and does not require permanent or temporary physical association with other polypeptides in order to form the discrete functioning unit, the terms "polypeptide" and "protein" may be used interchangeably. If the discrete functional unit is comprised of more than one polypeptide that physically associate with one another, the term "protein" refers to the multiple polypeptides that are physically coupled and function together as the discrete unit.
*Risk:* As will be understood from context, a "risk" of a disease, disorder, and/or condition comprises a likelihood that a particular individual will develop a disease, disorder, and/or condition (*e.g.,* muscular dystrophy). In some embodiments, risk is expressed as a percentage. In some embodiments, risk is from 0,1, 2, 3, 4, 5, 6, 7, 8, 9, 10, 20, 30, 40, 50, 60, 70, 80, 90 up to 100%. In some embodiments risk is expressed as a risk relative to a risk associated with a reference sample or group of reference samples. In some embodiments, a reference sample or group of reference samples have a known risk of a disease, disorder, condition and/or event (e.g., muscular dystrophy). In some embodiments a reference sample or group of reference samples are from individuals comparable to a particular individual. In some embodiments, relative risk is 0, 1, 2, 3, 4, 5, 6, 7, 8, 9, 10, or more.
*Subject:* As used herein, the term "subject" refers to a human or any non-human animal (e.g., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.
*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.
*Substantial homology:* The phrase "substantial homology" is used herein to refer to a comparison between amino acid or nucleic acid sequences. As will be appreciated by those of ordinary skill in the art, two sequences are generally considered to be "substantially homologous" if they contain homologous residues in corresponding positions. Homologous residues may be identical residues. Alternatively, homologous residues may be non-identical residues will appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains Substitution of one amino acid for another of the same type may often be considered a "homologous" substitution.
   As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology*;* Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology. In some embodiments, two sequences are considered to be substantially homologous if at least 50%, 55%, 60%, 65%, 70%, 75%, 80%, 85%, 90%, 91%, 92%, 93%, 94%, 95%, 96%, 97%, 98%, 99% or more of their corresponding residues are homologous over a relevant stretch of residues. In some embodiments, the relevant stretch is a complete sequence. In some embodiments, the relevant stretch is at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more residues.
*Substantial identity:* The phrase "substantial identity" is used herein to refer to a comparison between amino acid or nucleic acid sequences. As will be appreciated by those of ordinary skill in the art, two sequences are generally considered to be "substantially identical" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology*;* Altschul et al., Nucleic Acids Res. 25:3389-3402, 1997; Baxevanis et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying identical sequences, the programs mentioned above typically provide an indication of the degree of identity. In some embodiments, two sequences are considered to be substantially identical if at least 50%, 55%, 60%, 65%, 70%, 75%, 80%, 85%, 90%, 91%, 92%, 93%, 94%, 95%, 96%, 97%, 98%, 99% or more of their corresponding residues are identical over a relevant stretch of residues. In some embodiments, the relevant stretch is a complete sequence. In some embodiments, the relevant stretch is at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more residues.
*Suffering from:* An individual who is "suffering from" a disease, disorder, and/or condition has been diagnosed with or displays one or more symptoms of the disease, disorder, and/or condition.
*Susceptible to:* An individual who is "susceptible to" a disease, disorder, and/or condition has not been diagnosed with the disease, disorder, and/or condition. In some embodiments, an individual who is susceptible to a disease, disorder, and/or condition may not exhibit symptoms of the disease, disorder, and/or condition. In some embodiments, an individual who is susceptible to a disease, disorder, condition, or event (for example, DMD) may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein associated with the disease, disorder, and/or condition; (4) habits and/or lifestyles associated with development of the disease, disorder, condition, and/or event (5) having undergone, planning to undergo, or requiring a transplant. In some embodiments, an individual who is susceptible to a disease, disorder, and/or condition will develop the disease, disorder, and/or condition. In some embodiments, an individual who is susceptible to a disease, disorder, and/or condition will not develop the disease, disorder, and/or condition.
*Therapeutically effective amount:* As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.
*Treating:* As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

### DETAILED DESCRIPTION OF CERTAIN EMBODIMENTS

The present invention provides, among other things, methods and compositions for treating complement-mediated disorders, including Hereditary angioedema (HAE), based on C1-INH as a protein therapeutic.

Purpose of fusion is to extend half-life. Result is decrease in dose frequency, increase in prophylactic efficacy.

Various aspects of the invention are described in detail in the following sections. The use of sections is not meant to limit the invention. Each section can apply to any aspect of the invention. In this application, the use of "or" means "and/or" unless stated otherwise. The disclosures of all of the art cited herein are incorporated by reference in their entirety.

### C1-INH

Human C1-INH is an important anti-inflammatory plasma protein with a wide range of inhibitory and non-inhibitory biological activities. By sequence homology, structure of its C-terminal domain, and mechanism of protease inhibition, it belongs to the serpin superfamily, the largest class of plasma protease inhibitors, which also includes antithrombin, α1-proteinase inhibitor, plasminogen activator inhibitor, and many other structurally similar proteins that regulate diverse physiological systems. C1-INH is an inhibitor of proteases in the complement system, the contact system of kinin generation, and the intrinsic coagulation pathway. Cai, S. & Davis, A. E., Complement Regulatory Protein C1 Inhibitor Binds to Selectins and Interferes with Endothelial-Leukocyte Adhesion, J Immunol, 171:4786-4791 (2003). Specifically, C1-INH has been shown to inhibit C1r and C1s of the complement system. C1-INH is also a major regulator of coagulation factors XI and XII, as well as kallikrein and other serine proteases of the coagulation and fibrinolytic systems including tissue type plasminogen activator and plasmin.

Low plasma content of C1-INH or its dysfunction result in the activation of both complement and contact plasma cascades, and may affect other systems as well. A decrease in C1-INH plasma content to levels lower than 55 µg/mL (∼25% of normal) has been shown to induce spontaneous activation of C1.

A schematic depicting the structure of C1-INH is provided in FIG. 1A. The signal peptide, N-terminal domain, and serpin domain are shown. C1-INH is The 22 amino acid signal peptide is required for secretion and cleaved from the rest of the C1-INH protein. C1-INH has two domains: a C-terminal domain having 365 amino acids, which is a typical serpin domain, and an N-terminal domain having 113 amino acids. The protein is stabilized by two disulfide bridges which connect the domains. These disulfide bridges are formed by Cys101 of the N-terminal domain which forms a disulfide bond with Cys406 of the C-terminal (serpin) domain and Cys108 of the N-terminal domain which forms a disulfide bond with Cys183 of C-terminal domain. The serpin domain is responsible for the protease activity of C1-INH. P1-P1' denotes the Arg444-Thr445 scissile bond.

More than 26% of the weight of the glycosylated protein is carbohydrate. The glycans are unevenly distributed over human C1-INH. The N-terminus is heavily glycosylated, having three N-linked (shown as long vertical lines with diamond heads) and at least seven O-linked (shown as short vertical lines) carbohydrate groups. Three N-attached glycans are attached to asparagine residues Asn216, Asn231, and Asn330 in the serpin domain (shown as long vertical lines with diamond heads). Although the functional role of the exceptionally long and heavily glycosylated N-terminal domain is still unclear, it may be essential for the protein's conformational stability, recognition, affinity to endotoxins and selectins, and clearance. The intrinsic heterogeneity of the carbohydrate moiety greatly contributes to the heterogeneity of the whole C1-INH, one of the reasons why production of a recombinant C1-INH mimicking the properties of plasma-derived C1-INH is difficult.

As used herein, C1-INH fusion proteins suitable for the present invention comprise any wild-type and modified C1-INH polypeptides (e.g., C1-INH proteins with amino acid mutations, deletions, truncations, and/or insertions) that retain substantial C1-INH biological activity. Typically, a C1-INH fusion protein is produced using recombinant technology.

Typically, a suitable recombinant C1-INH fusion protein has an *in vivo* half-life of or greater than about 12 hours, 18 hours, 24 hours, 36 hours, 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, or 10 days. In some embodiments, a recombinant C1-INH fusion protein has an *in vivo* half-life of between 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

Typically, a suitable recombinant C1-INH fusion protein has an *in vivo* half-life of or greater than about 4 days, 4.5 days, 5 days, 5.5 days, 6 days, 6.5 days, 7 days, 7.5 days, 8 days, 8.5 days, 9 days, 9.5 days, or 10 days. In some embodiments, a recombinant C1-INH fusion protein has an *in vivo* half-life of between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

In some embodiments, a recombinant C1-INH polypeptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the wild-type human C1-INH protein (amino acids 1-478) (amino acids 1-97 are underlined):

NPNATSSSSQDPESLQDRGEGKVATTVISKMLFVEPILEVSSLPTTNSTTNSATKITANTT
DEPTTQPTTEPTTQPTIQPTQPTTQLPTDSPTQPTTGSFCPGPVTLCSDLESHSTEAVLGDA
LVDFSLKLYHAFSAMKKVETNMAFSPFSIASLLTQVLLGAGENTKTNLESILSYPKDFTC
VHQALKGFTTKGVTSVSQIFHSPDLAIRDTFVNASRTLYSSSPRVLSNNSDANLELINTW
VAKNTNNKISRLLDSLPSDTRLVLLNAIYLSAKWKTTFDPKKTRMEPFHFKNSVIKVPM
MNSKKYPVAHFIDQTLKAKVGQLQLSHNLSLVILVPQNLKHRLEDMEQALSPSVFKAIM
EKLEMSKFQPTLLTLPRIKVTTSQDMLSIMEKLEFFDFSYDLNLCGLTEDPDLQVSAMQH
QTVLELTETGVEAAAASAISVARTLLVFEVQQPFLFVLWDQQHKFPVFMGRVYDPRA
(SEQ ID NO:1).

In some embodiments, a recombinant C1-INH polypeptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the wild-type human C1-INH protein (amino acids 98-478):

GSFCPGPVTLCSDLESHSTEAVLGDALVDFSLKLYHAFSAMKKVETNMAFSPFSIASLLT
QVLLGAGENTKTNLESILSYPKDFTCVHQALKGFTTKGVTSVSQIFHSPDLAIRDTFVNA
SRTLYSSSPRVLSNNSDANLELINTWVAKNTNNKISRLLDSLPSDTRLVLLNAIYLSAKW
KTTFDPKKTRMEPFHFKNSVIKVPMMNSKKYPVAHFIDQTLKAKVGQLQLSHNLSLVIL
VPQNLKHRLEDMEQALSPSVFKAIMEKLEMSKFQPTLLTLPRIKVTTSQDMLSIMEKLEF
FDFSYDLNLCGLTEDPDLQVSAMQHQTVLELTETGVEAAAASAISVARTLLVFEVQQPF
LFVLWDQQHKFPVFMGRVYDPRA (SEQ ID NO:2).

As disclosed herein, SEQ ID NO:1 represents the canonical amino acid sequence for the human C1-INH protein. In some embodiments, a C1-INH polypeptide may be a truncated C1-INH such as SEQ ID NO:2. In some embodiments, a suitable recombinant C1-INH polypeptide may be a homologue or an analogue of a wild-type or naturally-occurring protein. For example, a homologue or an analogue of human wild-type or naturally-occurring C1-INH polypeptide may contain one or more amino acid or domain substitutions, deletions, and/or insertions as compared to a wild-type or naturally-occurring C1-INH protein (e.g., SEQ ID NO:1), while retaining substantial C1-INH protein activity. Thus, in some embodiments, a recombinant C1-INH polypeptide suitable for the present invention is substantially homologous to human C1-INH protein (SEQ ID NO:1). In some embodiments, a recombinant C1-INH polypeptide suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to SEQ ID NO:1. In some embodiments, a recombinant C1-INH polypeptide suitable for the present invention is substantially identical to human C1-INH protein (SEQ ID NO:1). In some embodiments, a recombinant C1-INH polypeptide suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to SEQ ID NO:1.

Homologues or analogues of human C1-INH proteins can be prepared according to methods for altering polypeptide sequence known to one of ordinary skill in the art such as are found in references that compile such methods. As will be appreciated by those of ordinary skill in the art, two sequences are generally considered to be "substantially homologous" if they contain homologous residues in corresponding positions. Homologous residues may be identical residues. Alternatively, homologous residues may be non-identical residues will appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains. Substitution of one amino acid for another of the same type may often be considered a "homologous" substitution. In some embodiments, conservative substitutions of amino acids include substitutions made among amino acids within the following groups: (a) M, I, L, V; (b) F, Y, W; (c) K, R, H; (d) A, G; (e) S, T; (f) Q, N; and (g) E, D. In some embodiments, a "conservative amino acid substitution" refers to an amino acid substitution that does not alter the relative charge or size characteristics of the protein in which the amino acid substitution is made.

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology*;* Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology.

In some embodiments, a recombinant C1-INH polypeptide suitable for the present invention contains one or more amino acid deletions, insertions or replacement as compared to a wild-type human C1-INH protein. For example, a suitable recombinant C1-INH polypeptide may be truncated, such as the polypeptide of SEQ ID NO:2.

In some embodiments, a recombinant C1-INH polypeptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the truncated wild-type human C1-INH protein (amino acids 98-478):

GSFCPGPVTLCSDLESHSTEAVLGDALVDFSLKLYHAFSAMKKVETNMAFSPFSIASLLT
QVLLGAGENTKTNLESILSYPKDFTCVHQALKGFTTKGVTSVSQIFHSPDLAIRDTFVNA
SRTLYSSSPRVLSNNSDANLELINTWVAKNTNNKISRLLDSLPSDTRLVLLNAIYLSAKW
KTTFDPKKTRMEPFHFKNSVIKVPMMNSKKYPVAHFIDQTLKAKVGQLQLSHNLSLVIL
VPQNLKHRLEDMEQALSPSVFKAIMEKLEMSKFQPTLLTLPRIKVTTSQDMLSIMEKLEF
FDFSYDLNLCGLTEDPDLQVSAMQHQTVLELTETGVEAAAASAISVARTLLVFEVQQPF
LFVLWDQQHKFPVFMGRVYDPRA (SEQ ID NO:2).

In some embodiments, a C1-INH polypeptide may be a truncated C1-INH while retaining substantial C1-INH protein activity, such as SEQ ID NO:2. Thus, in some embodiments, a recombinant C1-INH polypeptide suitable for the present invention is substantially homologous to human C1-INH protein (SEQ ID NO:2). In some embodiments, a recombinant C1-INH polypeptide suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to SEQ ID NO:2. In some embodiments, a recombinant C1-INH polypeptide suitable for the present invention is substantially identical to human C1-INH protein (SEQ ID NO:2). In some embodiments, a recombinant C1-INH polypeptide suitable for the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to SEQ ID NO:2.

The recombinant C1-INH fusion protein suitable for the present invention is a fusion protein between a C1-INH domain and another domain or moiety that typically can facilitate a therapeutic effect of C1-INH by, for example, enhancing or increasing half-life, stability, potency, and/or delivery of C1-INH protein, or reducing or eliminating immunogenicity, clearance, or toxicity. Such suitable domains or moieties for a C1-INH fusion protein include but are not limited to Fc domains and albumin domains.

### Fc Domains

In some embodiments, a suitable C1-INH fusion protein contains an Fc domain or a portion thereof that binds to the FcRn receptor. As a non-limiting example, a suitable Fc domain may be derived from an immunoglobulin subclass such as IgG. In some embodiments, a suitable Fc domain is derived from IgG1, IgG2, IgG3, or IgG4. In some embodiments, a suitable Fc domain is derived from IgM, IgA, IgD, or IgE. Particularly suitable Fc domains include those derived from human or humanized antibodies. In some embodiments, a suitable Fc domain is a modified Fc portion, such as a modified human Fc portion.

### A. IgG1

C1-inhibitor Fc fusion proteins may exist as dimers, as shown in FIG. 1A.

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the wild-type human IgG1 Fc domain:

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO:3).

### i. LALA

Wild type IgG1 has a low level of complement cascade activation, any may undesirable for patients suffering from a complement-mediated disorder. IgG choice for effector dead constructs which reduce or eliminate complement activation and antibody-dependent cell-mediated cytotoxicity (ADCC) activity may be important. Suitable Fc domains include IgG1 with mutations of L234A and L235A (LALA).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain having a LALA mutation (mutated residues underlined):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO:4).

### ii. NHance

It is contemplated that improved binding between Fc domain and the FcRn receptor results in prolonged scrum half-life. Thus, in some embodiments, a suitable Fc domain comprises one or more amino acid mutations that lead to improved binding to FcRn. Various mutations within the Fc domain that effect improved binding to FcRn are known in the art and can be adapted to practice the present invention. In some embodiments, a suitable Fc domain comprises one or more mutations at one or more positions corresponding to Thr 250, Met 252, Ser 254, Thr 256, Thr 307, Glu 380, Met 428, His 433, and/or Asn 434 of human IgG1.

For example, a suitable Fc domain may contain mutations of H433K (His433Lys) and/or N434F (Asn434Phe). As a non-limiting example, a suitable Fc domain may contain mutations H433K (His433Lys) and N434F (Asn434Phe) (Nhance). Additional amino acid substitutions that can be included in a Fc domain include those described in, e.g., U.S. Patent Nos. 6,277,375; 8,012,476; and 8,163,881, which are incorporated herein by reference.

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain having Nhance mutation (mutated residues underlined):

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP

VLDSDGSFFLYSKLTVDKSRWQQGNVFSCSVMHEALKFHYTQKSLSLSPGK (SEQ ID
NO:5).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain having both LALA and Nhance mutations (mutated residues underlined):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALKFHYTQKSLSLSPGK (SEQ ID
NO:6).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain with a signal peptide, and having Nhance mutation (signal peptide and mutated residues underlined):

METPAQLLFLLLLWLPDTTGDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHEALKFH
YTQKSLSLSPGK (SEQ ID NO:7).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to a human IgG1 Fc domain with a signal peptide, and having both LALA and Nhance mutations (mutated residues underlined):

METPAQLLFLLLLWLPDTTGDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALKF
HYTQKSLSLSPGK (SEQ ID NO:8).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the wild-type human IgG4 Fc domain:

ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP

VLDSDGSFFLYSRLTVDKSRWQEGNVFSCSVMHEALHNHYTQKSLSLSLGK (SEQ ID
NO:9).

In some embodiments, an Fc domain suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the human IgG4 Fc domain having an S241P mutation (mutated residue underlined):

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGK (SEQ ID
NO: 10).

In some embodiments, a suitable Fc domain comprises an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to SEQ ID NO:3, SEQ ID NO:4, or SEQ ID NO:5, SEQ ID NO:6, SEQ ID NO:7, SEQ ID NO:8, SEQ ID NO:9, or SEQ ID NO:10.

### B. IgG4

In some embodiments IgG4 IgG choice for "effector dead" Fc fusion proteins is important in HAE (eliminate complement activation and ADCC). Specifically, IgG4 is reported to have lower complement activation than WT IgG1. Expression of native IgG4 results in mixed species of construct sizing. Accordingly, IgG4 S241P was chosen for this program due to the increased stability and maintenance of dimeric structure exhibited by IgG4 S241P mutants. based on prior art.

The human IgG4 core hinge region sequence according to the invention preferably comprises an S228P substitution according to the EU index as in Kabat. This substitution has also been referred to as S241P according to Kabat et al (1987 Sequences of proteins of immunological interest. United States Department of Health and Human Services, Washington DC.). The substitution has the effect of making the sequence of the core of the hinge region the same as that of a Wild-type IgG1 or IgG2 isotype antibody. With respect to the IgG4 isotype antibody, it results in the production of the homogenous form of the IgG4 antibody and hence abrogates the dissociation and reassociation of the heavy chains which often leads to the production of heterodimeric IgG4 antibodies. In some embodiments, IgG4 is preferred for stability at high concentrations.

### C. Exemplary CI-INH Fusion Proteins

In particular embodiments, a suitable recombinant C1-INH fusion protein includes a C1-INH polypeptide, an Fc domain. wherein the C1-INH polypeptide comprises an amino acid sequence at least 50%, 55%, 60%, 65%, 70%, 75%, 80%, 85%, 90%, 91%, 92%, 93%, 94%, 95%, 96%, 97%, 98%, 99% or 100% identical to the wild-type human C1-INH polypeptide (SEQ ID NO:1) or a truncated C1-INH polypeptide (SEQ ID NO:2). In some embodiments there is a linker that associates the C1-INH polypeptide with the Fc domain. In some embodiments, a suitable recombinant C1-INH fusion protein has an *in vivo* half-life ranging from about 0.5-10 days (e.g., about 0.5-5.5 days, about 0.5-5 days, about 1-5 days, about 1.5-5 days, about 1.5-4.5 days, about 1.5-4.0 days, about 1.5-3.5 days, about 1.5-3 days, about 1.5-2.5 days, about 2-6 days, about 2-5.5 days, about 2-5 days, about 2-4.5 days, about 2-4 days, about 2-3.5 days, about 2-3 days). In some embodiments, a suitable recombinant C1-INH fusion protein has an *in vivo* half-life ranging from about 2-10 days (e.g., ranging from about 2.5-10 days, from about 3-10 days, from about 3.5-10 days, from about 4-10 days, from about 4.5-10 days, from about 5-10 days, from about 3-8 days, from about 3.5-8 days, from about 4-8 days, from about 4.5-8 days, from about 5-8 days, from about 3-6 days, from about 3.5-6 days, from about 4-6 days, from about 4.5-6 days, from about 5-6 days).

Typically, a suitable recombinant C1-INH fusion protein has an *in vivo* half-life of or greater than about 12 hours, 18 hours, 24 hours, 36 hours, 2 days, 2.5 days, 3 days, 3.5 days, 4 days, 4.5 days, 5 days, 5.5 days, 6 days, 6.5 days, 7 days, 7.5 days, 8 days, 8.5 days, 9 days, 9.5 days, or 10 days. In some embodiments, a recombinant C1-INH fusion protein has an *in vivo* half-life of between 0.5 and 10 days, between 1 day and 10 days, between 1 day and 9 days, between 1 day and 8 days, between 1 day and 7 days, between 1 day and 6 days, between 1 day and 5 days, between 1 day and 4 days, between 1 day and 3 days, between 2 days and 10 days, between 2 days and 9 days, between 2 days and 8 days, between 2 days and 7 days, between 2 days and 6 days, between 2 days and 5 days, between 2 days and 4 days, between 2 day and 3 days, between 2.5 days and 10 days, between 2.5 days and 9 days, between 2.5 days and 8 days, between 2.5 days and 7 days, between 2.5 days and 6 days, between 2.5 days and 5 days, between 2.5 days and 4 days, between 3 days and 10 days, between 3 days and 9 days, between 3 days and 8 days, between 3 days and 7 days, between 3 days and 6 days, between 3 days and 5 days, between 3 days and 4 days, between 3.5 days and 10 days, between 3.5 days and 9 days, between 3.5 days and 8 days, between 3.5 days and 7 days, between 3.5 days and 6 days, between 3.5 days and 5 days, between 3.5 days and 4 days, between 4 days and 10 days, between 4 days and 9 days, between 4 days and 8 days, between 4 days and 7 days, between 4 days and 6 days, between 4 days and 5 days, between 4.5 days and 10 days, between 4.5 days and 9 days, between 4.5 days and 8 days, between 4.5 days and 7 days, between 4.5 days and 6 days, between 4.5 days and 5 days, between 5 days and 10 days, between 5 days and 9 days, between 5 days and 8 days, between 5 days and 7 days, between 5 days and 6 days, between 5.5 days and 10 days, between 5.5 days and 9 days, between 5.5 days and 8 days, between 5.5 days and 7 days, between 5.5 days and 6 days, between 6 days and 10 days, between 7 days and 10 days, between 8 days and 10 days, between 9 days and 10 days.

In certain embodiments, as shown in FIG. 2A and FIG. 2B, Fc moieties may be directly fused to the N-terminal region of the full length (1-478 aa) as well as truncated (98-478) C1-inhibitor. As non-limiting examples, suitable C1-INH Fc fusion proteins may have an amino acid sequence shown below: 
DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:11)
 or 
DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
 
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:12)
 or 
DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:13)
 or 
DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:14)
 or 
ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
 
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:32)
 or 
ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:33)
 or 
ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:15)
 or

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN

LCGLTEDPDLQVSAMQHQTVLELTETGVEAAAASAISVARTLLVFEVQQPFLFVLWDQ
QHKFPVFMGRVYDPRA (SEQ ID NO:16).

In some embodiments, a suitable recombinant C1-INH Fc fusion protein has an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to SEQ ID NO:11, SEQ ID NO:12, SEQ ID NO:13, SEQ ID NO:14, SEQ ID NO:15, SEQ ID NO:16, SEQ ID NO:32, or SEQ ID NO:33.

It is contemplated that a C1-INH-Fc fusion protein may be provided in various configurations including homodimeric or monomeric configurations. For example, a suitable homodimeric configuration may be designed to have the C-terminal end of fusion partner (e.g., a C1-INH polypeptide plus linker) attached to the N-terminal end of both Fc polypeptide strands. A suitable monomeric configuration may be designed to have the C-terminal end of fusion partner (e.g., a C1-INH polypeptide plus linker) fused to one Fc dimer.

Monomeric, also referred to herein as monovalent, forms may be preferred for certain applications and routes of administration, e.g., subcutaneous administration. A monomeric configuration may decrease steric hindrance, increase half-life, and/or may increase bioavailability.

Monovalent forms may also be preferred for C1-INH Fc fusion constructs because C1-INH is a suicide inhibitor. Since it is a suicide inhibitor, the binding of one C1-INH "arm" of a dimer Fc fusion will result in increased rate of clearance of the bound C1-INH fusion protein, even in the event that a second arm remain unbound.

An advantage of the Fc fusion proteins, both monomeric and dimeric, is that Fc expression was found to occur at higher levels than expression of C1-INH alone. Activity assays comparing the dimeric C1-INH Fc constructs with recombinant C1-INH have been shown to have similar C1q binding activity. The inclusion of a linker was also tested and found not to affect the ability of Fc-C1-INH fusion protein to bind its target.

Methods of making monomeric antibody fusion proteins include those described in, e.g., PCT Publication Nos. WO2011/063348; WO2012/020096; WO2013/138643; WO2014087299; Dumont, J. et al., Monomeric Fc Fusions: Impact on Pharmacokinetic and Biological Activity of Protein Therapeutics, Biodrugs, 20(3): 151-160 (2006); Ishino, T. et al, Protein Structure and Folding: Half-life Extension of Biotherapeutics Modality by N-Glycosylation for the Engineering a Monomeric Fc Domain, J. Biol. Chem., 288:16529-16537 (2013), the disclosures of which are incorporated herein by reference.

Monovalent C1-inhibitor can be made by using a plasmid containing the Fc-C1 co transfected with a plasmid expressing Fc alone. In addition, it could be made by using a dual promoter plasmid with one promoter generating Fc-C1 and the other promoter generating Fc alone. Monovalent Fc could also be made using bispecific technology where specific amino acids in the hinge region of the Fc are mutated to impart stability of the Fc region (e.g. Knob and hole technology or other stabilizing mutations which drive formation of the monovalent C1).

As used herein, "percent (%) amino acid sequence identity" with respect to a reference protein sequence (e.g., a reference C1-INH protein sequence) identified herein is defined as the percentage of amino acid residues in a candidate sequence that are identical with the amino acid residues in the reference sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared. Preferably, the WU-BLAST-2 software is used to determine amino acid sequence identity (Altschul et al., Methods in Enzymology 266, 460-480 (1996); http://blast.wustl/edu/blast/README.html). WU-BLAST-2 uses several search parameters, most of which are set to the default values. The adjustable parameters are set with the following values: overlap span=1, overlap fraction=0.125, world threshold (T)=11. HSP score (S) and HSP S2 parameters are dynamic values and are established by the program itself, depending upon the composition of the particular sequence, however, the minimum values may be adjusted and are set as indicated above.

### Albumin Domains

Albumin is a soluble, monomeric protein which comprises about one-half of the blood serum protein. Albumin functions primarily as a carrier protein for steroids, fatty acids, and thyroid hormones and plays a role in stabilizing extracellular fluid volume. Alubumin has a globular unglycosylated serum protein of molecular weight 66,500. Albumin is synthesized in the liver as preproalbumin which has an N-terminal peptide that is removed before the nascent protein is released from the rough endoplasmic reticulum. The product, proalbumin, is in turn cleaved in the Golgi vesicles to produce the secreted albumin.

Albumin is made up of three homologous domains (I-III), and each of these is comprised of two subdomains (A and B). The principal regions of ligand binding to human serum albumin are located in cavities in subdomains IIA and IIIA, which are formed mostly of hydrophobic and positively charged residues and exhibit similar chemistry. Human serum albumin has 585 amino acids and a molecular mass of 66,500 Da. The amino acids include 35 cysteines, all but one of which are involved in the formation of 17 stabilizing disulfide bonds.

Albumin has a prolonged serum half-life of 19 days. FcRn controls the long scrum half-life of albumin. FcRn is a dual binding receptor that, in addition to albumin, binds IgG, and protects both proteins from intracellular degradation. The C-terminal domain of the albumin molecule has been shown to be crucial for binding to FcRn. Lack of domain IIIB or mutations of 464His, 510His, and 535His almost completely abolish FcRn binding.

Albumin fusion proteins of the invention are monomeric. In some embodiments, this feature may be an advantage over the dimeric Fc fusion embodiments for the reasons described above with regard to monomeric Fc fusion embodiments.

In some embodiments, an albumin polypeptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the wild-type human serum albumin:












 (SEQ ID NO:17).

In some embodiments, an albumin polypeptide suitable for the present invention includes an amino acid sequence at least 50% (e.g., at least 55%, 60%, 65%, 70%, 75%, 80%, 85%, 90%, 95%, 96%, 97%, 98%, 99%, or 100%) identical to the D3 domain of wild-type human serum albumin:

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGL (SEQ ID
NO:20).

### Linker or Spacer

A C1-INH domain may be directly or indirectly linked to an Fc domain or an albumin domain. In some embodiments, a suitable recombinant C1-INH fusion protein contains a linker or spacer that joins a C1-INH polypeptide and an Fc domain. In some embodiments, a suitable recombinant C1-INH fusion protein contains a linker or spacer that joins a C1-INH polypeptide and an albumin polypeptide. An amino acid linker or spacer is generally designed to be flexible or to interpose a structure, such as an alpha-helix, between the two protein moieties. A linker or spacer can be relatively short, or can be longer. Typically, a linker or spacer contains for example 3-100 (e.g., 5-100, 10-100, 20-100 30-100, 40-100, 50-100, 60-100, 70-100, 80-100, 90-100, 5-55, 10-50, 10-45, 10-40, 10-35, 10-30, 10-25, 10-20) amino acids in length. In some embodiments, a linker or spacer is equal to or longer than 2, 3, 4, 5, 6, 7, 8, 9, 10, 15, 20, 25, 30, 35, 40, 45, 50, 55, 60, 65, 70, 75, 80, 85, 90, 95, or 100 amino acids in length. Typically, a longer linker may decrease steric hindrance. In some embodiments, a linker will comprise a mixture of glycine and serine residues. In some embodiments, the linker may additionally comprise threonine, proline, and/or alanine residues. Thus, in some embodiments, the linker comprises between 10-100, 10-90, 10-80, 10-70, 10-60, 10-50, 10-40, 10-30, 10-20, 10-15 amino acids. In some embodiments, the linker comprises at least 10, 15, 20, 25, 30, 35, 40, 45, 50, 55, 60, 65, 70, 75, 80, 85, 90, or 95 amino acids. In some embodiments, the linker is not a linker consisting of ALEVLFQGP.

As non-limiting examples, linkers or spacers suitable for the present invention include but are not limited to GGG linker and GGGGSGGGGS ((GGGGS)2 linker SEQ ID NO:27). In some embodiments, the linker comprises the sequence GGG and/or the sequence of SEQ ID NO:27.

Other suitable linkers include GAPGGGGGAAAAAGGGGGGAP (GAG linker, SEQ ID NO:34); GAPGGGGGAAAAAGGGGGGAPGGGGGAAAAAGGGGGGAP (GAG2 linker, SEQ ID NO:35); and

GAPGGGGGAAAAAGGGGGGAPGGGGGAAAAAGGGGGGAPGGGGGAAAAAGGGGG
GAP (GAG3 linker, SEQ ID NO:36).

Suitable linkers or spacers also include those having an amino acid sequence at least 50%, 55%, 60%, 65%, 70%, 75%, 80%, 85%, 90%, 91%, 92%, 93%, 94%, 95%, 96%, 97%, 98%, 99% or more homologous or identical to the above exemplary linkers, e.g., GGG linker, GGGGSGGGGS ((GGGGS)2 linker SEQ ID NO:27), GAG linker (SEQ ID NO:34), GAG2 linker (SEQ ID NO:35), or GAG3 linker (SEQ ID NO:36). Additional linkers suitable for use with some embodiments may be found in US2012/0232021, filed on March 2, 2012, the disclosure of which is hereby incorporated by reference in its entirety.

In some embodiments, a linker is provided that associates the C1-INH polypeptide with the albumin domain without substantially affecting the ability of the C1-INH polypeptide to bind to any of its cognate ligands (e.g., C1s, etc.). In some embodiments, a linker is provided such that the binding of a C1-INH peptide to one or more of its cognate ligands is not altered as compared to the C1-INH polypeptide alone. In some embodiments, a linker is provided such that the binding of a C1-INH peptide to one or more of its cognate ligands is not reduced or decreased as compared to the C1-INH polypeptide alone.

### Production of Recombinant C1-INH Fusion Proteins

A recombinant C1-INH fusion protein suitable for the present invention may be produced by any available means. For example, a recombinant C1-INH fusion protein may be recombinantly produced by utilizing a host cell system engineered to express a recombinant C1-INH fusion protein-encoding nucleic acid. Alternatively or additionally, a recombinant C1-INH fusion protein may be produced by activating endogenous genes. Alternatively or additionally, a recombinant C1-INH fusion protein may be partially or fully prepared by chemical synthesis.

Where proteins are recombinantly produced, any expression system can be used. To give but a few examples, known expression systems include, for example, *E.coli,* egg, baculovirus, plant, yeast, or mammalian cells.

In some embodiments, recombinant C1-INH fusion proteins suitable for the present invention are produced in mammalian cells. Non-limiting examples of mammalian cells that may be used in accordance with the present invention include BALB/c mouse myeloma line (NSO/l, ECACC No: 85110503); human retinoblasts (PER.C6, CruCell, Leiden, The Netherlands); monkey kidney CV1 line transformed by SV40 (COS-7, ATCC CRL 1651); human embryonic kidney line (HEK293 or 293 cells subcloned for growth in suspension culture, Graham et al., J. Gen Virol., 36:59,1977); human fibrosarcoma cell line (e.g., HT1080); baby hamster kidney cells (BHK21, ATCC CCL 10); Chinese hamster ovary cells +/-DHFR (CHO, Urlaub and Chasin, Proc. Natl. Acad. Sci. USA, 77:4216, 1980); mouse sertoli cells (TM4, Mather, Biol. Reprod., 23:243-251, 1980); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68, 1982); MRC 5 cells; FS4 cells; and a human hepatoma line (Hep G2).

In some embodiments, the present invention provides recombinant C1-INH fusion proteins produced from human cells. In some embodiments, the present invention provides recombinant C1-INH fusion proteins produced from CHO cells or HT1080 cells.

Typically, cells that are engineered to express a recombinant C1-INH fusion protein may comprise a transgene that encodes a recombinant C1-INH fusion protein described herein. It should be appreciated that the nucleic acids encoding recombinant C1-INH fusion protein may contain regulatory sequences, gene control sequences, promoters, non-coding sequences and/or other appropriate sequences for expressing the recombinant C1-INH fusion protein. Typically, the coding region is operably linked with one or more of these nucleic acid components.

The coding region of a transgene may include one or more silent mutations to optimize codon usage for a particular cell type. For example, the codons of a C1-INH fusion transgene may be optimized for expression in a vertebrate cell. In some embodiments, the codons of a C1-INH fusion transgene may be optimized for expression in a mammalian cell. In some embodiments, the codons of a C1-INH fusion transgene may be optimized for expression in a human cell. In some embodiments, the codons of a C1-INH fusion transgene may be optimized for expression in a CHO cell.

### Nucleic Acids Encoding C1-Inhibitor Fc Fusion Proteins and/or C1-Inhibitor Albumin Fusion Proteins

In some embodiments, nucleic acid molecules are provided comprising nucleic acid sequences encoding for a recombinant gene of interest (herein referred to as a transgene) such as a C1-inhibitor Fc fusion protein and/or C1-inhibitor albumin fusion protein described in various embodiments herein. In some embodiments, the nucleic acid encoding a transgene may be modified to provide increased expression of the encoded C1-inhibitor Fc fusion protein and/or C1-inhibitor albumin fusion protein, which is also referred to as codon optimization. For example, the nucleic acid encoding a transgene can be modified by altering the open reading frame for the coding sequence. As used herein, the term "open reading frame" is synonymous with "ORF" and means any nucleotide sequence that is potentially able to encode a protein, or a portion of a protein. An open reading frame usually begins with a start codon (represented as, e.g. AUG for an RNA molecule and ATG in a DNA molecule in the standard code) and is read in codon-triplets until the frame ends with a STOP codon (represented as, e.g. UAA, UGA or UAG for an RNA molecule and TAA, TGA or TAG in a DNA molecule in the standard code). As used herein, the term "codon" means a sequence of three nucleotides in a nucleic acid molecule that specifies a particular amino acid during protein synthesis; also called a triplet or codon-triplet. For example, of the 64 possible codons in the standard genetic code, two codons, GAA and GAG encode the amino acid Glutamine whereas the codons AAA and AAG specify the amino acid Lysine. In the standard genetic code three codons are stop codons, which do not specify an amino acid. As used herein, the term "synonymous codon" means any and all of the codons that code for a single amino acid. Except for Methionine and Tryptophan, amino acids are coded by two to six synonymous codons. For example, in the standard genetic code the four synonymous codons that code for the amino acid Alanine are GCA, GCC, GCG and GCU, the two synonymous codons that specify Glutamine are GAA and GAG and the two synonymous codons that encode Lysine are AAA and AAG.

In some embodiments, a nucleic acid encoding the open reading frame of a C1-inhibitor Fc fusion protein and/or C1-inhibitor albumin fusion protein may be modified using standard codon optimization methods. Various commercial algorithms for codon optimization are available and can be used to practice the present invention. Typically, codon optimization does not alter the encoded amino acid sequences. In some embodiments, codon optimization may lead to amino acids alteration such as substitution, deletion or insertion. Typically, such amino acid alteration does not substantially alter the protein activity.
Exemplary nucleic acid sequences encode for C1-inhibitor Fc fusion proteins and C1-inhibitor albumin fusion proteins having an amino acid sequence at least 50%, 55%, 60%, 65%, 70%, 75%, 80%, 85%, 90%, 91%, 92%, 93%, 94%, 95%, 96%, 97%, 98%, 99% or more identical or homologous to SEQ ID NO:11, SEQ ID NO:12, SEQ ID NO:13, SEQ ID NO:14, SEQ ID NO:15, SEQ ID NO:16, SEQ ID NO:18, SEQ ID NO:19, SEQ ID NO:21, SEQ ID NO:22, SEQ ID NO:23, SEQ ID NO:24, SEQ ID NO:25, SEQ ID NO:26, SEQ ID NO:28, SEQ ID NO:29, SEQ ID NO:30, SEQ ID NO:31, SEQ ID NO:32, or SEQ ID NO:33.

In some embodiments, a nucleotide change may alter a synonymous codon within the open reading frame in order to agree with the endogenous codon usage found in a particular heterologous cell selected to express C1-inhibitor Fc fusion protein and/or C1-inhibitor albumin fusion protein. Alternatively or additionally, a nucleotide change may alter the G+C content within the open reading frame to better match the average G+C content of open reading frames found in endogenous nucleic acid sequence present in the heterologous host cell. A nucleotide change may also alter a polymononucleotide region or an internal regulatory or structural site found within a C1-inhibitor Fc fusion protein or C1-inhibitor albumin fusion sequence. Thus, a variety of modified or optimized nucleotide sequences are envisioned including, without limitation, nucleic acid sequences providing increased expression of C1-inhibitor Fc fusion proteins and/or C1-inhibitor albumin fusion proteins in a prokaryotic cell; yeast cell; insect cell; and in a mammalian cell.

Typically, a modified nucleic acid encodes a C1-inhibitor Fc fusion protein and/or C1-inhibitor albumin fusion protein with or without amino acid sequence alteration. In the event there is amino acid alteration, such alteration typically does not substantially decrease the C1-inhibitor Fc fusion protein or C1-inhibitor albumin fusion protein activity. In some embodiments, such alteration increases and/or enhances the C1-inhibitor Fc fusion protein or C1-inhibitor albumin fusion protein activity. Activity may refer to the following non-limiting list of parameters: increased half-life, increased/elevated protein expression by host cells, increased stability of expressed protein, increased solubility of expressed protein, decreased aggregation of expressed protein, simpler formulation of expressed protein, simpler purification of expressed protein, increased tolerance of expressed protein to changes in pH, increased ability of protein to tolerate high and low pH conditions, expressed protein that is suitable for formulating at high concentrations.

### Expression Vectors

A nucleic acid sequence encoding a C1-inhibitor Fc fusion protein and/or C1-inhibitor albumin fusion protein as described in the present application, can be molecularly cloned (inserted) into a suitable vector for propagation or expression in a host cell. A wide variety of expression vectors can be used to practice the present invention, including, without limitation, a prokaryotic expression vector; a yeast expression vector; an insect expression vector and a mammalian expression vector. Exemplary vectors suitable for the present invention include, but are not limited to, viral based vectors (e.g., AAV based vectors, retrovirus based vectors, plasmid based vectors). In some embodiments, a nucleic acid sequence encoding a C1-inhibitor Fc fusion protein can be inserted into a suitable vector. In some embodiments, a nucleic acid sequence encoding a C1-inhibitor albumin fusion protein can be inserted into a suitable vector. Typically, a nucleic acid encoding a C1-inhibitor Fc fusion protein or C1-inhibitor albumin fusion protein is operably linked to various regulatory sequences or elements.

### Regulatory Sequences or Elements

Various regulatory sequences or elements may be incorporated in an expression vector suitable for the present invention. Exemplary regulatory sequences or elements include, but are not limited to, promoters, enhancers, repressors or suppressors, 5' untranslated (or non-coding) sequences, introns, 3' untranslated (or non-coding) sequences.

As used herein, a "Promoter" or "Promoter sequence" is a DNA regulatory region capable of binding an RNA polymerase in a cell (e.g., directly or through other promoter bound proteins or substances) and initiating transcription of a coding sequence. A promoter sequence is, in general, bound at its 3' terminus by the transcription initiation site and extends upstream (5' direction) to include the minimum number of bases or elements necessary to initiate transcription at any level. The promoter may be operably associated with or operably linked to the expression control sequences, including enhancer and repressor sequences or with a nucleic acid to be expressed. In some embodiments, the promoter may be inducible. In some embodiments, the inducible promoter may be unidirectional or bio-directional. In some embodiments, the promoter may be a constitutive promoter. In some embodiments, the promoter can be a hybrid promoter, in which the sequence containing the transcriptional regulatory region is obtained from one source and the sequence containing the transcription initiation region is obtained from a second source. Systems for linking control elements to coding sequence within a transgene are well known in the art (general molecular biological and recombinant DNA techniques are described in Sambrook, Fritsch, and Maniatis, Molecular Cloning: A Laboratory Manual, Second Edition, Cold Spring Harbor Laboratory Press, Cold Spring Harbor, NY, 1989, which is incorporated herein by reference). Commercial vectors suitable for inserting a transgene for expression in various host cells under a variety of growth and induction conditions are also well known in the art.

In some embodiments, a specific promoter may be used to control expression of the transgene in a mammalian host cell such as, but are not limited to, SRα-promoter (Takebe et al., Molec. and Cell. Bio. 8:466-472 (1988)), the human CMV immediate early promoter (Boshart ct al., Cell 41:521-530 (1985); Foccking ct al., Gene 45:101-105 (1986)), human CMV promoter, the human CMV5 promoter, the murine CMV immediate early promoter, the EF1-α-promoter, a hybrid CMV promoter for liver specific expression (e.g., made by conjugating CMV immediate early promoter with the transcriptional promoter elements of either human α-1-antitrypsin (HAT) or albumin (HAL) promoter), or promoters for hepatoma specific expression (e.g., wherein the transcriptional promoter elements of either human albumin (HAL; about 1000 bp) or human α-1-antitrypsin (HAT, about 2000 bp) are combined with a 145 long enhancer element of human α-1-microglobulin and bikunin precursor gene (AMBP); HAL-AMBP and HAT-AMBP); the SV40 early promoter region (Benoist at al., Nature 290:304-310 (1981)), the Orgyia pseudotsugata immediate early promoter, the herpes thymidine kinase promoter (Wagner at al., Proc. Natl. Acad. Sci. USA 78:1441-1445 (1981)); or the regulatory sequences of the metallothionein gene (Brinster et al., Nature 296:39-42 (1982)). In some embodiments, the mammalian promoter is a is a constitutive promoter such as, but not limited to, the hypoxanthine phosphoribosyl transferase (HPTR) promoter, the adenosine deaminase promoter, the pyruvate kinase promoter, the beta-actin promoter as well as other constitutive promoters known to those of ordinary skill in the art.

In some embodiments, a specific promoter may be used to control expression of a transgene in a prokaryotic host cell such as, but are not limited to, the β-lactamase promoter (Villa-Komaroff et al., Proc. Natl. Acad. Sci. USA 75:3727-3731 (1978)); the tac promoter (DeBoer et al., Proc. Natl. Acad. Sci. USA 80:21-25 (1983)); the T7 promoter, the T3 promoter, the M13 promoter or the M16 promoter; in a yeast host cell such as, but are not limited to, the GAL1, GAL4 or GAL10 promoter, the ADH (alcohol dehydrogenase) promoter, PGK (phosphoglycerol kinase) promoter, alkaline phosphatase promoter, glyceraldehyde-3-phosphate dehydrogenase III (TDH3) promoter, glyceraldehyde-3-phosphate dehydrogenase II (TDH2) promoter, glyceraldehyde-3-phosphate dehydrogenase I (TDH1) promoter, pyruvate kinase (PYK), enolase (ENO), or triose phosphate isomerase (TPI).

In some embodiments, the promoter may be a viral promoter, many of which are able to regulate expression of a transgene in several host cell types, including mammalian cells. Viral promoters that have been shown to drive constitutive expression of coding sequences in eukaryotic cells include, for example, simian virus promoters, herpes simplex virus promoters, papilloma virus promoters, adenovirus promoters, human immunodeficiency virus (HIV) promoters, Rous sarcoma virus promoters, cytomegalovirus (CMV) promoters, the long terminal repeats (LTRs) of Moloney murine leukemia virus and other retroviruses, the thymidine kinase promoter of herpes simplex virus as well as other viral promoters known to those of ordinary skill in the art.

In some embodiments, the gene control elements of an expression vector may also include 5' non-transcribing and 5' non-translating sequences involved with the initiation of transcription and translation, respectively, such as a TATA box, capping sequence, CAAT sequence, Kozak sequence and the like. Enhancer elements can optionally be used to increase expression levels of a polypeptide or protein to be expressed. Examples of enhancer elements that have been shown to function in mammalian cells include the SV40 early gene enhancer, as described in Dijkema et al., EMBO J. (1985) 4: 761 and the enhancer/promoter derived from the long terminal repeat (LTR) of the Rous Sarcoma Virus (RSV), as described in Gorman et al., Proc. Natl. Acad. Sci. USA (1982b) 79:6777 and human cytomegalovirus, as described in Boshart et al., Cell (1985) 41:521. Genetic control elements of an expression vector will also include 3' non-transcribing and 3' non-translating sequences involved with the termination of transcription and translation. Respectively, such as a poly polyadenylation (polyA) signal for stabilization and processing of the 3' end of an mRNA transcribed from the promoter. Poly A signals included, for example, the rabbit beta globin polyA signal, bovine growth hormone polyA signal, chicken beta globin terminator/polyA signal, or SV40 late polyA region.

### Selectable Markers

Expression vectors will preferably but optionally include at least one selectable marker. In some embodiments, the selectable maker is a nucleic acid sequence encoding a resistance gene operably linked to one or more genetic regulatory elements, to bestow upon the host cell the ability to maintain viability when grown in the presence of a cytotoxic chemical and/or drug. In some embodiments, a selectable agent may be used to maintain retention of the expression vector within the host cell. In some embodiments, the selectable agent is may be used to prevent modification (i.e. methylation) and/or silencing of the transgene sequence within the expression vector. In some embodiments, a selectable agent is used to maintain episomal expression of the vector within the host cell. In some embodiments, the selectable agent is used to promote stable integration of the transgene sequence into the host cell genome. In some embodiments, an agent and/or resistance gene may include, but is not limited to, methotrexate (MTX), dihydrofolate reductase (DHFR, U.S. Pat. Nos. 4,399,216; 4,634,665; 4,656,134; 4,956,288; 5,149,636; 5,179,017, ampicillin, neomycin (G418), zeomycin, mycophenolic acid, or glutamine synthetase (GS, U.S. Pat. Nos. 5,122,464; 5,770,359; 5,827,739) for eukaryotic host cell; tetracycline, ampicillin, kanamycin or chlorampenichol for a prokaryotic host cell; and URA3, LEU2, HIS3, LYS2, HIS4, ADE8, CUP1 or TRP1 for a yeast host cell.

Expression vectors may be transfected, transformed or transduced into a host cell. As used herein, the terms "transfection," "transformation" and "transduction" all refer to the introduction of an exogenous nucleic acid sequence into a host cell. In some embodiments, expression vectors containing nucleic acid sequences encoding for a C1-inhibitor Fc fusion protein and/or a C1-inhibitor albumin fusion protein are transfected, transformed or transduced into a host cell at the same time. In some embodiments, expression vectors containing nucleic acid sequences encoding for a C1-inhibitor Fc fusion protein and/or a C1-inhibitor albumin fusion protein are transfected, transformed or transduced into a host cell sequentially. For example, a vector encoding an C1-inhibitor fusion protein may be transfected, transformed or transduced into a host cell first, followed by the transfection, transformation or transduction of a vector encoding one or more proteins which enhance glycosylation, preferably by increased sialylation, of the expressed C1-inhibitor fusion protein, and vice versa.

Examples of transformation, transfection and transduction methods, which are well known in the art, include liposome delivery, i.e., lipofectamine™ (Gibco BRL) Method of Hawley-Nelson, Focus 15:73 (1193), electroporation, CaPO₄ delivery method of Graham and van der Erb, Virology, 52:456-457 (1978), DEAE-Dextran medicated delivery, microinjection, biolistic particle delivery, polybrene mediated delivery, cationic mediated lipid delivery, transduction, and viral infection, such as, e.g., retrovirus, lentivirus, adenovirus adeno-associated virus and Baculovirus (Insect cells). General aspects of cell host transformations have been described in the art, such as by Axel in U.S. Pat. No. 4,399,216; Sambrook, supra, Chapters 1-4 and 16-18; Ausubel, supra, chapters 1, 9, 13, 15, and 16. For various techniques for transforming mammalian cells, see Keown et al., Methods in Enzymology (1989), Keown et al., Methods in Enzymology, 185:527-537 (1990), and Mansour et al., Nature, 336:348-352 (1988).

Once introduced inside cells, expression vectors may be integrated stably in the genome or exist as extra-chromosomal constructs. Vectors may also be amplified and multiple copies may exist or be integrated in the genome. In some embodiments, cells of the invention may contain 1, 2, 3, 4, 5, 6, 7, 8, 9, 10, 15, 20 or more copies of nucleic acids encoding a C1-inhibitor fusion protein. In some embodiments, cells of the invention may contain 1, 2, 3, 4, 5, 6, 7, 8, 9, 10, 15, 20 or more copies of nucleic acids encoding a C1-inhibitor fusion protein. In some embodiments, cells of the invention may contain multiple copies (e.g., 2, 3, 4, 5, 6, 7, 8, 9, 10, 15, 20 or more) of nucleic acids encoding both C1-inhibitor Fc fusion protein and one or more proteins which enhance glycosylation, preferably by increased sialylation, of the expressed C1-inhibitor fusion protein. Sialylation can also be manipulated through the various methods described herein. Without wishing to be bound by any theory, decreased sialylation was found to be associated with increased heparin binding of the disclosed constructs, thereby preventing the C1-INH binding site from binding to targets. Heparin binding also increases likelihood of internalization into the lysosome, thereby increasing the rate of clearance.

### Host Cells

As used herein, the term "host cells" refers to cells that can be used to produce recombinant C1-inhibitor fusion protein. In particular, host cells are suitable for producing recombinant C1-inhibitor fusion protein at a large scale. Suitable host cells can be derived from a variety of organisms, including, but not limited to, mammals, plants, birds (e.g., avian systems), insects, yeast, and bacteria. In some embodiments, host cells are mammalian cells. In some embodiments, a suitable host cell is engineered to improve the glycosylation profile of the expressed recombinant C1-inhibitor fusion protein. In some embodiments of the invention, the C1-INH polypeptide has a the same or similar glycosylation profile to the analogous portions of native plasma-derived C1-INH. In some embodiments, the C1-INH polypeptide has at least 50%, 55%, 60%, 65%, 70%, 75%, 80%, 85%, 90%, 91%, 92%, 93%, 94%, 95%, 96%, 97%, 98%, 99% glycans equivalent to native plasma-derived C1-INH. In some embodiments, the glycosylation profile of the C1-INH fusion protein has a humanized glycosylation profile. In some embodiments, the C1-INH fusion protein has increased sialylation compared to native plasma-derived C1-INH.

Improvement of the glycosylation profile may refer to increasing sialylation and/or humanizing the glycosylation profile, e.g., expressing in an engineered cell a recombinant C1-inhibitor fusion protein comprising a C1-INH polypeptide, thereby producing a glycosylation profile more similar to native human C1-INH than a C1-INH polypeptide expressed in a non-engineered cell. Improvement may also refer to increasing, enhancing, and/or optimizing the glycosylation profile of the C1-INH fusion compared to Ruconest.

Various methods of changing, controlling, manipulating, improving, enhancing, and/or optimizing the glycosylation profile of proteins are known in the art. The glycosylation profile characterisitcs that may be optimized include the number of glycan residues, location of glycan residue attachment, manner of glycan attachment (e.g., type of bond), process of glycan attachment, and identity of glycan residues attached to the protein or polypeptide. The glycosylation profile of any portion of the fusion proteins of the invention are contemplated as suitable targets for glycosylation optimization. Portions of the fusion proteins of the invention that may be optimized for glycosylation , include but are not limited to, the C1-INH polypeptide, the Fc domain, albumin polypeptides, and/or linkers disclosed herein. These methods of controlling the glycosylation profile, as well as others yet to be discovered that a person of skill would understand as having utility in view of the instant disclosure in optimizing glycosylation of the fusion proteins of the invention, are contemplated. Methods of changing, controlling, manipulating, improving, enhancing, and/or optimizing the glycosylation profile of C1-INH proteins and polypeptides of the invention include *in vitro, in situ,* and *in vivo* methods.

In some embodiments the glycosylation profile of expressed proteins or polypeptides is altered through post-translational and/or chemical modification of the expressed protein or polypeptide.

In some embodiments, the C1-INH fusion protein that has undergone post-translational and/or chemical modification has a glycosylation profile that is changed, improved, enhanced, humanized, and/or optimized compared to a C1-INH fusion protein having the same sequence that has not undergone said post-translational and/or chemical modification. In some embodiments, the C1-INH fusion protein that has undergone post-translational and/or chemical modification has a sialylation profile that is changed, improved, enhanced, humanized, and/or optimized compared to a C1-INH fusion protein having the same sequence that has not undergone said post-translational and/or chemical modification.

In some embodiments, the C1-INH fusion protein is expressed from a cell line engineered to enhance glycosylation has a glycosylation profile that is changed, improved, enhanced, humanized, and/or optimized compared to a C1-INH fusion protein having the same sequence that is expressed from the same cell line that has not been engineered to enhance glycosylation. In some embodiments, the C1-INH fusion protein is expressed from a cell line engineered to enhance sialylation has a sialylation profile that is changed, improved, enhanced, humanized, and/or optimized compared to a C1-INH fusion protein having the same sequence that is expressed from the same cell line that has not been engineered to enhance sialylation.

In some embodiments, the C1-INH fusion protein is expressed from a cell line a cell line cultured under conditions to enhance glycosylation has a glycosylation profile that is changed, improved, enhanced, humanized, and/or optimized compared to a C1-INH fusion protein having the same sequence that is expressed from a cell line cultured under conditions to enhance glycosylation. In some embodiments, the C1-INH fusion protein is expressed from a cell line a cell line cultured under conditions to enhance sialylation has a sialylation profile that is changed, improved, enhanced, humanized, and/or optimized compared to a C1-INH fusion protein having the same sequence that is expressed from a cell line cultured under conditions to enhance sialylation.

In some embodiments the C1-INH fusion protein has a glycosylation profile that is changed, improved, enhanced, humanized, and/or optimized compared to Ruconest. In some embodiments the C1-INH fusion protein has a glycosylation profile that is changed, improved, enhanced, humanized, and/or optimized compared to human plasma-derived C1-INH.

In some embodiments the cell culture conditions are manipulated to achieve expression of proteins having a desired glycosylation profile. These cell culture conditions include control of the production and culture process including length of culture, additives to culture medium, co-expression of genes to enhance glycosylation via increased glycosylation, and/or engineering of cells to knock out, prevent expression of, inactivate, or disrupt enzymes associated with glycan degradation (e.g., sialyladase). Suitable methods for manipulation of glycosylation include, but are not limited to, those described in, e.g., U.S. Patent Nos. 5,047,335; 5,096,816; 5,705,364; 7,645,609; 8,273,723 ; 8,524,477; 8,617,878; 8,871,723; PCT Publication Nos. WO2006/106348; WO2007/095506; WO2008/025856; WO2010/007214; WO2010/099394; and WO2013/093760, the disclosures of which are incorporated herein by reference.

Selection of host cells and specific clones of transfected host cells may also be used to enhance glycosylation. Other methods of enhancing glycosylation include development of purification processes to enrich for proteins or polypeptides having the desired glycosylation profile.

Various methods of manipulating the sialylation profile of proteins are known in the art. These methods as well as others yet to be discovered arc contemplated by the instant invention. Methods of manipulating the sialylation profile of C1-INH proteins and polypeptides of the invention include in vitro, in situ, and in vivo methods. In some embodiments the sialylation profile of expressed proteins or polypeptides is altered through post-expression chemical modification of the expressed protein or polypeptide. In some embodiments the cell culture conditions arc manipulated to achieve expression of proteins having a desired sialylation profile. These cell culture conditions include control of the production and culture process including length of culture, additives to culture medium, and/or co-expression of genes to enhance sialylation. Selection of host cells and specific clones of transfected host cells may also be used to enhance sialylation. Other methods of enhancing sialylation include development of purification processes to enrich for proteins or polypeptides having the desired sialylation profile.

Sialylation can also be manipulated through the various methods described herein. Without wishing to be bound by any theory, decreased sialylation was found to be associated with increased heparin binding of the disclosed constructs, thereby preventing the C1-INH binding site from binding to targets. Heparin binding also increases likelihood of internalization into the lysosome, thereby increasing the rate of clearance.

### Mammalian Cell Lines

Any mammalian cell or cell type susceptible to cell culture, and to expression of polypeptides, may be utilized in accordance with the present invention as a host cell. Non-limiting examples of mammalian cells that may be used in accordance with the present invention include human embryonic kidney 293 cells (HEK293), HeLa cells; BALB/c mouse myeloma line (NSO/l, ECACC No: 85110503); human retinoblasts (PER.C6 (CruCell, Leiden, The Netherlands)); monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); baby hamster kidney cells (BHK, ATCC CCL 10); Chinese hamster ovary cells +/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 (1982)); MRC 5 cells; FS4 cells; and a human hepatoma line (Hep G2). In some embodiments, a suitable mammalian cell is not an endosomal acidification-deficient cell.

Additionally, any number of commercially and non-commercially available hybridoma cell lines that express polypeptides or proteins may be utilized in accordance with the present invention. One skilled in the art will appreciate that hybridoma cell lines might have different nutrition requirements and/or might require different culture conditions for optimal growth and polypeptide or protein expression, and will be able to modify conditions as needed.

### Non-Mammalian Cell Lines

Any non-mammalian derived cell or cell type susceptible to cell culture, and to expression of polypeptides, may be utilized in accordance with the present invention as a host cell. Non-limiting examples of non-mammalian host cells and cell lines that may be used in accordance with the present invention include cells and cell lines derived from *Pichia pastoris, Pichia methanolica, Pichia angusta, Schizosacccharomyces pombe, Saccharomyces cerevisiae,* and *Yarrowia lipolytica* for yeast; *Sodoptera frugiperda, Trichoplusis ni, Drosophila melangoster* and *Manduca sexta* for insects; and *Escherichia coli, Salmonella typhimurium, Bacillus subtilis, Bacillus lichenifonnis, Bacteroides fragilis, Clostridia perfringens, Clostridia difficile* for bacteria; and *Xenopus Laevis* from amphibian.

### Adaptable to Adherent vs Suspension Growth

In certain embodiments, a host cell is selected for generating a cell line based on certain preferable attributes or growth under particular conditions chosen for culturing cells. It will be appreciated by one skilled in the art, such attributes may be ascertained based on known characteristic and/or traits of an established line (i.e. a characterized commercially available cell line) or though empirical evaluation. In some embodiments, a cell line may be selected for its ability to grow on a feeder layer of cells. In some embodiments, a cell line may be selected for its ability to grow in suspension. In some embodiments, a cell line may be selected for its ability to grow as an adherent monolayer of cells. In some embodiments, such cells can be used with any tissue culture vessel or any vessel treated with a suitable adhesion substrate. In some embodiments, a suitable adhesion substrate is selected from the group consisting of collagen (e.g. collagen I, II, II, or IV), gelatin, fibronectin, laminin, vitronectin, fibrinogen, BD Matrigel™, basement membrane matrix, dermatan sulfate proteoglycan, Poly-D-Lysine and/or combinations thereof. In some embodiments, an adherent host cell may be selected and modified under specific growth conditions to grow in suspension. Such methods of modifying an adherent cell to grown in suspension are known in the art. For example, a cell may be conditioned to grow in suspension culture, by gradually removing animal scrum from the growth media over time.

### Cell Line Selection and Evaluation

According to the present invention, a cell engineered to express recombinant C1-INH fusion protein is selected for its ability to produce the recombinant C1-INH fusion protein at commercially viable scale. In particular, engineered cells according to the present invention are able to produce recombinant C1-INH fusion protein at a high level and/or with high enzymatic activity. In some embodiments, desirable cells, once cultivated under a cell culture condition (e.g., a standard large scale suspension or adherent culture condition), can produce C1-INH fusion protein in an amount of or greater than about 5 picogram/cell/day (e.g., greater than about 10, 15, 20, 25, 30, 35, 40, 45, 50, 55, 60, 65, 70, 75, 80, 85, 90, 95, or 100 picogram/cell/day). In some embodiments, desired cells, once cultivated under a cell culture condition (e.g., a standard large scale suspension or adherent culture condition), are able to produce C1-INH fusion protein in an amount ranging from about 5-100 picogram/cell/day (e.g., about 5-90 picogram/cell/day, about 5-80 picogram/cell/day, about 5-70 picogram/cell/day, about 5-60 picogram/cell/day, about 5-50 picogram/cell/day, about 5-40 picogram/cell/day, about 5-30 picogram/cell/day, about 10-90 picogram/cell/day, about 10-80 picogram/cell/day, about 10-70 picogram/cell/day, about 10-60 picogram/cell/day, about 10-50 picogram/cell/day, about 10-40 picogram/cell/day, about 10-30 picogram/cell/day, about 20-90 picogram/cell/day, about 20-80 picogram/cell/day, about 20-70 picogram/cell/day, about 20-60 picogram/cell/day, about 20-50 picogram/cell/day, about 20-40 picogram/cell/day, about 20-30 picogram/cell/day).

The half-life of C1-INH may be affected by the glycosylation profile. As discussed above, cells of the invention may be engineered to improve the glycosylation profile of the expressed C1-INH fusion protein. For example, Ruconest® (Pharming N.V.), a recombinant C1-INH polypeptide that is less sialylated and/or has a different sialylation distribution from plasma-derived human C1-INH has been shown to have a shorter half-life than plasma-derived human C1-INH. Davis, B. & Bernstein, J. A., Conestat alfa for the treatment of angioedema attacks, Ther Clin Risk Manag. 7: 265-273 (2011); Koles, K. et al., Influence of lactation parameters on the N-glycosylation of recombinant human C1 inhibitor isolated from the milk of transgenic rabbits, Glycobiology, 14(11):979-986 (2004); Koles, K. et al., N- and O-glycans of recombinant human C1 inhibitor expressed in the milk of transgenic rabbits, Glycobiology, 14(1):51-64 (2004).

### Cell Culture Medium and Condition

Various cell culture medium and conditions may be used to produce a recombinant C1-INH fusion protein using engineered cells according to the present invention. For example, a recombinant C1-INH fusion protein may be produced in serum-containing or serum-free medium. In some embodiments, a recombinant C1-INH fusion protein is produced in serum-free medium. In some embodiments, a recombinant C1-INH fusion protein is produced in an animal free medium, i.e., a medium that lacks animal-derived components. In some embodiments, a recombinant C1-INH fusion protein is produced in a chemically defined medium. As used herein, the term "chemically-defined nutrient medium" refers to a medium of which substantially all of the chemical components are known. In some embodiments, a chemically defined nutrient medium is free of animal-derived components such as serum, serum derived proteins (e.g., albumin or fetuin), and other components. In some cases, a chemically-defined medium comprises one or more proteins (*e.g*., protein growth factors or cytokines.) In some cases, a chemically-defined nutrient medium comprises one or more protein hydrolysates. In other cases, a chemically-defined nutrient medium is a protein-free media, i.e., a serum-free media that contains no proteins, hydrolysates or components of unknown composition.

In some embodiments, a chemically defined medium may be supplemented by one or more animal derived components. Such animal derived components include, but are not limited to, fetal calf serum, horse serum, goat serum, donkey serum, human serum, and serum derived proteins such as albumins (e.g., bovine serum albumin or human serum albumin).

Various cell culture conditions may be used to produce recombinant C1-INH fusion proteins at large scale including, but not limited to, roller bottle cultures, bioreactor batch cultures, perfusion cultures, and bioreactor fed-batch cultures. In some embodiments, recombinant C1-INH fusion protein is produced by cells cultured in suspension. In some embodiments, recombinant C1-INH fusion protein is produced by adherent cells. In some embodiments, perfusion culture is used to control glycosylation of expressed protein. Exemplary methods of perfusion culture include, but are not limited to, those described in, e.g., U.S. Patent Nos. 6,528,286 and PCT Publication No. WO1996/039488A1, the disclosures of which are incorporated herein by reference.

Exemplary cell media and culture conditions are described in the Examples sections. The examples are not intended to be limiting.

### Purification of Expressed C1-INH Fusion Protein

Various methods may be used to purify or isolate C1-INH fusion protein produced according to various methods described herein. In some embodiments, the expressed C1-INH fusion protein is secreted into the medium and thus cells and other solids may be removed, as by centrifugation or filtering for example, as a first step in the purification process. Alternatively or additionally, the expressed C1-INH fusion protein is bound to the surface of the host cell. In this embodiment, the host cells expressing the polypeptide or protein are lysed for purification. Lysis of mammalian host cells can be achieved by any number of means well known to those of ordinary skill in the art, including physical disruption by glass beads and exposure to high pH conditions.

The C1-INH fusion protein may be isolated and purified by standard methods including, but not limited to, chromatography (e.g., ion exchange, affinity, size exclusion, and hydroxyapatite chromatography), gel filtration, centrifugation, or differential solubility, ethanol precipitation or by any other available technique for the purification of proteins (See, e.g., Scopes, Protein Purification Principles and Practice 2nd Edition, Springer-Verlag, New York, 1987; Higgins, S. J. and Hames, B. D. (eds.), Protein Expression: A Practical Approach, Oxford Univ Press, 1999; and Deutscher, M. P., Simon, M. I., Abelson, J. N. (eds.), Guide to Protein Purification: Methods in Enzymology (Methods in Enzymology Series, Vol 182), Academic Press, 1997, all incorporated herein by reference). For immunoaffinity chromatography in particular, the protein may be isolated by binding it to an affinity column comprising antibodies that were raised against that protein and were affixed to a stationary support. Alternatively, affinity tags such as an influenza coat sequence, poly-histidine, or glutathione-S-transferase can be attached to the protein by standard recombinant techniques to allow for easy purification by passage over the appropriate affinity column. Protease inhibitors such as phenyl methyl sulfonyl fluoride (PMSF), leupeptin, pepstatin, or aprotinin may be added at any or all stages in order to reduce or eliminate degradation of the polypeptide or protein during the purification process. Protease inhibitors are particularly desired when cells must be lysed in order to isolate and purify the expressed polypeptide or protein.

Protein A HP Purification of IgG1 LALA Fc full length C1-inhibitor is shown in FIG. 3. Protein A capture of fusion proteins was successful on small scale, but aggregation was observed when scaled up. Without wishing to be bound by any theory, the low pH necessary for elution appeared to cause the aggregation and inactivate C1-INH. In some embodiments purification does not involve a low pH step. In some embodiments, the fusion proteins are mutated in order to stabilize the protein at low pH. In other embodiments, additives are used to protect the C1-INH fusion proteins from aggregation during the low pH elution step. In still other embodiments, non-protein A resins are used to purify proteins,

Exemplary purification methods that avoid the problems associated with Protein A purification of C1-INH Fc fusion proteins are described in the Examples sections below. Suitable resins for purification include, but are not limited to, anion exchange resin, albumin affinity resin, C1 inhibitor affinity resin, and protein A resin. In some embodiments, a purification method that does not require a drop in pH is preferred. In some embodiments, a purification method that does not require an acidic pH for elution is preferred. In other embodiments, a stabilizer that prevents aggregation is utilized. In some embodiments, the stabilizer that prevents aggregation is used in a method requiring a drop in pH. Non-limiting examples of suitable stabilizers include EDTA.

Suitable methods for purification of the C1-INH fusion proteins of the invention include, but are not limited to, those described in, e.g., U.S. Patent Nos. 5,276,141; US7384754; 8,802,816; PCT Publication Nos. WO2012107572; and WO2013009526, the disclosures of which are incorporated herein by reference.

### Pharmaceutical compositions

The present invention further provides a pharmaceutical composition containing a recombinant C1-INH fusion protein described herein and a physiologically acceptable carrier or excipient. The carrier and recombinant C1-INH fusion protein can be sterile. The formulation should suit the mode of administration.

Suitable pharmaceutically acceptable carriers include but are not limited to water, salt solutions (*e.g*., NaCl), saline, buffered saline, alcohols, glycerol, ethanol, gum arabic, vegetable oils, benzyl alcohols, polyethylene glycols, gelatin, carbohydrates such as lactose, amylose or starch, sugars such as mannitol, sucrose, or others, dextrose, magnesium stearate, talc, silicic acid, viscous paraffin, perfume oil, fatty acid esters, hydroxymethylcellulose, polyvinyl pyrrolidone, *etc.,* as well as combinations thereof. The pharmaceutical preparations can, if desired, be mixed with auxiliary agents (*e.g*., lubricants, preservatives, stabilizers, wetting agents, emulsifiers, salts for influencing osmotic pressure, buffers, coloring, flavoring and/or aromatic substances and the like) which do not deleteriously react with the active compounds or interference with their activity. In a preferred embodiment, a water-soluble carrier suitable for intravenous administration is used.

A suitable pharmaceutical composition or medicament, if desired, can also contain minor amounts of wetting or emulsifying agents, or pH buffering agents. A composition can be a liquid solution, suspension, emulsion, tablet, pill, capsule, sustained release formulation, or powder. A composition can also be formulated as a suppository, with traditional binders and carriers such as triglycerides. Oral formulations can include standard carriers such as pharmaceutical grades of mannitol, lactose, starch, magnesium stearate, polyvinyl pyrrolidone, sodium saccharine, cellulose, magnesium carbonate, *etc.*

A pharmaceutical composition or medicament can be formulated in accordance with the routine procedures as a pharmaceutical composition adapted for administration to human beings. For example, in some embodiments, a composition for intravenous administration typically is a solution in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

A recombinant C1-INH fusion protein described herein can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, *etc.*

A preferred formulation comprises 50 mM NaPO4 (pH 7.2), 50 mM Sorbitol, and 150 mM Glycine. The formulation may be liquid, or may be lyophilized and reconstituted prior to administration.

### Routes of Administration

A recombinant C1-INH fusion protein described herein (or a composition or medicament containing a recombinant C1-INH fusion protein described herein) is administered by any appropriate route. In some embodiments, a recombinant CI-INH fusion protein or a pharmaceutical composition containing the same is administered systemically. Systemic administration may be intravenous, intradermal, intracranial, intrathecal, inhalation, transdermal (topical), intraocular, intramuscular, subcutaneous, intramuscular, oral, and/or transmucosal administration. In some embodiments, a recombinant C1-INH fusion protein or a pharmaceutical composition containing the same is administered subcutaneously. As used herein, the term "subcutaneous tissue", is defined as a layer of loose, irregular connective tissue immediately beneath the skin. For example, the subcutaneous administration may be performed by injecting a composition into areas including, but not limited to, the thigh region, abdominal region, gluteal region, or scapular region. In some embodiments, a recombinant C1-INH fusion protein or a pharmaceutical composition containing the same is administered intravenously. In some embodiments, a recombinant C1-INH fusion protein or a pharmaceutical composition containing the same is administered orally. In some embodiments, a recombinant C1-INH fusion protein or a pharmaceutical composition containing the same is administered intracranially. In some embodiments, a recombinant C1-INH fusion protein or a pharmaceutical composition containing the same is administered intrathecally. More than one route can be used concurrently, if desired.

In some embodiments, a recombinant C1-INH fusion protein or a pharmaceutical composition containing the same is administered to a subject by intrathecal administration. As used herein, the term "intrathecal administration" or "intrathecal injection" refers to an injection into the spinal canal (intrathecal space surrounding the spinal cord). Various techniques may be used including, without limitation, lateral cerebroventricular injection through a burrhole or cisternal or lumbar puncture or the like. In some embodiments, "intrathecal administration" or "intrathecal delivery" according to the present invention refers to IT administration or delivery via the lumbar area or region, i.e., lumbar IT administration or delivery. As used herein, the term "lumbar region" or "lumbar area" refers to the area between the third and fourth lumbar (lower back) vertebrae and, more inclusively, the L2-S1 region of the spine.

In some embodiments, a recombinant C1-INH fusion protein or a pharmaceutical composition containing the same is administered to the subject by subcutaneous (i.e., beneath the skin) administration. For such purposes, the formulation may be injected using a syringe. However, other devices for administration of the formulation are available such as injection devices (e.g., the Inject-ease™ and Genject™ devices); injector pens (such as the GenPen™); needleless devices (e.g., MediJector™ and BioJector™); and subcutaneous patch delivery systems.

In some embodiments, intrathecal administration may be used in conjunction with other routes of administration (e.g., intravenous, subcutaneously, intramuscularly, parenterally, transdermally, or transmucosally (*e.g.*, orally or nasally)).

The present invention contemplates single as well as multiple administrations of a therapeutically effective amount of a recombinant C1-INH fusion protein or a pharmaceutical composition containing the same described herein. A recombinant C1-INH fusion protein or a pharmaceutical composition containing the same can be administered at regular intervals, depending on the nature, severity and extent of the subject's condition (e.g., a lysosomal storage disease). In some embodiments, a therapeutically effective amount of a recombinant C1-INH fusion protein or a pharmaceutical composition containing the same may be administered periodically at regular intervals (e.g., once every year, once every six months, once every five months, once every three months, bimonthly (once every two months), monthly (once every month), biweekly (once every two weeks), weekly, daily or continuously).

In some embodiments, administration results only in a localized effect in an individual, while in other embodiments, administration results in effects throughout multiple portions of an individual, for example, systemic effects. Typically, administration results in delivery of a recombinant C1-INH fusion protein to one or more target tissues. In some embodiments, the recombinant CI-INH fusion protein is delivered to one or more target tissues including, but not limited to, heart, brain, skin, blood, spinal cord, striated muscle (e.g., skeletal muscle), smooth muscle, kidney, liver, lung, and/or spleen. In some embodiments, the recombinant CI-INH fusion protein is delivered to the heart. In some embodiments, the recombinant C1-INH fusion protein is delivered to the central nervous system, particularly the brain and/or spinal cord. In some embodiments, the recombinant C1-INH fusion protein is delivered to triceps, tibialis anterior, soleus, gastrocnemius, biceps, trapezius, deltoids, quadriceps, and/or diaphragm.

### Dosage Forms and Dosing Regimen

In some embodiments, a composition is administered in a therapeutically effective amount and/or according to a dosing regimen that is correlated with a particular desired outcome (e.g., with prophylaxis of a complement-mediated chronic disease, such as HAE).

Particular doses or amounts to be administered in accordance with the present invention may vary, for example, depending on the nature and/or extent of the desired outcome, on particulars of route and/or timing of administration, and/or on one or more characteristics (e.g., weight, age, personal history, genetic characteristic, lifestyle parameter, severity of cardiac defect and/or level of risk of cardiac defect, etc., or combinations thereof). Such doses or amounts can be determined by those of ordinary skill. In some embodiments, an appropriate dose or amount is determined in accordance with standard clinical techniques. Alternatively or additionally, in some embodiments, an appropriate dose or amount is determined through use of one or more *in vitro* or *in vivo* assays to help identify desirable or optimal dosage ranges or amounts to be administered.

In various embodiments, a recombinant C1-INH fusion protein is administered at a therapeutically effective amount. Generally, a therapeutically effective amount is sufficient to achieve a meaningful benefit to the subject (e.g., prophylaxis, treating, modulating, curing, preventing and/or ameliorating the underlying disease or condition). Generally, the amount of a therapeutic agent (e.g., a recombinant C1-INH fusion protein) administered to a subject in need thereof will depend upon the characteristics of the subject. Such characteristics include the condition, disease severity, general health, age, sex and body weight of the subject. One of ordinary skill in the art will be readily able to determine appropriate dosages depending on these and other related factors. In addition, both objective and subjective assays may optionally be employed to identify optimal dosage ranges. In some particular embodiments, appropriate doses or amounts to be administered may be extrapolated from dose-response curves derived from *in vitro* or animal model test systems.

In some embodiments, a composition is provided as a pharmaceutical formulation. In some embodiments, a pharmaceutical formulation is or comprises a unit dose amount for administration in accordance with a dosing regimen correlated with achievement of the reduced incidence or risk of an HAE attack.

In some embodiments, a formulation comprising a recombinant C1-INH fusion protein described herein administered as a single dose. In some embodiments, a formulation comprising a recombinant C1-INH fusion protein described herein is administered at regular intervals. Administration at an "interval," as used herein, indicates that the therapeutically effective amount is administered periodically (as distinguished from a one-time dose). The interval can be determined by standard clinical techniques. In some embodiments, a formulation comprising a recombinant C1-INH fusion protein described herein is administered bimonthly, monthly, twice monthly, triweekly, biweekly, weekly, twice weekly, thrice weekly, daily, twice daily, or every six hours. The administration interval for a single individual need not be a fixed interval, but can be varied over time, depending on the needs of the individual.

A therapeutically effective amount is commonly administered in a dosing regimen that may comprise multiple unit doses. For any particular therapeutic protein, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific fusion protein employed; the duration of the treatment; and like factors as is well known in the medical arts.

As used herein, the term "bimonthly" means administration once per two months (*i.e.,* once every two months); the term "monthly" means administration once per month; the term "triweekly" means administration once per three weeks (*i.e.,* once every three weeks); the term "biweekly" means administration once per two weeks (*i.e.,* once every two weeks); the term "weekly" means administration once per week; and the term "daily" means administration once per day.

In some embodiments, a formulation comprising a recombinant C1-INH fusion protein described herein is administered at regular intervals indefinitely. In some embodiments, a formulation comprising a recombinant C1-INH fusion protein described herein is administered at regular intervals for a defined period.

It is to be further understood that for any particular subject, specific dosage regimens should be adjusted over time according to the individual need and the professional judgment of the person administering or supervising the administration of the enzyme replacement therapy and that dosage ranges set forth herein are exemplary only and are not intended to limit the scope or practice of the claimed invention.

### Combination Therapy

In some embodiments, a recombinant CI-INH fusion protein is administered in combination with one or more known therapeutic agents (e.g., corticosteroids) currently used for treatment of a complement-mediated disease. In some embodiments, the known therapeutic agent(s) is/are administered according to its standard or approved dosing regimen and/or schedule. In some embodiments, the known therapeutic agent(s) is/are administered according to a regimen that is altered as compared with its standard or approved dosing regimen and/or schedule. In some embodiments, such an altered regimen differs from the standard or approved dosing regimen in that one or more unit doses is altered (e.g., reduced or increased) in amount, and/or in that dosing is altered in frequency (e.g., in that one or more intervals between unit doses is expanded, resulting in lower frequency, or is reduced, resulting in higher frequency).

### A. Disorders

Preferred embodiment is treatment of chronic disorders
In some embodiments, the fusion proteins provided by the invention are suitable for acute attacks associated with complement-mediated disorders, e.g., NMOSD AMR, and HAE events. These attacks may be long or short. In some embodiments, the disease or disorder is chronic. In some embodiments the compositions and methods of the invention are used prophylactically. Exemplary complement-mediated disease that may be treated using the compositions and methods disclosed herein include, but are not limited to, hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

### EXAMPLES

Other features, objects, and advantages of the present invention are apparent in the examples that follow. It should be understood, however, that the examples, while indicating embodiments of the present invention, are given by way of illustration only, not limitation. Various changes and modifications within the scope of the invention will become apparent to those skilled in the art from the examples.

### Example 1. Fc Fusion Proteins

This example illustrates various exemplary fusion protein constructs and transient or scale-up expression of such fusion protein constructs in mammalian cells. As shown below, wild-type or mutant Fc moieties are fused to full length (1-478 aa) or truncated (98-478 aa) human C1-inhibitor.

### A. Fc-C1-Inhibitor Fusion Expression Constructs

A fusion protein of full Length C1-inhibitor with an N-terminal IgG1 Fc was made according to the methods described above, having the amino acid sequence (the Fc moiety is underlined):

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:11).

A fusion protein of truncated C1-inhibitor with an N-terminal IgG1 Fc was made according to the methods described above, having the amino acid sequence (the Fc moiety is underlined):

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:12).

Truncated C1-inhibitor with an N-terminal IgG1 Fc eliminates a large amount of the carbohydrate sites due to the removal of the serine and threonine residues on the truncated portion of the C1-INH polypeptide. These amino acids have OH moieties that become glycosylated in post-translational processing. Without wishing to be bound by any theory, the elimination of this carbohydrate reduces potential clearance of the molecule via the asialoglycoprotein receptor, which may extend half-life of the Fc-C1-inhibitor fusion protein.

Without wishing to be bound by any theory, asialoglycoprotein is not the only clearance mechanism. There are also active clearance pathways based on presence of other glycans. The Fc domain does not bind the FcRN receptor until the complex has been internalized in an endosome. The drop in pH within the endosome allows for the binding to occur. In some embodiments, the active clearance processes are reduced, slowed, and/or eliminated by manipulation of the glycosylation profile of the C1-INH fusion construct. This allows for the passive Fc recycling process to be effective in increasing half-life. In some embodiments, a truncated C1-INH polypeptide is preferred. The truncation may remove glycosylation moieties associated with active clearance. Further, the truncated form is smaller in size which may increase absorption, particularly when administered subcutaneously.

IgG1 LALA mutation eliminates the complement activation and ADCC that can happen from wild type IgG1 Fc sequence. Both full length and truncated C1-INH IgG1 Fc effector dead constructs were made.

Full-length C1-inhibitor with an N-terminal IgG1 LALA Fc was made according to the methods described above having the amino acid sequence (the Fc moiety is underlined, LALA mutation bolded):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:13).

Truncated C1-inhibitor with an N-terminal IgG1 LALA Fc was made according to the methods described above having the amino acid sequence (the Fc moiety is underlined, LALA mutation bolded):

DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGSFCPGP
VTLCSDLESHSTEAVLGDALVDFSLKLYHAFSAMKKVETNMAFSPFSIASLLTQVLLGA
GENTKTNLESILSYPKDFTCVHQALKGFTTKGVTSVSQIFHSPDLAIRDTFVNASRTLYSS
SPRVLSNNSDANLELINTWVAKNTNNKISRLLDSLPSDTRLVLLNAIYLSAKWKTTFDPK
KTRMEPFHFKNSVIKVPMMNSKKYPVAHFIDQTLKAKVGQLQLSHNLSLVILVPQNLKH
RLEDMEQALSPSVFKAIMEKLEMSKFQPTLLTLPRIKVTTSQDMLSIMEKLEFFDFSYDL
NLCGLTEDPDLQVSAMQHQTVLELTETGVEAAAASAISVARTLLVFEVQQPFLFVLWD
QQHKFPVFMGRVYDPRA (SEQ ID NO:14).

A fusion protein of truncated C1-inhibitor with an N-terminal IgG4 Fc is made according to the methods described above, having the amino acid sequence (the Fc moiety is underlined):

ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG

FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:32).

A fusion protein of truncated C1-inhibitor with an N-terminal IgG4 Fc is made according to the methods described above, having the amino acid sequence (the Fc moiety is underlined):

ESKYGPPCPSCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:33).

Full-length C1-inhibitor with an N-terminal IgG4 S241P Fc was made according to the methods described above having the amino acid sequence (the Fc moiety is underlined, S241P mutation bolded):

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKNPNATSSS
SQDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:15).

Truncated C1-inhibitor with an N-terminal IgG4 S241P Fc was made according to the methods described above having the amino acid sequence (the Fc moiety is underlined, S241P mutation bolded):

ESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGKGSFCPGPV
TLCSDLESHSTEAVLGDALVDFSLKLYHAFSAMKKVETNMAFSPFSIASLLTQVLLGAG
ENTKTNLESILSYPKDFTCVHQALKGFTTKGVTSVSQIFHSPDLAIRDTFVNASRTLYSSS
PRVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:16).

### B. Small Scale Transient Expression of Fc-C1-inhibitor Fusion Proteins

FreeStyle™ 293-F Cells (suspension human embryonal kidney cells, Invitrogen, Cat. No. R790-07), FreeStyle™ CHO-S Cells (suspension Chinese Hamster Ovary cells, Invitrogen, Cat. No. R800-07), and HT1080 cells were transfected with various C1-inhibitor fusion plasmids (N-hFc, N-hFcLALA and N-hFcIgG4m), and with no DNA as a control.

The transfections of the 293-F and CHO-S cells were carried out using 293-FreeStyle™ MAX Reagent (Invitrogen, Cat. No. 16447-500) following Invitrogen's protocol (Invitrogen Protocol Pub. No. MAN0007818 Rev. 1.0, available at https ://tools.thermofisher.com/content/sfs/manuals/FreeStyle_MAX_Reagent_man.pdf). After transfection, the 293-F cells were seeded into serum-free FreeStyle™ 293 Expression Medium (Invitrogen, Cat. No. 12338-018) and the CHO-S cells were seeded into serum-free FreeStyle™ CHO Expression Medium (Invitrogen, Cat. No. 12651-014), both having a final culture volume of 30 mL.

Similarly, HT1080 cells were transfected using a standard transfection protocol (350V, 960uF, 12e6 cells, 35µg DNA).

Conditioned Medium (CM) from the transfected cell cultures was harvested 4 days and 7 days (3 day accumulation after re-feed) post transfection. Fusion protein expression was evaluated by SDS-PAGE (8-16% TG gel, 150V for 1.5h) and visualized with Coomassie blue.

In order to generate stable pools of effector dead constructs, the HT1080 transiently transfected cells were seeded at 1x10⁶ cells/mL at 37°C. After two days of recovery, cells wereselected for 2 weeks in a suitable chemically defined (CD) medium_with 250 µg/mL zeocin until cells exhibited 95% viability. Fusion protein expression was evaluated by SDS-PAGE and visualized with Coomassie blue. Expression of the C1-INH Fc fusion constructs was found to be greater than that of the C1-INH albumin constructs.

### C. Scale-up Expression using FreeStyle™ 293-F Cells

FreeStyle™ 293-F Cells were transfected with LALA-Cl-inhibitor fusion plasmids (both full-length and truncated C1) as well as IgG4m-C1-inhibitor fusion plasmids (full-length and truncated C1). Transfections were carried out using 293-FreeStyle™ MAX Reagent as described above.

Cells were transfected in 1 L shake flasks with 320 mL working volume. DNA and FreeStyle™ MAX Reagent quantities were scaled-up proportionally from the 30 mL transfection volume of the transfections described above. CM from transfected cells was harvested 4 days post transfection and expression was evaluated with SDS-PAGE, as described above. Cells were re-fed for a second harvest (7 days post transfection, 3 day batch collection).

### C. Expression Data Summary

Fc fusions with full length C1-inhibitor consistently express at higher levels than native recombinant C1. Fc fusions with truncated C1-inhibitor express similarly to native recombinant C1-inhibitor. No severe clipping of the proteins was observed in any of the host cell culture samples for any of the tested constructs.

HEK293 transient cell expression was particularly increased compared to the other tested cell types. Placement of the Fc sequence at the N-terminus resulted in particularly increased expression than if on the C-terminus

### Example 2. Characterization of Fc-C1-INH Fusion Proteins

To characterize Fc-C1-inhibitor fusion proteins, the fusion proteins were first purified using a process involving protein A column. Exemplary purification results are shown in FIG. 3.

C1-inhibitor fusion composition concentrations and total protein detected in samples are depicted in FIG. 4. Highly purified material was obtained after a single affinity column purification. Excellent protein yields were achieved using the purification process described above.

C1-inhibitor fusion composition endotoxin concentrations detected in samples are depicted in FIG. 5. Low endotoxin levels were found in the final products, making them eminently suitable for pharmaceutical use.

The purified Fc Fusion proteins outlined above were analyzed for protein purity, SEC, thermal stability, binding to FcRN, binding to C1q, and binding to FcgR1.

All samples for PK analysis were above 97% pure by RP HPLC.
Size exclusion chromatography of C1-inhibitor fusion proteins was performed under the following exemplary conditions: Column: Tosoh G3000swxl; Mobile Phase Buffer:0.2M sodium phosphate pH 6.8; Flow Rate:0.5 mL/min

Samples were injected at volume of 40 µL. The samples run were LALA C1-inhibitor full length(FL) R1; LALA C1-inhibitor truncated (TR) R1; IgG4 C1-inhibitor full length (FL) R1; IgG4 C1-inhibitor truncated (TR) R1; and a 20 µL BSA control injection. results

Exemplary results of the SEC-MALS are depicted in Table 1.

**Table 1: SEC-MALS Results**

| **Sample Name** | **g/mol** |
|---|---|
| **LALA FL R1** | 195,600 |
| **LALA TR R1** | Peak1=385,000 Peak2=184,000 |
| **IgG4 FL R1** | Peak1=984,200 Peak2=212,300 |
| **IgG4 TR R1** | Peak1=360,300 Peak2=191,900 |

Thermal stability of C1-inhibitor fusion protein constructs was assessed by differential scanning calorimetry (DSC).

### A. Fc Fusion Protein Binding to FcRN and Fc Effector Function

Binding to the Fc neonatal receptor (FcRN) allows for recycling of the molecules and leads to an extended in vivo serum half-life of the Fc fusion proteins. Recycling occurs as the molecules are passively taken into the cells and the pH of the endosomes is lower. That leads to binding of the Fc portion of the molecule to the FcRN. When the FcRN recycles back to the surface of the cell, the pH is then neutral and the protein is released back into the serum.

Binding to the extracellular domain of the FcRN was measured by surface plasmon resonance (SPR) using a Biacore system.

Direct immobilization with FcRn was achieved via amine coupling of a CM5 chip with FcRn under the following conditions:
FcRn (Sino Biological Inc or in-house BD1) is diluted in Acetate buffer pH 5.0 (Cat#BR-1003-51) to 10 µg/mL.
Flow rate: 10 µl/min
Final coupling signal 354 Ru
Running buffer: PBS-P+, pHed to 6.0

The kinetic binding study was done using the following protocol. Samples were diluted in PBS-P+ to 100, 50, 25, 12.5, 6.25, 3.125, 0 nM. The parameters were set as follows:
Association 300s and Dissociation 600s at Flow rate 30 µL/min
Regeneration with 25 mM Tris, 150mM NaCl pH 8.0

A summary of exemplary kinetic binding data with FcRn is depicted in Table 2.

**Table 2: Summary of Kinetic binding data with FcRn**

| | **Constructs** | **ka (1/Ms)** | **kd (1/s)** | **KD** |
|---|---|---|---|---|
| **C1-Inh Full Length** | **N-hFcLala-C1-INH** | 2.19E+05 | 0.003019 | 1.38E-08 |
| | **N-IgG4m-C1-INH** | 1.87E+05 | 0.005616 | 3.00E-08 |
| **C1-Inh Truncated** | **N-hFcLala-C1-INH Tr** | 8.42E+04 | 0.008214 | 9.76E-08 |
| | **N-IgG4m-C1-INH Tr** | 1.34E+05 | 0.009462 | 7.07E-08 |

### B. Fc Fusion Protein Binding to C1q Binding

Wild Type Fc molecules have varying amounts of effector function, e.g., the ability to activate the complement cascade, ADCC activity, and/or to bind to specific Fc receptors that are not desired for the purposes of the present invention. Patients in need of the C1-INH fusion proteins of the invention suffer from complement-mediated disease, thus it is an object of the invention to reduce or eliminate effector functions associated with Fc domains. The C1-INH Fc fusion constructs disclosed herein preferably reduce or eliminate effector functions. The C1-INH Fc fusion constructs preferably reduce or eliminate complement activation, ADCC, and/or Fcγ Receptors. Effector function can be measured by binding to C1q, also by binding to Fcγ Receptors.

### C1q Binding ELISA

The C1q binding ELISA protocol used was:
- Protein was coated onto maxisorp plate and titrated from 100µg/mL to 0µg/mL in 50mM Carbonate Buffer pH 9.6 O/N at 4°C
- 3 washes with ELISA Wash Buffer (PBS/0.05% TWEEN-20)
- Add 2µg/mL C1q in Assay Buffer (PBS/0.05% TWEEN-20/0.1% fish gelatin) and incubate for 2h at RT
- 3 washes with ELISA Wash Buffer (PBS/0.05% TWEEN-20)
- Add anti-Clq-HRP antibody (Thermo PA1-84324) at 4µg/mL and incubate 1h at RT
- 3 washes with ELISA Wash Buffer (PBS/0.05% TWEEN-20)
- TMB for 15min at RT
- Read OD450

Exemplary C1q binding ELISA results for the hFc, hFcLALA and IgG4m fusions with C1-INH and rhC1 (plasma-derived commercial product) Inhibitor are depicted in FIG. 6 which plots the OD450 against the concentration of protein in each sample. The samples were hFc IgG1-C1-INH, hFc LALA IgG1-C1-INH, and hFc IgG4m-C1-INH fusions, recombinant human C1-INH (rhC1-INH) (expressed in 1080 cells), and IgG1 Fc-human follistatin(hFc IgG1-hFst-XTEN) fusion as a positive control and human follistatin-Xten (hFst-XTEN) fusion as a negative control.

### Fc Fusion Protein Binding to FcγR1

Binding to the extracellular domain of the FcγR1 was measured by surface plasmon resonance (SPR) using a Biacore system. The Biacore capture approach is depicted in FIG. 7. The assay setup was as follows:
HBS-P+ used as running buffer
Coupling anti-His (GE, anti-His Kit) on CM5 to 16000Ru

### Capture level

For FcγRI (5 µg/mL R&D system), capture level at ∼350 Ru

### Sample preparation

Positive control: N-hFc-C1-INH is diluted in HBS-P+ buffer to 12.5, 6.25, 3.175, 1.59 and 0.79 nM
Other samples (N-hFcIgG4m-C1 Inh, N-hFcIgG4m-C1-INH Tr, N-hFcLala-C1-INH and N-hFclala-C1-INH Tr) are diluted in HBS-P+ buffer to 100, 50, 25, 12.5, 6.25 nM

### Binding Kinetic setup:

Capture: 12s injection at 10 µl/mL
Capture stabilization: 30s
Association: 300s at Flow rate 30 µl/mL
Dissociation: 600s at Flow rate 30 µl/mL
Regeneration: 40s at 30 µl/mL with 10 mM phosphate 500mM NaCl pH 2.5

Summary of exemplary data illustrating C1-INH constructs binding with FcγRI is shown in Table 3. N-hFcLala-C1-INH abolished binding with FcγRI. N-hFcIgG4m-C1-INH and N-hFcIgG4m-C1-INH Tr binds to FcγR1, but weaker than N-hFc-C1-INH. Truncated C1-INH with hFcLala or hFcIgG4m fusion increases binding affinity with FcγRI.

**Table 3: Summary of C1-INH construct FcγRI binding**

| **Samples** | **ka1 (1/Ms)** | **kd1 (1/s)** | **KD** |
|---|---|---|---|
| **N-hFc-C1 Inh** | 2.496E+5 | 5.277E-4 | 2.114E-9 |
| **N-hFcLala-C1 Inh** | No binding | | |
| **N-hFcLala-C1-INH Tr** | 1.403E+4 | 0.001833 | 1.306E-7 |
| **N-hFcIgG4m-C1 Inh** | 7.232E+4 | 0.001673 | 2.313E-8 |
| **N-hFcIgG4m-C1-INH Tr** | 1.183E+5 | 8.616E-4 | 7.281E-9 |

### C1-inhibitor Activity of the C1-inhibitor Fc Fusion Proteins

C1-inhibitor is capable of inhibiting many enzymes from the coagulation, contact and complement pathways, and thus useful in the treatment of diseases such as HAE, AMR, NMOSD, and PNH.

C1-INH is a suicide inhibitor, meaning it is inactivated during the process of inhibition. It forms a 1:1 stoichiometric complex with the target protease, followed by clearance of the entire complex. The C1-INH is then no longer available to inhibit other enzymes. The following experiments examined the ability of the Fc-C1-inhibitor fusion proteins to inhibit C1s by two exemplary methods.

Two exemplary in vitro approaches were taken to examine the inhibitory activity of C1-inhibitor on C1s activity: measuring the complex of C1S and C1-inhibitor in an ELISA based method (functional ELISA)- only used on effector dead constructs and measuring the inhibition of the ability of C1s to cleave a colorimetric substrate. The two assays resulted in agreeing data.

### Functional ELISA measuring the complex of C1S and C1-inhibitor

This method was used to test the effector dead Fc constructs made for PK studies FIG. 8. The ELISA protocol was as follows:
- 60 µL diluted C1-INH + 12 µL biotin-C1s
   ∘ 30 min incubation at RT
- Transfer 50 µL of complexes to streptavidin coated plate
   ∘ 10 min incubation at RT, wash 5x
- Add 50 µL of anti-C1-INH-HRP
   ∘ 60 min incubation at RT, wash 5x
- Add 100 µL of TMB substrate
   ∘ 15 minute incubation at RT
- Add 100 µL of Stop solution (4%HCl)
- Read at OD450

Plasma-derived C1-INH molecular weight (MW) was estimated by gel to be about 93,000 Da. This value was used to calculate the concentration of protein used in the SPR analysis. The concentration of the constructs was calculated by the weight of each construct as determined using mass spectrometry. FL C1-INH hFc IgG1 LALA and FL C1-INH hFc IgG4m have MW of approximately 200,000 Da. The Tr C1-INH hFc IgG1 LALA and Tr C1-INH hFc IgG4m have MW of approximately 160,000 Da.

In multiple tests, the fusion proteins demonstrated a higher affinity to C1s over native C1-inhibitor. The truncated C1-INH fusion protein similarly demonstrated a higher affinity to C1s over native C1-inhibitor.

### Inhibition of the ability of C1s to cleave a colorimetric substrate

The ability of the effector dead constructs' ability to inhibit C1s cleavage of a colorimetric peptide by C1s +/- C1-INH was tested using an R&D systems activity assay for C1-inhibitor. The assay protocol can be found at https://www.rndsystems.com/products/human-serpin-g1-c1-inhibitor-plasma-protein-cf_2488-pi.

### Final Assay Conditions Per Well:

- rhC1s: 0.010 µg (1.33 nM)
- hSerpin G1 curve: 100, 50, 25, 5, 2.5, 1.25, 0.625, 0.2, 0.04 and 0.01 nM
- Substrate: 100 µM
- DTNB: 100 µM

### Activity Assay setup

25 µL C1-inhibitor + 25 µL C1s (2µg/mL)
30 minute incubation at RT
Dilute complexes 5 fold with assay buffer
50 µL of diluted C1-INH-C1s complexes + 50 µL of substrate(500uM)/reactant(200uM)
15 minute incubation at RT
Read at OD405

Titration curves for each of the effector dead constructs tested in the assay were calculated using mass spec or gel estimates for molecular weights as shown in FIGs. 9A and 9B. The full length fusion protein demonstrated a higher affinity to C1s over native C1-inhibitor. The truncated C1-INH fusion protein similarly demonstrated a higher affinity to C1s over native C1-inhibitor.

### Hemolysis assay

FIGs. 10A and 10B depict a schematic overview of hemolysis assays of the alternative and classical complement pathways. Seelen ct al., J. of Immun. Methods, 296: 187-198 (2005). FIGs. 11A, 11B, and 11C depict the comparison of hemolysis activity, e.g, the degree of activation of the alternative pathway of complement, exhibited by plasma-derived C1-INH, IgG1 LALA Fc truncated C1-inhibitor, IgG1 LALA Full Length C1-inhibitor, and two preparation of plasma-derived C1-INH. Fusion proteins behaved similarly to plasma-derived C1 inhibitor.

### Summary of C1-inhibitor Fc fusion Protein Activity

All of the Fc-C1-inhibitor fusion proteins are able to inhibit C1s activity. The Fc fusion proteins, in particular, full length C1-inhibitor fusion proteins consistently inhibit the C1s with a higher affinity than the plasma-derived C1-inhibitor. Both full length and truncated C1-inhibitor fusion proteins (e.g. made with Fc IgG1 LALA) are able to inhibit lysis of red blood cells in vitro.

### In vivo PK profiles of Fc Fusion Proteins

FIG. 12 shows exemplary results of a Rabbit PK study of the effector dead fusion constructs compared with plasma-derived C1-inhibitor and recombinant C1-INH. Plasma-derived C1-INH administered by IV exhibits a monophasic serum concentration-time profile. rhC1-INH-IgG constructs administered by IV exhibit bi-phasic serum concentration-time profiles. The initial rapid clearance phase (approximately 2 hrs) was indicative of receptor-mediated cellular uptake. The constructs exhibited a secondary slower clearance phase.

### Example 3. Albumin Fusion Proteins

### A. Albumin-C1-Inhibitor Fusion Expression Constructs

A number of albumin protein constructs were generated. Schematics of these constructs are depicted in FIGs. 13A-F. Fusion construct expression vectors comprising albumin or the D3 domain of albumin joined directly to the N-terminus of full length and truncated C1-INH joined directly to the N-terminus of full length C1-INH were made. Fusion construct expression vectors comprising albumin or the D3 domain of albumin joined using linkers to the N-terminus of full length and truncated C1-INH joined directly to the N-terminus of full length C1-INH were also made. The amino acid sequences of the fusion proteins expressed by these constructs comprised the following sequences:

Albumin full length C1-INH Direct Fusion (the albumin domain is underlined):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLNPNATSSSSQDPESLQDRGEGKVATTVISKMLFVE
PILEVSSLPTTNSTTNSATKITANTTDEPTTQPTTEPTTQPTIQPTQPTTQLPTDSPTQPTTG
SFCPGPVTLCSDLESHSTEAVLGDALVDFSLKLYHAFSAMKKVETNMAFSPFSIASLLTQ
VLLGAGENTKTNLESILSYPKDFTCVHQALKGFTTKGVTSVSQIFHSPDLAIRDTFVNAS
RTLYSSSPRVLSNNSDANLELINTWVAKNTNNKISRLLDSLPSDTRLVLLNAIYLSAKWK
TTFDPKKTRMEPFHFKNSVIKVPMMNSKKYPVAHFIDQTLKAKVGQLQLSHNLSLVILV
PQNLKHRLEDMEQALSPSVFKAIMEKLEMSKFQPTLLTLPRIKVTTSQDMLSIMEKLEFF
DFSYDLNLCGLTEDPDLQVSAMQHQTVLELTETGVEAAAASAISVARTLLVFEVQQPFL
FVLWDQQHKFPVFMGRVYDPRA (SEQ ID NO:18).

Albumin truncated C1-INH Direct Fusion (the albumin domain is underlined):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ

DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGSFCPGPVTLCSDLESHSTEAVLGDALVDFSLKLY
HAFSAMKKVETNMAFSPFSIASLLTQVLLGAGENTKTNLESILSYPKDFTCVHQALKGFT
TKGVTSVSQIFHSPDLAIRDTFVNASRTLYSSSPRVLSNNSDANLELINTWVAKNTNNKIS
RLLDSLPSDTRLVLLNAIYLSAKWKTTFDPKKTRMEPFHFKNSVIKVPMMNSKKYPVAH
FIDQTLKAKVGQLQLSHNLSLVILVPQNLKHRLEDMEQALSPSVFKAIMEKLEMSKFQP
TLLTLPRIKVTTSQDMLSIMEKLEFFDFSYDLNLCGLTEDPDLQVSAMQHQTVLELTETG
VEAAAASAISVARTLLVFEVQQPFLFVLWDQQHKFPVFMGRVYDPRA (SEQ ID NO:19).

D3 Albumin full length C1-INH Direct Fusion (the D3 Albumin domain is underlined):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLNPNATSSSS
QDPESLQDRGEGKVATTVISKMLFVEPILEVSSLPTTNSTTNSATKITANTTDEPTTQPTT
EPTTQPTIQPTQPTTQLPTDSPTQPTTGSFCPGPVTLCSDLESHSTEAVLGDALVDFSLKL
YHAFSAMKKVETNMAFSPFSIASLLTQVLLGAGENTKTNLESILSYPKDFTCVHQALKG
FTTKGVTSVSQIFHSPDLAIRDTFVNASRTLYSSSPRVLSNNSDANLELINTWVAKNTNN
KISRLLDSLPSDTRLVLLNAIYLSAKWKTTFDPKKTRMEPFHFKNSVIKVPMMNSKKYP
VAHFIDQTLKAKVGQLQLSHNLSLVILVPQNLKHRLEDMEQALSPSVFKAIMEKLEMSK
FQPTLLTLPRIKVTTSQDMLSIMEKLEFFDFSYDLNLCGLTEDPDLQVSAMQHQTVLELT
ETGVEAAAASAISVARTLLVFEVQQPFLFVLWDQQHKFPVFMGRVYDPRA (SEQ ID
NO:21).

D3 Albumin truncated C1-INH Direct Fusion (the D3 Albumin domain is underlined):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGSFCPGPVT
LCSDLESHSTEAVLGDALVDFSLKLYHAFSAMKKVETNMAFSPFSIASLLTQVLLGAGE
NTKTNLESILSYPKDFTCVHQALKGFTTKGVTSVSQIFHSPDLAIRDTFVNASRTLYSSSP
RVLSNNSDANLELINTWVAKNTNNKISRLLDSLPSDTRLVLLNAIYLSAKWKTTFDPKK
TRMEPFHFKNSVIKVPMMNSKKYPVAHFIDQTLKAKVGQLQLSHNLSLVILVPQNLKHR
LEDMEQALSPSVFKAIMEKLEMSKFQPTLLTLPRIKVTTSQDMLSIMEKLEFFDFSYDLN
LCGLTEDPDLQVSAMQHQTVLELTETGVEAAAASAISVARTLLVFEVQQPFLFVLWDQ
QHKFPVFMGRVYDPRA (SEQ ID NO:22).

Albumin full length C1-INH GGG Linker Fusion (the Albumin domain is underlined, the linker is bolded):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGGGNPNATSSSSQDPESLQDRGEGKVATTVISKM
LFVEPILEVSSLPTTNSTTNSATKITANTTDEPTTQPTTEPTTQPTIQPTQPTTQLPTDSPTQ
PTTGSFCPGPVTLCSDLESHSTEAVLGDALVDFSLKLYHAFSAMKKVETNMAFSPFSIAS
LLTQVLLGAGENTKTNLESILSYPKDFTCVHQALKGFTTKGVTSVSQIFHSPDLAIRDTFV
NASRTLYSSSPRVLSNNSDANLELINTWVAKNTNNKISRLLDSLPSDTRLVLLNAIYLSA
KWKTTFDPKKTRMEPFHFKNSVIKVPMMNSKKYPVAHFIDQTLKAKVGQLQLSHNLSL
VILVPQNLKHRLEDMEQALSPSVFKAIMEKLEMSKFQPTLLTLPRIKVTTSQDMLSIMEK
LEFFDFSYDLNLCGLTEDPDLQVSAMQHQTVLELTETGVEAAAASAISVARTLLVFEVQ
QPFLFVLWDQQHKFPVFMGRVYDPRA (SEQ ID NO:23).

Albumin truncated CI-INH GGG Linker Fusion (the Albumin domain is underlined, the linker is bolded):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGGGGSFCPGPVTLCSDLESHSTEAVLGDALVDFSL
KLYHAFSAMKKVETNMAFSPFSIASLLTQVLLGAGENTKTNLESILSYPKDFTCVHQAL
KGFTTKGVTSVSQIFHSPDLAIRDTFVNASRTLYSSSPRVLSNNSDANLELINTWVAKNT
NNKISRLLDSLPSDTRLVLLNAIYLSAKWKTTFDPKKTRMEPFHFKNSVIKVPMMNSKK
YPVAHFIDQTLKAKVGQLQLSHNLSLVILVPQNLKHRLEDMEQALSPSVFKAIMEKLEM
SKFQPTLLTLPRIKVTTSQDMLSIMEKLEFFDFSYDLNLCGLTEDPDLQVSAMQHQTVLE
LTETGVEAAAASAISVARTLLVFEVQQPFLFVLWDQQHKFPVFMGRVYDPRA (SEQ ID
NO:24).

D3 Albumin full length C1-INH GGG Linker Fusion (the D3 Albumin domain is underlined, the linker is bolded):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGNPNATS
SSSQDPESLQDRGEGKVATTVISKMLFVEPILEVSSLPTTNSTTNSATKITANTTDEPTTQP
TTEPTTQPTIQPTQPTTQLPTDSPTQPTTGSFCPGPVTLCSDLESHSTEAVLGDALVDFSLK
LYHAFSAMKKVETNMAFSPFSIASLLTQVLLGAGENTKTNLESILSYPKDFTCVHQALK
GFTTKGVTSVSQIFHSPDLAIRDTFVNASRTLYSSSPRVLSNNSDANLELINTWVAKNTN
NKISRLLDSLPSDTRLVLLNAIYLSAKWKTTFDPKKTRMEPFHFKNSVIKVPMMNSKKY
PVAHFIDQTLKAKVGQLQLSHNLSLVILVPQNLKHRLEDMEQALSPSVFKAIMEKLEMS
KFQPTLLTLPRIKVTTSQDMLSIMEKLEFFDFSYDLNLCGLTEDPDLQVSAMQHQTVLEL
TETGVEAAAASAISVARTLLVFEVQQPFLFVLWDQQHKFPVFMGRVYDPRA (SEQ ID
NO:25).

D3 Albumin truncated C1-INH GGG Linker Fusion (the D3 Albumin domain is underlined, the linker is bolded):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGGSFCPG
PVTLCSDLESHSTEAVLGDALVDFSLKLYHAFSAMKKVETNMAFSPFSIASLLTQVLLG
AGENTKTNLESILSYPKDFTCVHQALKGFTTKGVTSVSQIFHSPDLAIRDTFVNASRTLYS
SSPRVLSNNSDANLELINTWVAKNTNNKISRLLDSLPSDTRLVLLNAIYLSAKWKTTFDP
KKTRMEPFHFKNSVIKVPMMNSKKYPVAHFIDQTLKAKVGQLQLSHNLSLVILVPQNLK
HRLEDMEQALSPSVFKAIMEKLEMSKFQPTLLTLPRIKVTTSQDMLSIMEKLEFFDFSYD
LNLCGLTEDPDLQVSAMQHQTVLELTETGVEAAAASAISVARTLLVFEVQQPFLFVLWD
QQHKFPVFMGRVYDPRA (SEQ ID NO:26).

Albumin full length C1-INH (GGGGS)2 Linker Fusion (the Albumin domain is underlined):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA

DDKETCFAEEGKKLVAASRAALGLGGGGSGGGGSNPNATSSSSQDPESLQDRGEGKV
ATTVISKMLFVEPILEVSSLPTTNSTTNSATKITANTTDEPTTQPTTEPTTQPTIQPTQPTTQ
LPTDSPTQPTTGSFCPGPVTLCSDLESHSTEAVLGDALVDFSLKLYHAFSAMKKVETNM
AFSPFSIASLLTQVLLGAGENTKTNLESILSYPKDFTCVHQALKGFTTKGVTSVSQIFHSP
DLAIRDTFVNASRTLYSSSPRVLSNNSDANLELINTWVAKNTNNKISRLLDSLPSDTRLV
LLNAIYLSAKWKTTFDPKKTRMEPFHFKNSVIKVPMMNSKKYPVAHFIDQTLKAKVGQ
LQLSHNLSLVILVPQNLKHRLEDMEQALSPSVFKAIMEKLEMSKFQPTLLTLPRIKVTTS
QDMLSIMEKLEFFDFSYDLNLCGLTEDPDLQVSAMQHQTVLELTETGVEAAAASAISVA
RTLLVFEVQQPFLFVLWDQQHKFPVFMGRVYDPRA (SEQ ID NO:28).

Albumin truncated C1-INH (GGGGS)2 Linker Fusion (the Albumin domain is underlined, the linker is bolded):

MKWVTFISLLFLFSSAYSRGVFRRDAHKSEVAHRFKDLGEENFKALVLIAFAQYLQQCP
FEDHVKLVNEVTEFAKTCVADESAENCDKSLHTLFGDKLCTVATLRETYGEMADCCAK
QEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERA
FKAWAVARLSQRFPKAEFAEVSKLVTDLTKVHTECCHGDLLECADDRADLAKYICENQ
DSISSKLKECCEKPLLEKSHCIAEVENDEMPADLPSLAADFVESKDVCKNYAEAKDVFL
GMFLYEYARRHPDYSVVLLLRLAKTYKTTLEKCCAAADPHECYAKVFDEFKPLVEEPQ
NLIKQNCELFEQLGEYKFQNALLVRYTKKVPQVSTPTLVEVSRNLGKVGSKCCKHPEAK
RMPCAEDYLSVVLNQLCVLHEKTPVSDRVTKCCTESLVNRRPCFSALEVDETYVPKEFN
AETFTFHADICTLSEKERQIKKQTALVELVKHKPKATKEQLKAVMDDFAAFVEKCCKA
DDKETCFAEEGKKLVAASRAALGLGGGGSGGGGSGSFCPGPVTLCSDLESHSTEAVLG
DALVDFSLKLYHAFSAMKKVETNMAFSPFSIASLLTQVLLGAGENTKTNLESILSYPKDF
TCVHQALKGFTTKGVTSVSQIFHSPDLAIRDTFVNASRTLYSSSPRVLSNNSDANLELINT
WVAKNTNNKISRLLDSLPSDTRLVLLNAIYLSAKWKTTFDPKKTRMEPFHFKNSVIKVP
MMNSKKYPVAHFIDQTLKAKVGQLQLSHNLSLVILVPQNLKHRLEDMEQALSPSVFKAI
MEKLEMSKFQPTLLTLPRIKVTTSQDMLSIMEKLEFFDFSYDLNLCGLTEDPDLQVSAM
QHQTVLELTETGVEAAAASAISVARTLLVFEVQQPFLFVLWDQQHKFPVFMGRVYDPR
A (SEQ ID NO:29).

D3 Albumin full length C1-INH (GGGGS)2 Linker Fusion (the D3 Albumin domain is underlined, the linker is bolded):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGGSGGG
GSNPNATSSSSQDPESLQDRGEGKVATTVISKMLFVEPILEVSSLPTTNSTTNSATKITAN
TTDEPTTQPTTEPTTQPTIQPTQPTTQLPTDSPTQPTTGSFCPGPVTLCSDLESHSTEAVLG
DALVDFSLKLYHAFSAMKKVETNMAFSPFSIASLLTQVLLGAGENTKTNLESILSYPKDF
TCVHQALKGFTTKGVTSVSQIFHSPDLAIRDTFVNASRTLYSSSPRVLSNNSDANLELINT
WVAKNTNNKISRLLDSLPSDTRLVLLNAIYLSAKWKTTFDPKKTRMEPFHFKNSVIKVP
MMNSKKYPVAHFIDQTLKAKVGQLQLSHNLSLVILVPQNLKHRLEDMEQALSPSVFKAI
MEKLEMSKFQPTLLTLPRIKVTTSQDMLSIMEKLEFFDFSYDLNLCGLTEDPDLQVSAM

QHQTVLELTETGVEAAAASAISVARTLLVFEVQQPFLFVLWDQQHKFPVFMGRVYDPR
A (SEQ ID NO:30).

D3 Albumin truncated C1-INH (GGGGS)2 Linker Fusion (the D3 Albumin domain is underlined):

METPAQLLFLLLLWLPDTTGVEEPQNLIKQNCELFEQLGEYKFQNALLVRYTKKVPQVS
TPTLVEVSRNLGKVGSKCCKHPEAKRMPCAEDYLSVVLNQLCVLHEKTPVSDRVTKCC
TESLVNRRPCFSALEVDETYVPKEFNAETFTFHADICTLSEKERQIKKQTALVELVKHKP
KATKEQLKAVMDDFAAFVEKCCKADDKETCFAEEGKKLVAASRAALGLGGGGSGGG
GSGSFCPGPVTLCSDLESHSTEAVLGDALVDFSLKLYHAFSAMKKVETNMAFSPFSIASL
LTQVLLGAGENTKTNLESILSYPKDFTCVHQALKGFTTKGVTSVSQIFHSPDLAIRDTFV
NASRTLYSSSPRVLSNNSDANLELINTWVAKNTNNKISRLLDSLPSDTRLVLLNAIYLSA
KWKTTFDPKKTRMEPFHFKNSVIKVPMMNSKKYPVAHFIDQTLKAKVGQLQLSHNLSL
VILVPQNLKHRLEDMEQALSPSVFKAIMEKLEMSKFQPTLLTLPRIKVTTSQDMLSIMEK
LEFFDFSYDLNLCGLTEDPDLQVSAMQHQTVLELTETGVEAAAASAISVARTLLVFEVQ
QPFLFVLWDQQHKFPVFMGRVYDPRA (SEQ ID NO:31).

### B. Expression of Albumin Fusion Proteins

FreeStyle™ CHO-S Cells were transfected with these fusion construct expression vectors as described above CHO-GS stable pools were prepared and used to generate CM for collection and analysis. Cells were seeded at 2x10⁶ cells/mL and incubated for 4 days at 33°C. Glutamine was supplemented on day 3.

Conditioned Medium (CM) from the transfected cell cultures was harvested 4 days post transfection and run on Coomassie gel to evaluate expression. The 4 day batch harvests were concentrated using a 50 kDa Vivaspin®20 centrifugal concentrator (Vivaproducts, Inc., Cat. No. VS2031) and purified using Invitrogen's CaptureSelect Albumin Affinity Matrix (Invitrogen, Cat. No. 19129701L). The elution buffer was 20mM Tris pH 7.4 + 2M MgCl₂). Samples were run on Coomassie gel against BSA to evaluate expression.

The yield based on this purification for HSA-C1-INH was approximately 3.6 mg/L and for D3-C1-INH was approximately 1.6 mg/L. Proteins were concentrated to ∼2 mg/mL using Vivaspin® 50 kDa and stored at -20°C. Purified proteins were dialyzcd overnight into C1-Inh formulation buffer (50 mM Tris pH7.2, 50 mM sorbitol, 150 mM glycine) and concentrated.

Proteins were checked for C1s activity. The data is shown in FIG. 14, which presents the results of an assay to measure the ability of some exemplary albumin C1-INH fusion constructs to inhibit C1s cleavage of a colorimetric peptide, including a plasma-derived C1-INH and HT1080 expressed recombinant C1-INH for comparison.

Proteins were run on SEC-MALS to check for aggregation and to determine size. Exemplary parameters of the assay were:
Column: Tosoh G3000swxl
Mobile Phase Buffer: 0.2M sodium phosphate pH 6.8
Flow Rate: 0.5 mL/min
Samples:
   HSA D3 C1-INH inject 30 µg
   HSA C1-INH inject 35 µg
   BSA control inject 40 µg
   C1-INH IgG1 LALA Fc injected at 35 µg

SEC-MALS determination was used for HSA-C1-INH molecules using the SEC method developed for 293 C1-INH samples. No large molecular weight molecules in the HSA samples were detected using this method.

Dynamic light scattering (DLS) analysis of the C1-inhibitor albumin fusion proteins was then performed. HSA and C1-INH C46 were used as controls. All samples and controls were diluted into 0.5mg/mL in DPBS buffer. Exemplary DLS results of C1-inhibitor Albumin fusion proteins are shown in Table 4.

**Table 4: DLS Analysis of C1-inhibitor Albumin fusion proteins**

| **Sample** | **mg/mL** |
|---|---|
| HSA | 5 |
| C46 C1-INH | 2.5 |
| HSA-GGG-C1-INH CHO-GNE | 3.5 |
| D3-GGG-C1-INH CHO-GNE | 8 |
| D3-GGGGS-C1-INH CHO-GNE | 3.4 |

Purification of both HSA-C1-INH as well as HSA_D3-C1-INH using Invitrogen CaptureSelect Human Albumin Affinity Matrix from CHO-GS CM worked well. Purified proteins had C1s activity comparable to plasma-derived C1-INH. SEC-MALS showed only a single peak with no HMW species at the expected size.

Stable pools expressing Albumin-C1-INH +/- linker constructs were generated. CHO-GS GNE cells were transfected via electroporation following manufacturer's protocol. Stable pools were selected and a 7 day batch harvest production was performed at 33°C. Batch harvests were evaluated by Coomassie Gel for expression. Coomassie results show Full-length HSA-C1-INH fusion proteins show less expression than HSA_D3 fusions. No significant differences were seen with the addition of a linker sequence.

### EQUIVALENTS AND SCOPE

Those skilled in the art will recognize, or be able to ascertain using no more than routine experimentation, many equivalents to the specific embodiments of the invention described herein. The scope of the present invention is not intended to be limited to the above Description, but rather is as set forth in the following claims:

### FURTHER EMBODIMENTS

1. A fusion protein comprising:
   a human C1-inhibitor polypeptide; and
   an Fc domain.
2. The fusion protein of embodiment 1, wherein the human C1-inhibitor polypeptide comprises an amino acid sequence at least 70%, 75%, 80%, 85%, 90%, or 95% identical to the full length human C1-inhibitor having SEQ ID NO:1.
3. The fusion protein of embodiment 1, wherein the human C1-inhibitor polypeptide comprises SEQ ID NO:1.
4. The fusion protein of embodiment 1, wherein the human C1-inhibitor polypeptide is truncated compared to SEQ ID NO:1.
5. The fusion protein of embodiment 4, wherein the human C1-inhibitor polypeptide comprises an amino acid sequence at least 70%, 75%, 80%, 85%, 90%, or 95% identical to SEQ ID NO:2.
6. The fusion protein of embodiment 4, wherein the human C1-inhibitor polypeptide comprises the amino acid sequence of SEQ ID NO:2.
7. The fusion protein of any one of the preceding embodiments, wherein the Fc domain is attached to the N-terminus of the human C1-inhibitor polypeptide.
8. The fusion protein of any one of the preceding embodiments, wherein the Fc domain is a human IgG1 Fc domain.
9. The fusion protein of embodiment 8 wherein the Fc domain comprises an amino acid sequence at least 70%, 75%, 80%, 85%, 90%, or 95% identical to SEQ ID NO:3.
10. The fusion protein of any one of the preceding embodiments, wherein the Fc domain comprises L234A and/or L235A mutations.
11. The fusion protein of embodiment 10, wherein the Fc domain comprises the amino acid sequence of SEQ ID NO:4.
12. The fusion protein of any one of the preceding embodiments, wherein the Fc domain comprises one or more mutations that prolong the half-life of the fusion protein.
13. The fusion protein of embodiment 12, wherein the one or more mutations are selected from one or more positions corresponding to Thr 250, Met 252, Ser 254, Thr 256, Thr 307, Glu 380, Met 428, His 433, and/or Asn 434 of human IgG1.
14. The fusion protein of embodiment 12, wherein the one or more mutations are selected from H433K and/or N434F.
15. The fusion protein of any one of embodiments 12-14, wherein the Fc domain comprises an amino acid sequence at least 80% identical to any one of SEQ ID NOs:5-8.
16. The fusion protein of embodiment 14, wherein the Fc domain comprises the amino acid sequence of any one of SEQ ID NOs:5-8.
17. The fusion protein of any one of embodiments 1-7, wherein the Fc domain is derived from human IgG4 Fc.
18. The fusion protein of embodiment 17 wherein the Fc domain comprises an amino acid sequence at least 70%, 75%, 80%, 85%, 90%, or 95% identical to SEQ ID NO:9.
19. The fusion protein of embodiment 18, wherein the Fc domain comprises an S241P mutation.
20. The fusion protein of embodiment 19, wherein the Fc domain comprises the amino acid sequence of SEQ ID NO: 10.
21. The fusion protein of embodiment 1, comprising an amino acid sequence at least 70%, 75%, 80%, 85%, 90%, or 95% identical to SEQ ID NO: 11.
22. The fusion protein of embodiment 1, comprising an amino acid sequence at least 70%, 75%, 80%, 85%, 90%, or 95% identical to SEQ ID NO:12.
23. The fusion protein of embodiment 1, comprising an amino acid sequence at least 70%, 75%, 80%, 85%, 90%, or 95% identical to SEQ ID NO:13.
24. The fusion protein of embodiment 1, comprising an amino acid sequence at least 70%, 75%, 80%, 85%, 90%, or 95% identical to SEQ ID NO:14.
25. The fusion protein of embodiment 1, comprising an amino acid sequence at least 70%, 75%, 80%, 85%, 90%, or 95% identical to SEQ ID NO:15.
26. The fusion protein of embodiment 1, comprising an amino acid sequence at least 70%, 75%, 80%, 85%, 90%, or 95% identical to SEQ ID NO:16.
27. The fusion protein of embodiment 1, comprising an amino acid sequence selected from SEQ ID NO:11, SEQ ID NO:12, SEQ ID NO:13, SEQ ID NO:14, SEQ ID NO:15, or SEQ ID NO:16.
28. The fusion protein of any one of the preceding embodiments, wherein the Fc domain comprises a mutation that reduces or eliminates ADCC activity.
29. The fusion protein of any one of the preceding embodiments, wherein the Fc domain comprises a mutation that reduces or eliminates CDC activity.
30. The fusion protein of any one of the preceding embodiments, wherein the Fc domain comprises a mutation that reduces or eliminates FcγR binding.
31. The fusion protein of any one of the preceding embodiments, wherein the Fc domain comprises a mutation that reduces or eliminates FcγR effector function.
32. The fusion protein of any one of the preceding embodiments, wherein the recombinant human C1-inhibitor Fc fusion protein comprises a mutation that reduces or eliminates C1q binding.
33. The fusion protein of embodiment 32, wherein the mutation that reduces or eliminates C1q binding is in the Fc domain.
34. The fusion protein of any one of the preceding embodiments, further comprising a linker between the human C1-inhibitor polypeptide and the Fc domain.
35. The fusion protein of embodiment 34, wherein the linker is a peptide comprising 3-100 amino acids.
36. The fusion protein of any one of the preceding embodiments, wherein the fusion protein inhibits and/or inactivates C1r and/or C1s protease activity.
37. The fusion protein of any one of the preceding embodiments, wherein the fusion protein inhibits the lysis of red blood cells *in vitro.*
38. The fusion protein of any one of the preceding embodiments, wherein the fusion protein has a longer half-life than plasma-derived human C1-inhibitor.
39. The fusion protein of any one of the preceding embodiments, wherein the fusion protein has a half-life of at least four days.
40. The fusion protein of any one of the preceding embodiments, wherein the fusion protein has a half-life of at least five days.
41. The fusion protein of any one of the preceding embodiments, wherein the fusion protein has a half-life of at least six days.
42. The fusion protein of any one of the preceding embodiments, wherein the fusion protein has a half-life of at least seven days.
43. The fusion protein of any one of the preceding embodiments, wherein the fusion protein is monovalent.
44. The fusion protein of any one of embodiments 1-42, wherein the fusion protein is dimeric.
45. A fusion protein comprising:
   a human C1-inhibitor polypeptide; and
   an albumin polypeptide.
46. The fusion protein of embodiment 45 wherein the human C1-inhibitor polypeptide comprises an amino acid sequence at least 70%, 75%, 80%, 85%, 90%, or 95% identical to the human full length C1-inhibitor having SEQ ID NO:1.
47. The fusion protein of embodiment 46, wherein the human C1-inhibitor polypeptide comprises SEQ ID NO:1.
48. The fusion protein of any one of embodiments 45-47, wherein the human C1-inhibitor polypeptide is truncated compared to SEQ ID NO:1.
49. The fusion protein of embodiment 48, wherein the human C1-inhibitor polypeptide comprises an amino acid sequence at least 70%, 75%, 80%, 85%, 90%, or 95% identical to SEQ ID NO:2.
50. The fusion protein of embodiment 49, wherein the human C1-inhibitor polypeptide comprises the amino acid sequence of SEQ ID NO:2.
51. The fusion protein of any one of embodiments 45-50, wherein the albumin is attached to the N-terminus of the human C1-inhibitor polypeptide.
52. The fusion protein of any one of embodiments 45-51, wherein the albumin polypeptide comprises one or more domains of human serum albumin.
53. The fusion protein of embodiment 52, wherein the albumin polypeptide comprises the D3 domain of human serum albumin.
54. The fusion protein of embodiment 53, wherein the albumin polypeptide comprises an amino acid sequence at least 70%, 75%, 80%, 85%, 90%, or 95% identical to SEQ ID NO:20.
55. The fusion protein of embodiment 54, wherein the albumin polypeptide comprises the amino acid sequence of SEQ ID NO:20.
56. The fusion protein of any one of embodiments 45-52, wherein the albumin polypeptide comprises an amino acid sequence at least 70%, 75%, 80%, 85%, 90%, or 95% identical to SEQ ID NO: 17.
57. The fusion protein of embodiment 56, wherein the albumin polypeptide comprises the amino acid sequence of SEQ ID NO: 17.
58. The fusion protein of any one of embodiments 45-57 comprising an amino acid sequence at least 70%, 75%, 80%, 85%, 90%, or 95% identical to SEQ ID NO: 18.
59. The fusion protein of any one of embodiments 45-57 comprising an amino acid sequence at least 70%, 75%, 80%, 85%, 90%, or 95% identical to SEQ ID NO:19.
60. The fusion protein of any one of embodiments 45-57 comprising an amino acid sequence at least at least 70%, 75%, 80%, 85%, 90%, or 95% identical to SEQ ID NO:21.
61. The fusion protein of any one of embodiments 45-57 comprising an amino acid sequence at least 70%, 75%, 80%, 85%, 90%, or 95% identical to SEQ ID NO:22.
62. The fusion protein of any one of embodiments 44-56 comprising an amino acid sequence identical to SEQ ID NO: 17, SEQ ID NO: 18, SEQ ID NO: 19, SEQ ID NO:21, or SEQ ID NO:22.
63. The fusion protein of any one of embodiments 45-62, wherein the fusion protein binds FcRN.
64. The fusion protein of any one of embodiments 45-63, wherein the fusion protein inhibits C1 esterase activity.
65. The fusion protein of any one of embodiments 45-64, wherein the fusion protein inhibits the lysis of red blood cells *in vitro.*
66. The fusion protein of any one of embodiments 45-65, further comprising a linker between the human C1-inhibitor polypeptide and the albumin polypeptide.
67. The fusion protein of embodiment 66, wherein the linker is a peptide comprising 3-100 amino acids.
68. The fusion protein of embodiment 67, wherein the linker comprises the sequence GGG
69. The fusion protein of embodiment 67, wherein the linker comprises the sequence of SEQ ID NO:27.
70. The fusion protein of any one of embodiments 45-69, wherein the albumin polypeptide does not comprise one or more mutations selected from the group consisting of 464His, 510 His, 535 His, and combination thereof.
71. The fusion protein of any one of embodiments 45-70, wherein the fusion protein has a longer half-life than plasma-derived human CI-inhibitor.
72. The fusion protein of any one of embodiments 45-71, wherein the fusion protein has a half-life of at least four days.
73. The recombinant human CI-inhibitor albumin fusion protein of any one of embodiments 45-71, wherein the fusion protein has a half-life of at least five days.
74. The fusion protein of any one of embodiments 45-71, wherein the fusion protein has a half-life of at least six days.
75. The fusion protein of any one of embodiments 45-71, wherein the fusion protein has a half-life of at least seven days.
76. A nucleic acid encoding a fusion protein of any one of the preceding embodiments.
77. A cell comprising a nucleic acid of embodiment 76.
78. The cell of embodiment 77, wherein the cell is a mammalian cell.
79. The cell of embodiment 78, wherein the mammalian cell is a human cell.
80. The cell of embodiment 78, wherein the mammalian cell is a Chinese Hamster Ovary (CHO) cell.
81. The cell of any one of embodiments 77-80, wherein the cell is engineered to modify glycosylation.
82. The cell of embodiment 81, wherein the cell is engineered to improve sialylation.
83. A method of producing a fusion protein comprising a step of cultivating a cell of any one of embodiments 77-82.
84. A fusion protein of any one of embodiments 1-75, wherein the fusion protein is produced by a cell engineered to improve sialylation.
85. A pharmaceutical composition comprising a fusion protein of any one of embodiments 1-75 and 84 and a pharmaceutically acceptable carrier.
86. A method of treating a complement-mediated disorder comprising administering to a subject in need of treatment the pharmaceutical composition of embodiment 85.
87. The method of embodiment 86, wherein the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, multifocal motor neuropathy.

## Claims

1. A fusion protein comprising:
a human C1-inhibitor polypeptide; and
an albumin polypeptide.

2. The fusion protein of claim 1, wherein the human C1-inhibitor polypeptide:
(a) comprises an amino acid sequence at least 70%, 75%, 80%, 85%, 90%, or 95% identical to the full length human C1-inhibitor having SEQ ID NO:1, optionally wherein the human CI-inhibitor polypeptide comprises SEQ ID NO:1; or
(b) is truncated compared to SEQ ID NO:1, optionally wherein the human C1-inhibitor polypeptide comprises an amino acid sequence at least 70%, 75%, 80%, 85%, 90%, or 95% identical to SEQ ID NO:2, such as wherein the human C1-inhibitor polypeptide comprises the amino acid sequence of SEQ ID NO:2.

3. The fusion protein of claim 1 or 2, wherein:
(a) the albumin is attached to the N-terminus of the human CI-inhibitor polypeptide;
(b) the albumin polypeptide comprises one or more domains of human serum albumin, optionally wherein the albumin polypeptide comprises the D3 domain of human serum albumin, such as wherein the albumin polypeptide comprises an amino acid sequence at least 70%, 75%, 80%, 85%, 90%, or 95% identical to SEQ ID NO:20, for instance the amino acid sequence of SEQ ID NO:20; and/or
(c) the albumin polypeptide comprises an amino acid sequence at least 70%, 75%, 80%, 85%, 90%, or 95% identical to SEQ ID NO: 17, optionally wherein the albumin polypeptide comprises the amino acid sequence of SEQ ID NO: 17.

4. The fusion protein of any one of claims 1-3, comprising an amino acid sequence:
(a) at least 70%, 75%, 80%, 85%, 90%, or 95% identical to SEQ ID NO: 18;
(b) at least 70%, 75%, 80%, 85%, 90%, or 95% identical to SEQ ID NO: 19;
(c) at least 70%, 75%, 80%, 85%, 90%, or 95% identical to SEQ ID NO:21;
(d) at least 70%, 75%, 80%, 85%, 90%, or 95% identical to SEQ ID NO:22; or
(e) identical to SEQ ID NO: 17, SEQ ID NO: 18, SEQ ID NO: 19, SEQ ID NO:21, or SEQ ID NO:22.

5. The fusion protein of any one of claims 1-4, wherein:
(a) the fusion protein binds FcRN;
(b) the fusion protein inhibits C1 esterase activity; and/or
(c) the fusion protein inhibits the lysis of red blood cells *in vitro.*

6. The fusion protein of any one of claims 1-5, further comprising a linker between the human CI-inhibitor polypeptide and the albumin polypeptide,
optionally wherein the linker is a peptide comprising 3-100 amino acids, such as wherein the linker comprises the sequence GGG or wherein the linker comprises the sequence of SEQ ID NO:27.

7. The fusion protein of any one of claims 1-6, wherein:
(a) the albumin polypeptide does not comprise one or more mutations selected from the group consisting of 464His, 510 His, 535 His, and combination thereof;
(b) the fusion protein has a longer half-life than plasma-derived human CI-inhibitor; and/or
(c) the fusion protein has a half-life of:
(i) at least four days;
(ii) at least five days;
(iii) at least six days; or
(iv) at least seven days.

8. A nucleic acid encoding a fusion protein of any one of claims 1-7.

9. A cell comprising a nucleic acid of claim 8, optionally wherein the cell is a mammalian cell, such as wherein the mammalian cell is a human cell or a Chinese Hamster Ovary (CHO) cell.

10. The cell of claim 9, wherein the cell is engineered to modify glycosylation, optionally wherein the cell is engineered to improve sialylation.

11. A method of producing a fusion protein comprising a step of cultivating a cell of claim 9 or claim 10.

12. A fusion protein of any one of claims 1-11, wherein the fusion protein is produced by a cell engineered to improve sialylation.

13. A pharmaceutical composition comprising a fusion protein of any one of claims 1-7 or 12 and a pharmaceutically acceptable carrier.

14. The pharmaceutical composition of claim 13 for use in a method of treating a complement-mediated disorder, the method comprising administering the composition to a subject in need of treatment.

15. The composition for use of claim 14, wherein the complement-mediated disorder is selected from hereditary angioedema, antibody mediated rejection, neuromyelitis optica spectrum disorders, traumatic brain injury, spinal cord injury, ischemic brain injury, burn injury, toxic epidermal necrolysis, multiple sclerosis, amyotrophic lateral sclerosis (ALS), Parkinson's disease, stroke, chronic inflammatory demyelinating polyneuropathy (CIDP), myasthenia gravis, or multifocal motor neuropathy.


SEQUENCE LISTING

<110> SHIRE HUMAN GENETIC THERAPIES, INC.

<120> C1 ESTERASE INHIBITOR FUSION PROTEINS AND USES THEREOF

<130> 2006685-1208

<140> PCT/US2015/058521
<141> 2015-10-31

<150> 62/073,657
<151> 2014-10-31

<160> 38

<170> PatentIn version 3.5

<210> 1
<211> 478
<212> PRT
<213> Homo sapiens

<400> 1
Asn Pro Asn Ala Thr Ser Ser Ser Ser Gln Asp Pro Glu Ser Leu Gln
1               5                   10                  15

Asp Arg Gly Glu Gly Lys Val Ala Thr Thr Val Ile Ser Lys Met Leu
            20                  25                  30

Phe Val Glu Pro Ile Leu Glu Val Ser Ser Leu Pro Thr Thr Asn Ser
        35                  40                  45

Thr Thr Asn Ser Ala Thr Lys Ile Thr Ala Asn Thr Thr Asp Glu Pro
    50                  55                  60

Thr Thr Gln Pro Thr Thr Glu Pro Thr Thr Gln Pro Thr Ile Gln Pro
65                  70                  75                  80

Thr Gln Pro Thr Thr Gln Leu Pro Thr Asp Ser Pro Thr Gln Pro Thr
                85                  90                  95

Thr Gly Ser Phe Cys Pro Gly Pro Val Thr Leu Cys Ser Asp Leu Glu
            100                 105                 110

Ser His Ser Thr Glu Ala Val Leu Gly Asp Ala Leu Val Asp Phe Ser
        115                 120                 125

Leu Lys Leu Tyr His Ala Phe Ser Ala Met Lys Lys Val Glu Thr Asn
    130                 135                 140

Met Ala Phe Ser Pro Phe Ser Ile Ala Ser Leu Leu Thr Gln Val Leu
145                 150                 155                 160

Leu Gly Ala Gly Glu Asn Thr Lys Thr Asn Leu Glu Ser Ile Leu Ser
                165                 170                 175

Tyr Pro Lys Asp Phe Thr Cys Val His Gln Ala Leu Lys Gly Phe Thr
            180                 185                 190

Thr Lys Gly Val Thr Ser Val Ser Gln Ile Phe His Ser Pro Asp Leu
        195                 200                 205

Ala Ile Arg Asp Thr Phe Val Asn Ala Ser Arg Thr Leu Tyr Ser Ser
    210                 215                 220

Ser Pro Arg Val Leu Ser Asn Asn Ser Asp Ala Asn Leu Glu Leu Ile
225                 230                 235                 240

Asn Thr Trp Val Ala Lys Asn Thr Asn Asn Lys Ile Ser Arg Leu Leu
                245                 250                 255

Asp Ser Leu Pro Ser Asp Thr Arg Leu Val Leu Leu Asn Ala Ile Tyr
            260                 265                 270

Leu Ser Ala Lys Trp Lys Thr Thr Phe Asp Pro Lys Lys Thr Arg Met
        275                 280                 285

Glu Pro Phe His Phe Lys Asn Ser Val Ile Lys Val Pro Met Met Asn
    290                 295                 300

Ser Lys Lys Tyr Pro Val Ala His Phe Ile Asp Gln Thr Leu Lys Ala
305                 310                 315                 320

Lys Val Gly Gln Leu Gln Leu Ser His Asn Leu Ser Leu Val Ile Leu
                325                 330                 335

Val Pro Gln Asn Leu Lys His Arg Leu Glu Asp Met Glu Gln Ala Leu
            340                 345                 350

Ser Pro Ser Val Phe Lys Ala Ile Met Glu Lys Leu Glu Met Ser Lys
        355                 360                 365

Phe Gln Pro Thr Leu Leu Thr Leu Pro Arg Ile Lys Val Thr Thr Ser
    370                 375                 380

Gln Asp Met Leu Ser Ile Met Glu Lys Leu Glu Phe Phe Asp Phe Ser
385                 390                 395                 400

Tyr Asp Leu Asn Leu Cys Gly Leu Thr Glu Asp Pro Asp Leu Gln Val
                405                 410                 415

Ser Ala Met Gln His Gln Thr Val Leu Glu Leu Thr Glu Thr Gly Val
            420                 425                 430

Glu Ala Ala Ala Ala Ser Ala Ile Ser Val Ala Arg Thr Leu Leu Val
        435                 440                 445

Phe Glu Val Gln Gln Pro Phe Leu Phe Val Leu Trp Asp Gln Gln His
    450                 455                 460

Lys Phe Pro Val Phe Met Gly Arg Val Tyr Asp Pro Arg Ala
465                 470                 475

<210> 2
<211> 381
<212> PRT
<213> Homo sapiens

<400> 2
Gly Ser Phe Cys Pro Gly Pro Val Thr Leu Cys Ser Asp Leu Glu Ser
1               5                   10                  15

His Ser Thr Glu Ala Val Leu Gly Asp Ala Leu Val Asp Phe Ser Leu
            20                  25                  30

Lys Leu Tyr His Ala Phe Ser Ala Met Lys Lys Val Glu Thr Asn Met
        35                  40                  45

Ala Phe Ser Pro Phe Ser Ile Ala Ser Leu Leu Thr Gln Val Leu Leu
    50                  55                  60

Gly Ala Gly Glu Asn Thr Lys Thr Asn Leu Glu Ser Ile Leu Ser Tyr
65                  70                  75                  80

Pro Lys Asp Phe Thr Cys Val His Gln Ala Leu Lys Gly Phe Thr Thr
                85                  90                  95

Lys Gly Val Thr Ser Val Ser Gln Ile Phe His Ser Pro Asp Leu Ala
            100                 105                 110

Ile Arg Asp Thr Phe Val Asn Ala Ser Arg Thr Leu Tyr Ser Ser Ser
        115                 120                 125

Pro Arg Val Leu Ser Asn Asn Ser Asp Ala Asn Leu Glu Leu Ile Asn
    130                 135                 140

Thr Trp Val Ala Lys Asn Thr Asn Asn Lys Ile Ser Arg Leu Leu Asp
145                 150                 155                 160

Ser Leu Pro Ser Asp Thr Arg Leu Val Leu Leu Asn Ala Ile Tyr Leu
                165                 170                 175

Ser Ala Lys Trp Lys Thr Thr Phe Asp Pro Lys Lys Thr Arg Met Glu
            180                 185                 190

Pro Phe His Phe Lys Asn Ser Val Ile Lys Val Pro Met Met Asn Ser
        195                 200                 205

Lys Lys Tyr Pro Val Ala His Phe Ile Asp Gln Thr Leu Lys Ala Lys
    210                 215                 220

Val Gly Gln Leu Gln Leu Ser His Asn Leu Ser Leu Val Ile Leu Val
225                 230                 235                 240

Pro Gln Asn Leu Lys His Arg Leu Glu Asp Met Glu Gln Ala Leu Ser
                245                 250                 255

Pro Ser Val Phe Lys Ala Ile Met Glu Lys Leu Glu Met Ser Lys Phe
            260                 265                 270

Gln Pro Thr Leu Leu Thr Leu Pro Arg Ile Lys Val Thr Thr Ser Gln
        275                 280                 285

Asp Met Leu Ser Ile Met Glu Lys Leu Glu Phe Phe Asp Phe Ser Tyr
    290                 295                 300

Asp Leu Asn Leu Cys Gly Leu Thr Glu Asp Pro Asp Leu Gln Val Ser
305                 310                 315                 320

Ala Met Gln His Gln Thr Val Leu Glu Leu Thr Glu Thr Gly Val Glu
                325                 330                 335

Ala Ala Ala Ala Ser Ala Ile Ser Val Ala Arg Thr Leu Leu Val Phe
            340                 345                 350

Glu Val Gln Gln Pro Phe Leu Phe Val Leu Trp Asp Gln Gln His Lys
        355                 360                 365

Phe Pro Val Phe Met Gly Arg Val Tyr Asp Pro Arg Ala
    370                 375                 380

<210> 3
<211> 227
<212> PRT
<213> Homo sapiens

<400> 3

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210> 4
<211> 227
<212> PRT

<213> Homo sapiens

<400> 4
Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210> 5
<211> 227
<212> PRT
<213> Homo sapiens

<400> 5
Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu Lys Phe His Tyr Thr Gln Lys Ser Leu Ser Leu Ser
    210                 215                 220

Pro Gly Lys

225

<210> 6
<211> 227
<212> PRT
<213> Homo sapiens

<400> 6
Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu Lys Phe His Tyr Thr Gln Lys Ser Leu Ser Leu Ser
    210                 215                 220

Pro Gly Lys
225

<210> 7
<211> 247
<212> PRT
<213> Artificial Sequence

<220>
<223> Description of Artificial Sequence: Synthetic
      polypeptide

<400> 7
Met Glu Thr Pro Ala Gln Leu Leu Phe Leu Leu Leu Leu Trp Leu Pro
1               5                   10                  15

Asp Thr Thr Gly Asp Lys Thr His Thr Cys Pro Pro Cys Pro Ala Pro
            20                  25                  30

Glu Leu Leu Gly Gly Pro Ser Val Phe Leu Phe Pro Pro Lys Pro Lys
        35                  40                  45

Asp Thr Leu Met Ile Ser Arg Thr Pro Glu Val Thr Cys Val Val Val
    50                  55                  60

Asp Val Ser His Glu Asp Pro Glu Val Lys Phe Asn Trp Tyr Val Asp
65                  70                  75                  80

Gly Val Glu Val His Asn Ala Lys Thr Lys Pro Arg Glu Glu Gln Tyr
                85                  90                  95

Asn Ser Thr Tyr Arg Val Val Ser Val Leu Thr Val Leu His Gln Asp
            100                 105                 110

Trp Leu Asn Gly Lys Glu Tyr Lys Cys Lys Val Ser Asn Lys Ala Leu
        115                 120                 125

Pro Ala Pro Ile Glu Lys Thr Ile Ser Lys Ala Lys Gly Gln Pro Arg
    130                 135                 140

Glu Pro Gln Val Tyr Thr Leu Pro Pro Ser Arg Asp Glu Leu Thr Lys
145                 150                 155                 160

Asn Gln Val Ser Leu Thr Cys Leu Val Lys Gly Phe Tyr Pro Ser Asp
                165                 170                 175

Ile Ala Val Glu Trp Glu Ser Asn Gly Gln Pro Glu Asn Asn Tyr Lys
            180                 185                 190

Thr Thr Pro Pro Val Leu Asp Ser Asp Gly Ser Phe Phe Leu Tyr Ser
        195                 200                 205

Lys Leu Thr Val Asp Lys Ser Arg Trp Gln Gln Gly Asn Val Phe Ser
    210                 215                 220

Cys Ser Val Met His Glu Ala Leu Lys Phe His Tyr Thr Gln Lys Ser
225                 230                 235                 240

Leu Ser Leu Ser Pro Gly Lys
                245

<210> 8
<211> 247
<212> PRT
<213> Artificial Sequence

<220>
<223> Description of Artificial Sequence: Synthetic
      polypeptide

<400> 8
Met Glu Thr Pro Ala Gln Leu Leu Phe Leu Leu Leu Leu Trp Leu Pro
1               5                   10                  15

Asp Thr Thr Gly Asp Lys Thr His Thr Cys Pro Pro Cys Pro Ala Pro
            20                  25                  30

Glu Ala Ala Gly Gly Pro Ser Val Phe Leu Phe Pro Pro Lys Pro Lys
        35                  40                  45

Asp Thr Leu Met Ile Ser Arg Thr Pro Glu Val Thr Cys Val Val Val
    50                  55                  60

Asp Val Ser His Glu Asp Pro Glu Val Lys Phe Asn Trp Tyr Val Asp
65                  70                  75                  80

Gly Val Glu Val His Asn Ala Lys Thr Lys Pro Arg Glu Glu Gln Tyr
                85                  90                  95

Asn Ser Thr Tyr Arg Val Val Ser Val Leu Thr Val Leu His Gln Asp
            100                 105                 110

Trp Leu Asn Gly Lys Glu Tyr Lys Cys Lys Val Ser Asn Lys Ala Leu
        115                 120                 125

Pro Ala Pro Ile Glu Lys Thr Ile Ser Lys Ala Lys Gly Gln Pro Arg
    130                 135                 140

Glu Pro Gln Val Tyr Thr Leu Pro Pro Ser Arg Asp Glu Leu Thr Lys
145                 150                 155                 160

Asn Gln Val Ser Leu Thr Cys Leu Val Lys Gly Phe Tyr Pro Ser Asp
                165                 170                 175

Ile Ala Val Glu Trp Glu Ser Asn Gly Gln Pro Glu Asn Asn Tyr Lys
            180                 185                 190

Thr Thr Pro Pro Val Leu Asp Ser Asp Gly Ser Phe Phe Leu Tyr Ser
        195                 200                 205

Lys Leu Thr Val Asp Lys Ser Arg Trp Gln Gln Gly Asn Val Phe Ser
    210                 215                 220

Cys Ser Val Met His Glu Ala Leu Lys Phe His Tyr Thr Gln Lys Ser
225                 230                 235                 240

Leu Ser Leu Ser Pro Gly Lys
                245

<210> 9
<211> 229
<212> PRT
<213> Homo sapiens

<400> 9
Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro

115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210> 10
<211> 229
<212> PRT
<213> Artificial Sequence

<220>
<223> Description of Artificial Sequence: Synthetic
      polypeptide

<400> 10
Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210> 11
<211> 705
<212> PRT
<213> Artificial Sequence

<220>
<223> Description of Artificial Sequence: Synthetic
      polypeptide

<400> 11
Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys Asn Pro Asn Ala Thr Ser Ser Ser Ser Gln Asp Pro Glu
225                 230                 235                 240

Ser Leu Gln Asp Arg Gly Glu Gly Lys Val Ala Thr Thr Val Ile Ser
                245                 250                 255

Lys Met Leu Phe Val Glu Pro Ile Leu Glu Val Ser Ser Leu Pro Thr
            260                 265                 270

Thr Asn Ser Thr Thr Asn Ser Ala Thr Lys Ile Thr Ala Asn Thr Thr
        275                 280                 285

Asp Glu Pro Thr Thr Gln Pro Thr Thr Glu Pro Thr Thr Gln Pro Thr
    290                 295                 300

Ile Gln Pro Thr Gln Pro Thr Thr Gln Leu Pro Thr Asp Ser Pro Thr
305                 310                 315                 320

Gln Pro Thr Thr Gly Ser Phe Cys Pro Gly Pro Val Thr Leu Cys Ser
                    325                 330                 335

Asp Leu Glu Ser His Ser Thr Glu Ala Val Leu Gly Asp Ala Leu Val
                340                 345                 350

Asp Phe Ser Leu Lys Leu Tyr His Ala Phe Ser Ala Met Lys Lys Val
            355                 360                 365

Glu Thr Asn Met Ala Phe Ser Pro Phe Ser Ile Ala Ser Leu Leu Thr
    370                 375                 380

Gln Val Leu Leu Gly Ala Gly Glu Asn Thr Lys Thr Asn Leu Glu Ser
385                 390                 395                 400

Ile Leu Ser Tyr Pro Lys Asp Phe Thr Cys Val His Gln Ala Leu Lys
                    405                 410                 415

Gly Phe Thr Thr Lys Gly Val Thr Ser Val Ser Gln Ile Phe His Ser
                420                 425                 430

Pro Asp Leu Ala Ile Arg Asp Thr Phe Val Asn Ala Ser Arg Thr Leu
            435                 440                 445

Tyr Ser Ser Ser Pro Arg Val Leu Ser Asn Asn Ser Asp Ala Asn Leu
    450                 455                 460

Glu Leu Ile Asn Thr Trp Val Ala Lys Asn Thr Asn Asn Lys Ile Ser
465                 470                 475                 480

Arg Leu Leu Asp Ser Leu Pro Ser Asp Thr Arg Leu Val Leu Leu Asn
                    485                 490                 495

Ala Ile Tyr Leu Ser Ala Lys Trp Lys Thr Thr Phe Asp Pro Lys Lys
                500                 505                 510

Thr Arg Met Glu Pro Phe His Phe Lys Asn Ser Val Ile Lys Val Pro
            515                 520                 525

Met Met Asn Ser Lys Lys Tyr Pro Val Ala His Phe Ile Asp Gln Thr
    530                 535                 540

Leu Lys Ala Lys Val Gly Gln Leu Gln Leu Ser His Asn Leu Ser Leu
545                 550                 555                 560

Val Ile Leu Val Pro Gln Asn Leu Lys His Arg Leu Glu Asp Met Glu

565                 570                 575

Gln Ala Leu Ser Pro Ser Val Phe Lys Ala Ile Met Glu Lys Leu Glu
            580                 585                 590

Met Ser Lys Phe Gln Pro Thr Leu Leu Thr Leu Pro Arg Ile Lys Val
        595                 600                 605

Thr Thr Ser Gln Asp Met Leu Ser Ile Met Glu Lys Leu Glu Phe Phe
    610                 615                 620

Asp Phe Ser Tyr Asp Leu Asn Leu Cys Gly Leu Thr Glu Asp Pro Asp
625                 630                 635                 640

Leu Gln Val Ser Ala Met Gln His Gln Thr Val Leu Glu Leu Thr Glu
                645                 650                 655

Thr Gly Val Glu Ala Ala Ala Ala Ser Ala Ile Ser Val Ala Arg Thr
            660                 665                 670

Leu Leu Val Phe Glu Val Gln Gln Pro Phe Leu Phe Val Leu Trp Asp
        675                 680                 685

Gln Gln His Lys Phe Pro Val Phe Met Gly Arg Val Tyr Asp Pro Arg
    690                 695                 700

Ala
705

<210> 12
<211> 608
<212> PRT
<213> Artificial Sequence

<220>
<223> Description of Artificial Sequence: Synthetic
      polypeptide

<400> 12
Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys Gly Ser Phe Cys Pro Gly Pro Val Thr Leu Cys Ser Asp
225                 230                 235                 240

Leu Glu Ser His Ser Thr Glu Ala Val Leu Gly Asp Ala Leu Val Asp
                245                 250                 255

Phe Ser Leu Lys Leu Tyr His Ala Phe Ser Ala Met Lys Lys Val Glu
            260                 265                 270

Thr Asn Met Ala Phe Ser Pro Phe Ser Ile Ala Ser Leu Leu Thr Gln
        275                 280                 285

Val Leu Leu Gly Ala Gly Glu Asn Thr Lys Thr Asn Leu Glu Ser Ile
    290                 295                 300

Leu Ser Tyr Pro Lys Asp Phe Thr Cys Val His Gln Ala Leu Lys Gly

305                 310                 315                 320

Phe Thr Thr Lys Gly Val Thr Ser Val Ser Gln Ile Phe His Ser Pro
                325                 330                 335

Asp Leu Ala Ile Arg Asp Thr Phe Val Asn Ala Ser Arg Thr Leu Tyr
            340                 345                 350

Ser Ser Ser Pro Arg Val Leu Ser Asn Asn Ser Asp Ala Asn Leu Glu
        355                 360                 365

Leu Ile Asn Thr Trp Val Ala Lys Asn Thr Asn Asn Lys Ile Ser Arg
    370                 375                 380

Leu Leu Asp Ser Leu Pro Ser Asp Thr Arg Leu Val Leu Leu Asn Ala
385                 390                 395                 400

Ile Tyr Leu Ser Ala Lys Trp Lys Thr Thr Phe Asp Pro Lys Lys Thr
                405                 410                 415

Arg Met Glu Pro Phe His Phe Lys Asn Ser Val Ile Lys Val Pro Met
            420                 425                 430

Met Asn Ser Lys Lys Tyr Pro Val Ala His Phe Ile Asp Gln Thr Leu
        435                 440                 445

Lys Ala Lys Val Gly Gln Leu Gln Leu Ser His Asn Leu Ser Leu Val
    450                 455                 460

Ile Leu Val Pro Gln Asn Leu Lys His Arg Leu Glu Asp Met Glu Gln
465                 470                 475                 480

Ala Leu Ser Pro Ser Val Phe Lys Ala Ile Met Glu Lys Leu Glu Met
                485                 490                 495

Ser Lys Phe Gln Pro Thr Leu Leu Thr Leu Pro Arg Ile Lys Val Thr
            500                 505                 510

Thr Ser Gln Asp Met Leu Ser Ile Met Glu Lys Leu Glu Phe Phe Asp
        515                 520                 525

Phe Ser Tyr Asp Leu Asn Leu Cys Gly Leu Thr Glu Asp Pro Asp Leu
    530                 535                 540

Gln Val Ser Ala Met Gln His Gln Thr Val Leu Glu Leu Thr Glu Thr
545                 550                 555                 560

Gly Val Glu Ala Ala Ala Ala Ser Ala Ile Ser Val Ala Arg Thr Leu
                565                 570                 575

Leu Val Phe Glu Val Gln Gln Pro Phe Leu Phe Val Leu Trp Asp Gln
            580                 585                 590

Gln His Lys Phe Pro Val Phe Met Gly Arg Val Tyr Asp Pro Arg Ala
        595                 600                 605

<210> 13
<211> 705
<212> PRT
<213> Artificial Sequence

<220>
<223> Description of Artificial Sequence: Synthetic
      polypeptide

<400> 13
Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro

                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys Asn Pro Asn Ala Thr Ser Ser Ser Ser Gln Asp Pro Glu
225                 230                 235                 240

Ser Leu Gln Asp Arg Gly Glu Gly Lys Val Ala Thr Thr Val Ile Ser
                245                 250                 255

Lys Met Leu Phe Val Glu Pro Ile Leu Glu Val Ser Ser Leu Pro Thr
            260                 265                 270

Thr Asn Ser Thr Thr Asn Ser Ala Thr Lys Ile Thr Ala Asn Thr Thr
        275                 280                 285

Asp Glu Pro Thr Thr Gln Pro Thr Thr Glu Pro Thr Thr Gln Pro Thr
    290                 295                 300

Ile Gln Pro Thr Gln Pro Thr Thr Gln Leu Pro Thr Asp Ser Pro Thr
305                 310                 315                 320

Gln Pro Thr Thr Gly Ser Phe Cys Pro Gly Pro Val Thr Leu Cys Ser
                325                 330                 335

Asp Leu Glu Ser His Ser Thr Glu Ala Val Leu Gly Asp Ala Leu Val
            340                 345                 350

Asp Phe Ser Leu Lys Leu Tyr His Ala Phe Ser Ala Met Lys Lys Val
        355                 360                 365

Glu Thr Asn Met Ala Phe Ser Pro Phe Ser Ile Ala Ser Leu Leu Thr
    370                 375                 380

Gln Val Leu Leu Gly Ala Gly Glu Asn Thr Lys Thr Asn Leu Glu Ser
385                 390                 395                 400

Ile Leu Ser Tyr Pro Lys Asp Phe Thr Cys Val His Gln Ala Leu Lys
                405                 410                 415

Gly Phe Thr Thr Lys Gly Val Thr Ser Val Ser Gln Ile Phe His Ser
            420                 425                 430

Pro Asp Leu Ala Ile Arg Asp Thr Phe Val Asn Ala Ser Arg Thr Leu
        435                 440                 445

Tyr Ser Ser Ser Pro Arg Val Leu Ser Asn Asn Ser Asp Ala Asn Leu
    450                 455                 460

Glu Leu Ile Asn Thr Trp Val Ala Lys Asn Thr Asn Asn Lys Ile Ser
465                 470                 475                 480

Arg Leu Leu Asp Ser Leu Pro Ser Asp Thr Arg Leu Val Leu Leu Asn
                485                 490                 495

Ala Ile Tyr Leu Ser Ala Lys Trp Lys Thr Thr Phe Asp Pro Lys Lys
            500                 505                 510

Thr Arg Met Glu Pro Phe His Phe Lys Asn Ser Val Ile Lys Val Pro
        515                 520                 525

Met Met Asn Ser Lys Lys Tyr Pro Val Ala His Phe Ile Asp Gln Thr
    530                 535                 540

Leu Lys Ala Lys Val Gly Gln Leu Gln Leu Ser His Asn Leu Ser Leu
545                 550                 555                 560

Val Ile Leu Val Pro Gln Asn Leu Lys His Arg Leu Glu Asp Met Glu
                565                 570                 575

Gln Ala Leu Ser Pro Ser Val Phe Lys Ala Ile Met Glu Lys Leu Glu
            580                 585                 590

Met Ser Lys Phe Gln Pro Thr Leu Leu Thr Leu Pro Arg Ile Lys Val
        595                 600                 605

Thr Thr Ser Gln Asp Met Leu Ser Ile Met Glu Lys Leu Glu Phe Phe
    610                 615                 620

Asp Phe Ser Tyr Asp Leu Asn Leu Cys Gly Leu Thr Glu Asp Pro Asp
625                 630                 635                 640

Leu Gln Val Ser Ala Met Gln His Gln Thr Val Leu Glu Leu Thr Glu
                645                 650                 655

Thr Gly Val Glu Ala Ala Ala Ala Ser Ala Ile Ser Val Ala Arg Thr
            660                 665                 670

Leu Leu Val Phe Glu Val Gln Gln Pro Phe Leu Phe Val Leu Trp Asp
        675                 680                 685

Gln Gln His Lys Phe Pro Val Phe Met Gly Arg Val Tyr Asp Pro Arg
    690                 695                 700

Ala
705

<210> 14
<211> 608
<212> PRT
<213> Artificial Sequence

<220>
<223> Description of Artificial Sequence: Synthetic
      polypeptide

<400> 14
Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys Gly Ser Phe Cys Pro Gly Pro Val Thr Leu Cys Ser Asp
225                 230                 235                 240

Leu Glu Ser His Ser Thr Glu Ala Val Leu Gly Asp Ala Leu Val Asp
                245                 250                 255

Phe Ser Leu Lys Leu Tyr His Ala Phe Ser Ala Met Lys Lys Val Glu
            260                 265                 270

Thr Asn Met Ala Phe Ser Pro Phe Ser Ile Ala Ser Leu Leu Thr Gln
        275                 280                 285

Val Leu Leu Gly Ala Gly Glu Asn Thr Lys Thr Asn Leu Glu Ser Ile
    290                 295                 300

Leu Ser Tyr Pro Lys Asp Phe Thr Cys Val His Gln Ala Leu Lys Gly
305                 310                 315                 320

Phe Thr Thr Lys Gly Val Thr Ser Val Ser Gln Ile Phe His Ser Pro
                325                 330                 335

Asp Leu Ala Ile Arg Asp Thr Phe Val Asn Ala Ser Arg Thr Leu Tyr
            340                 345                 350

Ser Ser Ser Pro Arg Val Leu Ser Asn Asn Ser Asp Ala Asn Leu Glu
        355                 360                 365

Leu Ile Asn Thr Trp Val Ala Lys Asn Thr Asn Asn Lys Ile Ser Arg
    370                 375                 380

Leu Leu Asp Ser Leu Pro Ser Asp Thr Arg Leu Val Leu Leu Asn Ala
385                 390                 395                 400

Ile Tyr Leu Ser Ala Lys Trp Lys Thr Thr Phe Asp Pro Lys Lys Thr
                405                 410                 415

Arg Met Glu Pro Phe His Phe Lys Asn Ser Val Ile Lys Val Pro Met
            420                 425                 430

Met Asn Ser Lys Lys Tyr Pro Val Ala His Phe Ile Asp Gln Thr Leu
        435                 440                 445

Lys Ala Lys Val Gly Gln Leu Gln Leu Ser His Asn Leu Ser Leu Val
    450                 455                 460

Ile Leu Val Pro Gln Asn Leu Lys His Arg Leu Glu Asp Met Glu Gln
465                 470                 475                 480

Ala Leu Ser Pro Ser Val Phe Lys Ala Ile Met Glu Lys Leu Glu Met
                485                 490                 495

Ser Lys Phe Gln Pro Thr Leu Leu Thr Leu Pro Arg Ile Lys Val Thr
            500                 505                 510

Thr Ser Gln Asp Met Leu Ser Ile Met Glu Lys Leu Glu Phe Phe Asp
        515                 520                 525

Phe Ser Tyr Asp Leu Asn Leu Cys Gly Leu Thr Glu Asp Pro Asp Leu
    530                 535                 540

Gln Val Ser Ala Met Gln His Gln Thr Val Leu Glu Leu Thr Glu Thr
545                 550                 555                 560

Gly Val Glu Ala Ala Ala Ala Ser Ala Ile Ser Val Ala Arg Thr Leu
                565                 570                 575

Leu Val Phe Glu Val Gln Gln Pro Phe Leu Phe Val Leu Trp Asp Gln
            580                 585                 590

Gln His Lys Phe Pro Val Phe Met Gly Arg Val Tyr Asp Pro Arg Ala
        595                 600                 605

<210> 15
<211> 707
<212> PRT
<213> Artificial Sequence

<220>
<223> Description of Artificial Sequence: Synthetic
      polypeptide

<400> 15
Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Asn Pro Asn Ala Thr Ser Ser Ser Ser Gln Asp
225                 230                 235                 240

Pro Glu Ser Leu Gln Asp Arg Gly Glu Gly Lys Val Ala Thr Thr Val
                245                 250                 255

Ile Ser Lys Met Leu Phe Val Glu Pro Ile Leu Glu Val Ser Ser Leu
            260                 265                 270

Pro Thr Thr Asn Ser Thr Thr Asn Ser Ala Thr Lys Ile Thr Ala Asn
        275                 280                 285

Thr Thr Asp Glu Pro Thr Thr Gln Pro Thr Thr Glu Pro Thr Thr Gln
    290                 295                 300

Pro Thr Ile Gln Pro Thr Gln Pro Thr Thr Gln Leu Pro Thr Asp Ser
305                 310                 315                 320

Pro Thr Gln Pro Thr Thr Gly Ser Phe Cys Pro Gly Pro Val Thr Leu
                325                 330                 335

Cys Ser Asp Leu Glu Ser His Ser Thr Glu Ala Val Leu Gly Asp Ala
            340                 345                 350

Leu Val Asp Phe Ser Leu Lys Leu Tyr His Ala Phe Ser Ala Met Lys
        355                 360                 365

Lys Val Glu Thr Asn Met Ala Phe Ser Pro Phe Ser Ile Ala Ser Leu
    370                 375                 380

Leu Thr Gln Val Leu Leu Gly Ala Gly Glu Asn Thr Lys Thr Asn Leu
385                 390                 395                 400

Glu Ser Ile Leu Ser Tyr Pro Lys Asp Phe Thr Cys Val His Gln Ala
                405                 410                 415

Leu Lys Gly Phe Thr Thr Lys Gly Val Thr Ser Val Ser Gln Ile Phe
            420                 425                 430

His Ser Pro Asp Leu Ala Ile Arg Asp Thr Phe Val Asn Ala Ser Arg
        435                 440                 445

Thr Leu Tyr Ser Ser Ser Pro Arg Val Leu Ser Asn Asn Ser Asp Ala
    450                 455                 460

Asn Leu Glu Leu Ile Asn Thr Trp Val Ala Lys Asn Thr Asn Asn Lys
465                 470                 475                 480

Ile Ser Arg Leu Leu Asp Ser Leu Pro Ser Asp Thr Arg Leu Val Leu
                485                 490                 495

Leu Asn Ala Ile Tyr Leu Ser Ala Lys Trp Lys Thr Thr Phe Asp Pro
            500                 505                 510

Lys Lys Thr Arg Met Glu Pro Phe His Phe Lys Asn Ser Val Ile Lys
        515                 520                 525

Val Pro Met Met Asn Ser Lys Lys Tyr Pro Val Ala His Phe Ile Asp
    530                 535                 540

Gln Thr Leu Lys Ala Lys Val Gly Gln Leu Gln Leu Ser His Asn Leu
545                 550                 555                 560

Ser Leu Val Ile Leu Val Pro Gln Asn Leu Lys His Arg Leu Glu Asp
                565                 570                 575

Met Glu Gln Ala Leu Ser Pro Ser Val Phe Lys Ala Ile Met Glu Lys
            580                 585                 590

Leu Glu Met Ser Lys Phe Gln Pro Thr Leu Leu Thr Leu Pro Arg Ile
        595                 600                 605

Lys Val Thr Thr Ser Gln Asp Met Leu Ser Ile Met Glu Lys Leu Glu
    610                 615                 620

Phe Phe Asp Phe Ser Tyr Asp Leu Asn Leu Cys Gly Leu Thr Glu Asp
625                 630                 635                 640

Pro Asp Leu Gln Val Ser Ala Met Gln His Gln Thr Val Leu Glu Leu
                645                 650                 655

Thr Glu Thr Gly Val Glu Ala Ala Ala Ala Ser Ala Ile Ser Val Ala
            660                 665                 670

Arg Thr Leu Leu Val Phe Glu Val Gln Gln Pro Phe Leu Phe Val Leu
        675                 680                 685

Trp Asp Gln Gln His Lys Phe Pro Val Phe Met Gly Arg Val Tyr Asp
    690                 695                 700

Pro Arg Ala
705


<210> 16
<211> 610
<212> PRT
<213> Artificial Sequence

<220>
<223> Description of Artificial Sequence: Synthetic
      polypeptide

<400> 16
Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Gly Ser Phe Cys Pro Gly Pro Val Thr Leu Cys
225                 230                 235                 240

Ser Asp Leu Glu Ser His Ser Thr Glu Ala Val Leu Gly Asp Ala Leu
                245                 250                 255

Val Asp Phe Ser Leu Lys Leu Tyr His Ala Phe Ser Ala Met Lys Lys
            260                 265                 270

Val Glu Thr Asn Met Ala Phe Ser Pro Phe Ser Ile Ala Ser Leu Leu
        275                 280                 285

Thr Gln Val Leu Leu Gly Ala Gly Glu Asn Thr Lys Thr Asn Leu Glu
    290                 295                 300

Ser Ile Leu Ser Tyr Pro Lys Asp Phe Thr Cys Val His Gln Ala Leu
305                 310                 315                 320

Lys Gly Phe Thr Thr Lys Gly Val Thr Ser Val Ser Gln Ile Phe His
                325                 330                 335

Ser Pro Asp Leu Ala Ile Arg Asp Thr Phe Val Asn Ala Ser Arg Thr
            340                 345                 350

Leu Tyr Ser Ser Ser Pro Arg Val Leu Ser Asn Asn Ser Asp Ala Asn
        355                 360                 365

Leu Glu Leu Ile Asn Thr Trp Val Ala Lys Asn Thr Asn Asn Lys Ile
    370                 375                 380

Ser Arg Leu Leu Asp Ser Leu Pro Ser Asp Thr Arg Leu Val Leu Leu
385                 390                 395                 400

Asn Ala Ile Tyr Leu Ser Ala Lys Trp Lys Thr Thr Phe Asp Pro Lys
                405                 410                 415

Lys Thr Arg Met Glu Pro Phe His Phe Lys Asn Ser Val Ile Lys Val
            420                 425                 430

Pro Met Met Asn Ser Lys Lys Tyr Pro Val Ala His Phe Ile Asp Gln
        435                 440                 445

Thr Leu Lys Ala Lys Val Gly Gln Leu Gln Leu Ser His Asn Leu Ser
    450                 455                 460

Leu Val Ile Leu Val Pro Gln Asn Leu Lys His Arg Leu Glu Asp Met
465                 470                 475                 480

Glu Gln Ala Leu Ser Pro Ser Val Phe Lys Ala Ile Met Glu Lys Leu
                485                 490                 495

Glu Met Ser Lys Phe Gln Pro Thr Leu Leu Thr Leu Pro Arg Ile Lys
            500                 505                 510

Val Thr Thr Ser Gln Asp Met Leu Ser Ile Met Glu Lys Leu Glu Phe

515                 520                 525

Phe Asp Phe Ser Tyr Asp Leu Asn Leu Cys Gly Leu Thr Glu Asp Pro
    530                 535                 540

Asp Leu Gln Val Ser Ala Met Gln His Gln Thr Val Leu Glu Leu Thr
545                 550                 555                 560

Glu Thr Gly Val Glu Ala Ala Ala Ala Ser Ala Ile Ser Val Ala Arg
                565                 570                 575

Thr Leu Leu Val Phe Glu Val Gln Gln Pro Phe Leu Phe Val Leu Trp
            580                 585                 590

Asp Gln Gln His Lys Phe Pro Val Phe Met Gly Arg Val Tyr Asp Pro
        595                 600                 605

Arg Ala
    610

<210> 17
<211> 609
<212> PRT
<213> Homo sapiens

<400> 17

<210> 18
<211> 1087
<212> PRT
<213> Artificial Sequence

<220>
<223> Description of Artificial Sequence: Synthetic
      polypeptide

<400> 18
Met Lys Trp Val Thr Phe Ile Ser Leu Leu Phe Leu Phe Ser Ser Ala
1               5                   10                  15

Tyr Ser Arg Gly Val Phe Arg Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys

210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Lys Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Arg Ala Ala Leu Gly
        595                 600                 605

Leu Asn Pro Asn Ala Thr Ser Ser Ser Ser Gln Asp Pro Glu Ser Leu
    610                 615                 620

Gln Asp Arg Gly Glu Gly Lys Val Ala Thr Thr Val Ile Ser Lys Met
625                 630                 635                 640

Leu Phe Val Glu Pro Ile Leu Glu Val Ser Ser Leu Pro Thr Thr Asn
                645                 650                 655

Ser Thr Thr Asn Ser Ala Thr Lys Ile Thr Ala Asn Thr Thr Asp Glu
            660                 665                 670

Pro Thr Thr Gln Pro Thr Thr Glu Pro Thr Thr Gln Pro Thr Ile Gln
        675                 680                 685

Pro Thr Gln Pro Thr Thr Gln Leu Pro Thr Asp Ser Pro Thr Gln Pro
    690                 695                 700

Thr Thr Gly Ser Phe Cys Pro Gly Pro Val Thr Leu Cys Ser Asp Leu
705                 710                 715                 720

Glu Ser His Ser Thr Glu Ala Val Leu Gly Asp Ala Leu Val Asp Phe
                725                 730                 735

Ser Leu Lys Leu Tyr His Ala Phe Ser Ala Met Lys Lys Val Glu Thr
            740                 745                 750

Asn Met Ala Phe Ser Pro Phe Ser Ile Ala Ser Leu Leu Thr Gln Val
        755                 760                 765

Leu Leu Gly Ala Gly Glu Asn Thr Lys Thr Asn Leu Glu Ser Ile Leu
    770                 775                 780

Ser Tyr Pro Lys Asp Phe Thr Cys Val His Gln Ala Leu Lys Gly Phe
785                 790                 795                 800

Thr Thr Lys Gly Val Thr Ser Val Ser Gln Ile Phe His Ser Pro Asp
                805                 810                 815

Leu Ala Ile Arg Asp Thr Phe Val Asn Ala Ser Arg Thr Leu Tyr Ser
            820                 825                 830

Ser Ser Pro Arg Val Leu Ser Asn Asn Ser Asp Ala Asn Leu Glu Leu
        835                 840                 845

Ile Asn Thr Trp Val Ala Lys Asn Thr Asn Asn Lys Ile Ser Arg Leu
    850                 855                 860

Leu Asp Ser Leu Pro Ser Asp Thr Arg Leu Val Leu Leu Asn Ala Ile
865                 870                 875                 880

Tyr Leu Ser Ala Lys Trp Lys Thr Thr Phe Asp Pro Lys Lys Thr Arg
                885                 890                 895

Met Glu Pro Phe His Phe Lys Asn Ser Val Ile Lys Val Pro Met Met
            900                 905                 910

Asn Ser Lys Lys Tyr Pro Val Ala His Phe Ile Asp Gln Thr Leu Lys
        915                 920                 925

Ala Lys Val Gly Gln Leu Gln Leu Ser His Asn Leu Ser Leu Val Ile
    930                 935                 940

Leu Val Pro Gln Asn Leu Lys His Arg Leu Glu Asp Met Glu Gln Ala
945                 950                 955                 960

Leu Ser Pro Ser Val Phe Lys Ala Ile Met Glu Lys Leu Glu Met Ser
                965                 970                 975

Lys Phe Gln Pro Thr Leu Leu Thr Leu Pro Arg Ile Lys Val Thr Thr
            980                 985                 990

Ser Gln Asp Met Leu Ser Ile Met  Glu Lys Leu Glu Phe  Phe Asp Phe
        995                 1000                1005

Ser Tyr  Asp Leu Asn Leu Cys  Gly Leu Thr Glu Asp  Pro Asp Leu
    1010                 1015                1020

Gln Val  Ser Ala Met Gln His  Gln Thr Val Leu Glu  Leu Thr Glu
    1025                 1030                1035

Thr Gly  Val Glu Ala Ala Ala  Ala Ser Ala Ile Ser  Val Ala Arg
    1040                 1045                1050

Thr Leu  Leu Val Phe Glu Val  Gln Gln Pro Phe Leu  Phe Val Leu
    1055                 1060                1065

Trp Asp  Gln Gln His Lys Phe  Pro Val Phe Met Gly  Arg Val Tyr
    1070                 1075                1080

Asp Pro  Arg Ala
    1085

<210> 19
<211> 990
<212> PRT
<213> Artificial Sequence

<220>
<223> Description of Artificial Sequence: Synthetic
      polypeptide

<400> 19
Met Lys Trp Val Thr Phe Ile Ser Leu Leu Phe Leu Phe Ser Ser Ala
1               5                   10                  15

Tyr Ser Arg Gly Val Phe Arg Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Lys Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe

            580                 585                 590

Ala Glu Glu Gly Lys Lys Leu Val Ala Ala Ser Arg Ala Ala Leu Gly
        595                 600                 605

Leu Gly Ser Phe Cys Pro Gly Pro Val Thr Leu Cys Ser Asp Leu Glu
    610                 615                 620

Ser His Ser Thr Glu Ala Val Leu Gly Asp Ala Leu Val Asp Phe Ser
625                 630                 635                 640

Leu Lys Leu Tyr His Ala Phe Ser Ala Met Lys Lys Val Glu Thr Asn
                645                 650                 655

Met Ala Phe Ser Pro Phe Ser Ile Ala Ser Leu Leu Thr Gln Val Leu
            660                 665                 670

Leu Gly Ala Gly Glu Asn Thr Lys Thr Asn Leu Glu Ser Ile Leu Ser
        675                 680                 685

Tyr Pro Lys Asp Phe Thr Cys Val His Gln Ala Leu Lys Gly Phe Thr
    690                 695                 700

Thr Lys Gly Val Thr Ser Val Ser Gln Ile Phe His Ser Pro Asp Leu
705                 710                 715                 720

Ala Ile Arg Asp Thr Phe Val Asn Ala Ser Arg Thr Leu Tyr Ser Ser
                725                 730                 735

Ser Pro Arg Val Leu Ser Asn Asn Ser Asp Ala Asn Leu Glu Leu Ile
            740                 745                 750

Asn Thr Trp Val Ala Lys Asn Thr Asn Asn Lys Ile Ser Arg Leu Leu
        755                 760                 765

Asp Ser Leu Pro Ser Asp Thr Arg Leu Val Leu Leu Asn Ala Ile Tyr
    770                 775                 780

Leu Ser Ala Lys Trp Lys Thr Thr Phe Asp Pro Lys Lys Thr Arg Met
785                 790                 795                 800

Glu Pro Phe His Phe Lys Asn Ser Val Ile Lys Val Pro Met Met Asn
                805                 810                 815

Ser Lys Lys Tyr Pro Val Ala His Phe Ile Asp Gln Thr Leu Lys Ala
            820                 825                 830

Lys Val Gly Gln Leu Gln Leu Ser His Asn Leu Ser Leu Val Ile Leu
        835                 840                 845

Val Pro Gln Asn Leu Lys His Arg Leu Glu Asp Met Glu Gln Ala Leu
    850                 855                 860

Ser Pro Ser Val Phe Lys Ala Ile Met Glu Lys Leu Glu Met Ser Lys
865                 870                 875                 880

Phe Gln Pro Thr Leu Leu Thr Leu Pro Arg Ile Lys Val Thr Thr Ser
                885                 890                 895

Gln Asp Met Leu Ser Ile Met Glu Lys Leu Glu Phe Phe Asp Phe Ser
            900                 905                 910

Tyr Asp Leu Asn Leu Cys Gly Leu Thr Glu Asp Pro Asp Leu Gln Val
        915                 920                 925

Ser Ala Met Gln His Gln Thr Val Leu Glu Leu Thr Glu Thr Gly Val
    930                 935                 940

Glu Ala Ala Ala Ala Ser Ala Ile Ser Val Ala Arg Thr Leu Leu Val
945                 950                 955                 960

Phe Glu Val Gln Gln Pro Phe Leu Phe Val Leu Trp Asp Gln Gln His
                965                 970                 975

Lys Phe Pro Val Phe Met Gly Arg Val Tyr Asp Pro Arg Ala
            980                 985                 990

<210> 20
<211> 225
<212> PRT
<213> Homo sapiens

<400> 20
Met Glu Thr Pro Ala Gln Leu Leu Phe Leu Leu Leu Leu Trp Leu Pro
1               5                   10                  15

Asp Thr Thr Gly Val Glu Glu Pro Gln Asn Leu Ile Lys Gln Asn Cys
            20                  25                  30

Glu Leu Phe Glu Gln Leu Gly Glu Tyr Lys Phe Gln Asn Ala Leu Leu
        35                  40                  45

Val Arg Tyr Thr Lys Lys Val Pro Gln Val Ser Thr Pro Thr Leu Val
    50                  55                  60

Glu Val Ser Arg Asn Leu Gly Lys Val Gly Ser Lys Cys Cys Lys His

65                  70                  75                  80

Pro Glu Ala Lys Arg Met Pro Cys Ala Glu Asp Tyr Leu Ser Val Val
                85                  90                  95

Leu Asn Gln Leu Cys Val Leu His Glu Lys Thr Pro Val Ser Asp Arg
            100                 105                 110

Val Thr Lys Cys Cys Thr Glu Ser Leu Val Asn Arg Arg Pro Cys Phe
        115                 120                 125

Ser Ala Leu Glu Val Asp Glu Thr Tyr Val Pro Lys Glu Phe Asn Ala
    130                 135                 140

Glu Thr Phe Thr Phe His Ala Asp Ile Cys Thr Leu Ser Glu Lys Glu
145                 150                 155                 160

Arg Gln Ile Lys Lys Gln Thr Ala Leu Val Glu Leu Val Lys His Lys
                165                 170                 175

Pro Lys Ala Thr Lys Glu Gln Leu Lys Ala Val Met Asp Asp Phe Ala
            180                 185                 190

Ala Phe Val Glu Lys Cys Cys Lys Ala Asp Asp Lys Glu Thr Cys Phe
        195                 200                 205

Ala Glu Glu Gly Lys Lys Leu Val Ala Ala Ser Arg Ala Ala Leu Gly
    210                 215                 220

Leu
225

<210> 21
<211> 703
<212> PRT
<213> Artificial Sequence

<220>
<223> Description of Artificial Sequence: Synthetic
      polypeptide

<400> 21
Met Glu Thr Pro Ala Gln Leu Leu Phe Leu Leu Leu Leu Trp Leu Pro
1               5                   10                  15

Asp Thr Thr Gly Val Glu Glu Pro Gln Asn Leu Ile Lys Gln Asn Cys
            20                  25                  30

Glu Leu Phe Glu Gln Leu Gly Glu Tyr Lys Phe Gln Asn Ala Leu Leu
        35                  40                  45

Val Arg Tyr Thr Lys Lys Val Pro Gln Val Ser Thr Pro Thr Leu Val
    50                  55                  60

Glu Val Ser Arg Asn Leu Gly Lys Val Gly Ser Lys Cys Cys Lys His
65                  70                  75                  80

Pro Glu Ala Lys Arg Met Pro Cys Ala Glu Asp Tyr Leu Ser Val Val
                85                  90                  95

Leu Asn Gln Leu Cys Val Leu His Glu Lys Thr Pro Val Ser Asp Arg
            100                 105                 110

Val Thr Lys Cys Cys Thr Glu Ser Leu Val Asn Arg Arg Pro Cys Phe
        115                 120                 125

Ser Ala Leu Glu Val Asp Glu Thr Tyr Val Pro Lys Glu Phe Asn Ala
    130                 135                 140

Glu Thr Phe Thr Phe His Ala Asp Ile Cys Thr Leu Ser Glu Lys Glu
145                 150                 155                 160

Arg Gln Ile Lys Lys Gln Thr Ala Leu Val Glu Leu Val Lys His Lys
                165                 170                 175

Pro Lys Ala Thr Lys Glu Gln Leu Lys Ala Val Met Asp Asp Phe Ala
            180                 185                 190

Ala Phe Val Glu Lys Cys Cys Lys Ala Asp Asp Lys Glu Thr Cys Phe
        195                 200                 205

Ala Glu Glu Gly Lys Lys Leu Val Ala Ala Ser Arg Ala Ala Leu Gly
    210                 215                 220

Leu Asn Pro Asn Ala Thr Ser Ser Ser Ser Gln Asp Pro Glu Ser Leu
225                 230                 235                 240

Gln Asp Arg Gly Glu Gly Lys Val Ala Thr Thr Val Ile Ser Lys Met
                245                 250                 255

Leu Phe Val Glu Pro Ile Leu Glu Val Ser Ser Leu Pro Thr Thr Asn
            260                 265                 270

Ser Thr Thr Asn Ser Ala Thr Lys Ile Thr Ala Asn Thr Thr Asp Glu
        275                 280                 285

Pro Thr Thr Gln Pro Thr Thr Glu Pro Thr Thr Gln Pro Thr Ile Gln

290                 295                 300

Pro Thr Gln Pro Thr Thr Gln Leu Pro Thr Asp Ser Pro Thr Gln Pro
305                 310                 315                 320

Thr Thr Gly Ser Phe Cys Pro Gly Pro Val Thr Leu Cys Ser Asp Leu
                325                 330                 335

Glu Ser His Ser Thr Glu Ala Val Leu Gly Asp Ala Leu Val Asp Phe
            340                 345                 350

Ser Leu Lys Leu Tyr His Ala Phe Ser Ala Met Lys Lys Val Glu Thr
        355                 360                 365

Asn Met Ala Phe Ser Pro Phe Ser Ile Ala Ser Leu Leu Thr Gln Val
    370                 375                 380

Leu Leu Gly Ala Gly Glu Asn Thr Lys Thr Asn Leu Glu Ser Ile Leu
385                 390                 395                 400

Ser Tyr Pro Lys Asp Phe Thr Cys Val His Gln Ala Leu Lys Gly Phe
                405                 410                 415

Thr Thr Lys Gly Val Thr Ser Val Ser Gln Ile Phe His Ser Pro Asp
            420                 425                 430

Leu Ala Ile Arg Asp Thr Phe Val Asn Ala Ser Arg Thr Leu Tyr Ser
        435                 440                 445

Ser Ser Pro Arg Val Leu Ser Asn Asn Ser Asp Ala Asn Leu Glu Leu
    450                 455                 460

Ile Asn Thr Trp Val Ala Lys Asn Thr Asn Asn Lys Ile Ser Arg Leu
465                 470                 475                 480

Leu Asp Ser Leu Pro Ser Asp Thr Arg Leu Val Leu Leu Asn Ala Ile
                485                 490                 495

Tyr Leu Ser Ala Lys Trp Lys Thr Thr Phe Asp Pro Lys Lys Thr Arg
            500                 505                 510

Met Glu Pro Phe His Phe Lys Asn Ser Val Ile Lys Val Pro Met Met
        515                 520                 525

Asn Ser Lys Lys Tyr Pro Val Ala His Phe Ile Asp Gln Thr Leu Lys
    530                 535                 540

Ala Lys Val Gly Gln Leu Gln Leu Ser His Asn Leu Ser Leu Val Ile
545                 550                 555                 560

Leu Val Pro Gln Asn Leu Lys His Arg Leu Glu Asp Met Glu Gln Ala
                565                 570                 575

Leu Ser Pro Ser Val Phe Lys Ala Ile Met Glu Lys Leu Glu Met Ser
            580                 585                 590

Lys Phe Gln Pro Thr Leu Leu Thr Leu Pro Arg Ile Lys Val Thr Thr
        595                 600                 605

Ser Gln Asp Met Leu Ser Ile Met Glu Lys Leu Glu Phe Phe Asp Phe
    610                 615                 620

Ser Tyr Asp Leu Asn Leu Cys Gly Leu Thr Glu Asp Pro Asp Leu Gln
625                 630                 635                 640

Val Ser Ala Met Gln His Gln Thr Val Leu Glu Leu Thr Glu Thr Gly
                645                 650                 655

Val Glu Ala Ala Ala Ala Ser Ala Ile Ser Val Ala Arg Thr Leu Leu
            660                 665                 670

Val Phe Glu Val Gln Gln Pro Phe Leu Phe Val Leu Trp Asp Gln Gln
        675                 680                 685

His Lys Phe Pro Val Phe Met Gly Arg Val Tyr Asp Pro Arg Ala
    690                 695                 700

<210> 22
<211> 606
<212> PRT
<213> Artificial Sequence

<220>
<223> Description of Artificial Sequence: Synthetic
      polypeptide

<400> 22
Met Glu Thr Pro Ala Gln Leu Leu Phe Leu Leu Leu Leu Trp Leu Pro
1               5                   10                  15

Asp Thr Thr Gly Val Glu Glu Pro Gln Asn Leu Ile Lys Gln Asn Cys
            20                  25                  30

Glu Leu Phe Glu Gln Leu Gly Glu Tyr Lys Phe Gln Asn Ala Leu Leu
        35                  40                  45

Val Arg Tyr Thr Lys Lys Val Pro Gln Val Ser Thr Pro Thr Leu Val

50                  55                  60

Glu Val Ser Arg Asn Leu Gly Lys Val Gly Ser Lys Cys Cys Lys His
65                  70                  75                  80

Pro Glu Ala Lys Arg Met Pro Cys Ala Glu Asp Tyr Leu Ser Val Val
                85                  90                  95

Leu Asn Gln Leu Cys Val Leu His Glu Lys Thr Pro Val Ser Asp Arg
            100                 105                 110

Val Thr Lys Cys Cys Thr Glu Ser Leu Val Asn Arg Arg Pro Cys Phe
        115                 120                 125

Ser Ala Leu Glu Val Asp Glu Thr Tyr Val Pro Lys Glu Phe Asn Ala
    130                 135                 140

Glu Thr Phe Thr Phe His Ala Asp Ile Cys Thr Leu Ser Glu Lys Glu
145                 150                 155                 160

Arg Gln Ile Lys Lys Gln Thr Ala Leu Val Glu Leu Val Lys His Lys
                165                 170                 175

Pro Lys Ala Thr Lys Glu Gln Leu Lys Ala Val Met Asp Asp Phe Ala
            180                 185                 190

Ala Phe Val Glu Lys Cys Cys Lys Ala Asp Asp Lys Glu Thr Cys Phe
        195                 200                 205

Ala Glu Glu Gly Lys Lys Leu Val Ala Ala Ser Arg Ala Ala Leu Gly
    210                 215                 220

Leu Gly Ser Phe Cys Pro Gly Pro Val Thr Leu Cys Ser Asp Leu Glu
225                 230                 235                 240

Ser His Ser Thr Glu Ala Val Leu Gly Asp Ala Leu Val Asp Phe Ser
                245                 250                 255

Leu Lys Leu Tyr His Ala Phe Ser Ala Met Lys Lys Val Glu Thr Asn
            260                 265                 270

Met Ala Phe Ser Pro Phe Ser Ile Ala Ser Leu Leu Thr Gln Val Leu
        275                 280                 285

Leu Gly Ala Gly Glu Asn Thr Lys Thr Asn Leu Glu Ser Ile Leu Ser
    290                 295                 300

Tyr Pro Lys Asp Phe Thr Cys Val His Gln Ala Leu Lys Gly Phe Thr
305                 310                 315                 320

Thr Lys Gly Val Thr Ser Val Ser Gln Ile Phe His Ser Pro Asp Leu
                325                 330                 335

Ala Ile Arg Asp Thr Phe Val Asn Ala Ser Arg Thr Leu Tyr Ser Ser
            340                 345                 350

Ser Pro Arg Val Leu Ser Asn Asn Ser Asp Ala Asn Leu Glu Leu Ile
        355                 360                 365

Asn Thr Trp Val Ala Lys Asn Thr Asn Asn Lys Ile Ser Arg Leu Leu
    370                 375                 380

Asp Ser Leu Pro Ser Asp Thr Arg Leu Val Leu Leu Asn Ala Ile Tyr
385                 390                 395                 400

Leu Ser Ala Lys Trp Lys Thr Thr Phe Asp Pro Lys Lys Thr Arg Met
                405                 410                 415

Glu Pro Phe His Phe Lys Asn Ser Val Ile Lys Val Pro Met Met Asn
            420                 425                 430

Ser Lys Lys Tyr Pro Val Ala His Phe Ile Asp Gln Thr Leu Lys Ala
        435                 440                 445

Lys Val Gly Gln Leu Gln Leu Ser His Asn Leu Ser Leu Val Ile Leu
    450                 455                 460

Val Pro Gln Asn Leu Lys His Arg Leu Glu Asp Met Glu Gln Ala Leu
465                 470                 475                 480

Ser Pro Ser Val Phe Lys Ala Ile Met Glu Lys Leu Glu Met Ser Lys
                485                 490                 495

Phe Gln Pro Thr Leu Leu Thr Leu Pro Arg Ile Lys Val Thr Thr Ser
            500                 505                 510

Gln Asp Met Leu Ser Ile Met Glu Lys Leu Glu Phe Phe Asp Phe Ser
        515                 520                 525

Tyr Asp Leu Asn Leu Cys Gly Leu Thr Glu Asp Pro Asp Leu Gln Val
    530                 535                 540

Ser Ala Met Gln His Gln Thr Val Leu Glu Leu Thr Glu Thr Gly Val
545                 550                 555                 560

Glu Ala Ala Ala Ala Ser Ala Ile Ser Val Ala Arg Thr Leu Leu Val
                565                 570                 575

Phe Glu Val Gln Gln Pro Phe Leu Phe Val Leu Trp Asp Gln Gln His
            580                 585                 590

Lys Phe Pro Val Phe Met Gly Arg Val Tyr Asp Pro Arg Ala
        595                 600                 605

<210> 23
<211> 1090
<212> PRT
<213> Artificial Sequence

<220>
<223> Description of Artificial Sequence: Synthetic
      polypeptide

<400> 23
Met Lys Trp Val Thr Phe Ile Ser Leu Leu Phe Leu Phe Ser Ser Ala
1               5                   10                  15

Tyr Ser Arg Gly Val Phe Arg Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Lys Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
        420                 425                 430

Val Arg Tyr Thr Lys Lys Val Pro Gln Val Ser Thr Pro Thr Leu Val
    435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Arg Ala Ala Leu Gly
        595                 600                 605

Leu Gly Gly Gly Asn Pro Asn Ala Thr Ser Ser Ser Ser Gln Asp Pro
    610                 615                 620

Glu Ser Leu Gln Asp Arg Gly Glu Gly Lys Val Ala Thr Thr Val Ile
625                 630                 635                 640

Ser Lys Met Leu Phe Val Glu Pro Ile Leu Glu Val Ser Ser Leu Pro
                645                 650                 655

Thr Thr Asn Ser Thr Thr Asn Ser Ala Thr Lys Ile Thr Ala Asn Thr
            660                 665                 670

Thr Asp Glu Pro Thr Thr Gln Pro Thr Thr Glu Pro Thr Thr Gln Pro
        675                 680                 685

Thr Ile Gln Pro Thr Gln Pro Thr Thr Gln Leu Pro Thr Asp Ser Pro
    690                 695                 700

Thr Gln Pro Thr Thr Gly Ser Phe Cys Pro Gly Pro Val Thr Leu Cys
705                 710                 715                 720

Ser Asp Leu Glu Ser His Ser Thr Glu Ala Val Leu Gly Asp Ala Leu
                725                 730                 735

Val Asp Phe Ser Leu Lys Leu Tyr His Ala Phe Ser Ala Met Lys Lys
            740                 745                 750

Val Glu Thr Asn Met Ala Phe Ser Pro Phe Ser Ile Ala Ser Leu Leu
        755                 760                 765

Thr Gln Val Leu Leu Gly Ala Gly Glu Asn Thr Lys Thr Asn Leu Glu
    770                 775                 780

Ser Ile Leu Ser Tyr Pro Lys Asp Phe Thr Cys Val His Gln Ala Leu
785                 790                 795                 800

Lys Gly Phe Thr Thr Lys Gly Val Thr Ser Val Ser Gln Ile Phe His
                805                 810                 815

Ser Pro Asp Leu Ala Ile Arg Asp Thr Phe Val Asn Ala Ser Arg Thr
            820                 825                 830

Leu Tyr Ser Ser Ser Pro Arg Val Leu Ser Asn Asn Ser Asp Ala Asn
        835                 840                 845

Leu Glu Leu Ile Asn Thr Trp Val Ala Lys Asn Thr Asn Asn Lys Ile
    850                 855                 860

Ser Arg Leu Leu Asp Ser Leu Pro Ser Asp Thr Arg Leu Val Leu Leu
865                 870                 875                 880

Asn Ala Ile Tyr Leu Ser Ala Lys Trp Lys Thr Thr Phe Asp Pro Lys
                885                 890                 895

Lys Thr Arg Met Glu Pro Phe His Phe Lys Asn Ser Val Ile Lys Val
            900                 905                 910

Pro Met Met Asn Ser Lys Lys Tyr Pro Val Ala His Phe Ile Asp Gln

915                 920                 925

Thr Leu Lys Ala Lys Val Gly Gln Leu Gln Leu Ser His Asn Leu Ser
    930                 935                 940

Leu Val Ile Leu Val Pro Gln Asn Leu Lys His Arg Leu Glu Asp Met
945                 950                 955                 960

Glu Gln Ala Leu Ser Pro Ser Val Phe Lys Ala Ile Met Glu Lys Leu
                965                 970                 975

Glu Met Ser Lys Phe Gln Pro Thr Leu Leu Thr Leu Pro Arg Ile Lys
            980                 985                 990

Val Thr Thr Ser Gln Asp Met Leu  Ser Ile Met Glu Lys  Leu Glu Phe
        995                 1000                1005

Phe Asp  Phe Ser Tyr Asp Leu  Asn Leu Cys Gly Leu  Thr Glu Asp
    1010                1015                1020

Pro Asp  Leu Gln Val Ser Ala  Met Gln His Gln Thr  Val Leu Glu
    1025                1030                1035

Leu Thr  Glu Thr Gly Val Glu  Ala Ala Ala Ala Ser  Ala Ile Ser
    1040                1045                1050

Val Ala  Arg Thr Leu Leu Val  Phe Glu Val Gln Gln  Pro Phe Leu
    1055                1060                1065

Phe Val  Leu Trp Asp Gln Gln  His Lys Phe Pro Val  Phe Met Gly
    1070                1075                1080

Arg Val  Tyr Asp Pro Arg Ala
    1085                1090

<210> 24
<211> 993
<212> PRT
<213> Artificial Sequence

<220>
<223> Description of Artificial Sequence: Synthetic
      polypeptide

<400> 24
Met Lys Trp Val Thr Phe Ile Ser Leu Leu Phe Leu Phe Ser Ser Ala
1               5                   10                  15

Tyr Ser Arg Gly Val Phe Arg Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile

        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Lys Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Arg Ala Ala Leu Gly
        595                 600                 605

Leu Gly Gly Gly Gly Ser Phe Cys Pro Gly Pro Val Thr Leu Cys Ser
    610                 615                 620

Asp Leu Glu Ser His Ser Thr Glu Ala Val Leu Gly Asp Ala Leu Val
625                 630                 635                 640

Asp Phe Ser Leu Lys Leu Tyr His Ala Phe Ser Ala Met Lys Lys Val
                645                 650                 655

Glu Thr Asn Met Ala Phe Ser Pro Phe Ser Ile Ala Ser Leu Leu Thr
            660                 665                 670

Gln Val Leu Leu Gly Ala Gly Glu Asn Thr Lys Thr Asn Leu Glu Ser
        675                 680                 685

Ile Leu Ser Tyr Pro Lys Asp Phe Thr Cys Val His Gln Ala Leu Lys
    690                 695                 700

Gly Phe Thr Thr Lys Gly Val Thr Ser Val Ser Gln Ile Phe His Ser
705                 710                 715                 720

Pro Asp Leu Ala Ile Arg Asp Thr Phe Val Asn Ala Ser Arg Thr Leu
                725                 730                 735

Tyr Ser Ser Ser Pro Arg Val Leu Ser Asn Asn Ser Asp Ala Asn Leu
            740                 745                 750

Glu Leu Ile Asn Thr Trp Val Ala Lys Asn Thr Asn Asn Lys Ile Ser
        755                 760                 765

Arg Leu Leu Asp Ser Leu Pro Ser Asp Thr Arg Leu Val Leu Leu Asn
    770                 775                 780

Ala Ile Tyr Leu Ser Ala Lys Trp Lys Thr Thr Phe Asp Pro Lys Lys
785                 790                 795                 800

Thr Arg Met Glu Pro Phe His Phe Lys Asn Ser Val Ile Lys Val Pro
                805                 810                 815

Met Met Asn Ser Lys Lys Tyr Pro Val Ala His Phe Ile Asp Gln Thr
            820                 825                 830

Leu Lys Ala Lys Val Gly Gln Leu Gln Leu Ser His Asn Leu Ser Leu
        835                 840                 845

Val Ile Leu Val Pro Gln Asn Leu Lys His Arg Leu Glu Asp Met Glu
    850                 855                 860

Gln Ala Leu Ser Pro Ser Val Phe Lys Ala Ile Met Glu Lys Leu Glu
865                 870                 875                 880

Met Ser Lys Phe Gln Pro Thr Leu Leu Thr Leu Pro Arg Ile Lys Val
                885                 890                 895

Thr Thr Ser Gln Asp Met Leu Ser Ile Met Glu Lys Leu Glu Phe Phe
            900                 905                 910

Asp Phe Ser Tyr Asp Leu Asn Leu Cys Gly Leu Thr Glu Asp Pro Asp
        915                 920                 925

Leu Gln Val Ser Ala Met Gln His Gln Thr Val Leu Glu Leu Thr Glu
    930                 935                 940

Thr Gly Val Glu Ala Ala Ala Ala Ser Ala Ile Ser Val Ala Arg Thr
945                 950                 955                 960

Leu Leu Val Phe Glu Val Gln Gln Pro Phe Leu Phe Val Leu Trp Asp
                965                 970                 975

Gln Gln His Lys Phe Pro Val Phe Met Gly Arg Val Tyr Asp Pro Arg
            980                 985                 990

Ala

<210> 25
<211> 706
<212> PRT
<213> Artificial Sequence

<220>

<223> Description of Artificial Sequence: Synthetic
      polypeptide

<400> 25
Met Glu Thr Pro Ala Gln Leu Leu Phe Leu Leu Leu Leu Trp Leu Pro
1               5                   10                  15

Asp Thr Thr Gly Val Glu Glu Pro Gln Asn Leu Ile Lys Gln Asn Cys
            20                  25                  30

Glu Leu Phe Glu Gln Leu Gly Glu Tyr Lys Phe Gln Asn Ala Leu Leu
        35                  40                  45

Val Arg Tyr Thr Lys Lys Val Pro Gln Val Ser Thr Pro Thr Leu Val
    50                  55                  60

Glu Val Ser Arg Asn Leu Gly Lys Val Gly Ser Lys Cys Cys Lys His
65                  70                  75                  80

Pro Glu Ala Lys Arg Met Pro Cys Ala Glu Asp Tyr Leu Ser Val Val
                85                  90                  95

Leu Asn Gln Leu Cys Val Leu His Glu Lys Thr Pro Val Ser Asp Arg
            100                 105                 110

Val Thr Lys Cys Cys Thr Glu Ser Leu Val Asn Arg Arg Pro Cys Phe
        115                 120                 125

Ser Ala Leu Glu Val Asp Glu Thr Tyr Val Pro Lys Glu Phe Asn Ala
    130                 135                 140

Glu Thr Phe Thr Phe His Ala Asp Ile Cys Thr Leu Ser Glu Lys Glu
145                 150                 155                 160

Arg Gln Ile Lys Lys Gln Thr Ala Leu Val Glu Leu Val Lys His Lys
                165                 170                 175

Pro Lys Ala Thr Lys Glu Gln Leu Lys Ala Val Met Asp Asp Phe Ala
            180                 185                 190

Ala Phe Val Glu Lys Cys Cys Lys Ala Asp Asp Lys Glu Thr Cys Phe
        195                 200                 205

Ala Glu Glu Gly Lys Lys Leu Val Ala Ala Ser Arg Ala Ala Leu Gly
    210                 215                 220

Leu Gly Gly Gly Asn Pro Asn Ala Thr Ser Ser Ser Ser Gln Asp Pro
225                 230                 235                 240

Glu Ser Leu Gln Asp Arg Gly Glu Gly Lys Val Ala Thr Thr Val Ile
                245                 250                 255

Ser Lys Met Leu Phe Val Glu Pro Ile Leu Glu Val Ser Ser Leu Pro
            260                 265                 270

Thr Thr Asn Ser Thr Thr Asn Ser Ala Thr Lys Ile Thr Ala Asn Thr
        275                 280                 285

Thr Asp Glu Pro Thr Thr Gln Pro Thr Thr Glu Pro Thr Thr Gln Pro
    290                 295                 300

Thr Ile Gln Pro Thr Gln Pro Thr Thr Gln Leu Pro Thr Asp Ser Pro
305                 310                 315                 320

Thr Gln Pro Thr Thr Gly Ser Phe Cys Pro Gly Pro Val Thr Leu Cys
                325                 330                 335

Ser Asp Leu Glu Ser His Ser Thr Glu Ala Val Leu Gly Asp Ala Leu
            340                 345                 350

Val Asp Phe Ser Leu Lys Leu Tyr His Ala Phe Ser Ala Met Lys Lys
        355                 360                 365

Val Glu Thr Asn Met Ala Phe Ser Pro Phe Ser Ile Ala Ser Leu Leu
    370                 375                 380

Thr Gln Val Leu Leu Gly Ala Gly Glu Asn Thr Lys Thr Asn Leu Glu
385                 390                 395                 400

Ser Ile Leu Ser Tyr Pro Lys Asp Phe Thr Cys Val His Gln Ala Leu
                405                 410                 415

Lys Gly Phe Thr Thr Lys Gly Val Thr Ser Val Ser Gln Ile Phe His
            420                 425                 430

Ser Pro Asp Leu Ala Ile Arg Asp Thr Phe Val Asn Ala Ser Arg Thr
        435                 440                 445

Leu Tyr Ser Ser Ser Pro Arg Val Leu Ser Asn Asn Ser Asp Ala Asn
    450                 455                 460

Leu Glu Leu Ile Asn Thr Trp Val Ala Lys Asn Thr Asn Asn Lys Ile
465                 470                 475                 480

Ser Arg Leu Leu Asp Ser Leu Pro Ser Asp Thr Arg Leu Val Leu Leu
                485                 490                 495

Asn Ala Ile Tyr Leu Ser Ala Lys Trp Lys Thr Thr Phe Asp Pro Lys
            500                 505                 510

Lys Thr Arg Met Glu Pro Phe His Phe Lys Asn Ser Val Ile Lys Val
        515                 520                 525

Pro Met Met Asn Ser Lys Lys Tyr Pro Val Ala His Phe Ile Asp Gln
    530                 535                 540

Thr Leu Lys Ala Lys Val Gly Gln Leu Gln Leu Ser His Asn Leu Ser
545                 550                 555                 560

Leu Val Ile Leu Val Pro Gln Asn Leu Lys His Arg Leu Glu Asp Met
                565                 570                 575

Glu Gln Ala Leu Ser Pro Ser Val Phe Lys Ala Ile Met Glu Lys Leu
            580                 585                 590

Glu Met Ser Lys Phe Gln Pro Thr Leu Leu Thr Leu Pro Arg Ile Lys
        595                 600                 605

Val Thr Thr Ser Gln Asp Met Leu Ser Ile Met Glu Lys Leu Glu Phe
    610                 615                 620

Phe Asp Phe Ser Tyr Asp Leu Asn Leu Cys Gly Leu Thr Glu Asp Pro
625                 630                 635                 640

Asp Leu Gln Val Ser Ala Met Gln His Gln Thr Val Leu Glu Leu Thr
                645                 650                 655

Glu Thr Gly Val Glu Ala Ala Ala Ala Ser Ala Ile Ser Val Ala Arg
            660                 665                 670

Thr Leu Leu Val Phe Glu Val Gln Gln Pro Phe Leu Phe Val Leu Trp
        675                 680                 685

Asp Gln Gln His Lys Phe Pro Val Phe Met Gly Arg Val Tyr Asp Pro
    690                 695                 700

Arg Ala
705

<210> 26
<211> 609
<212> PRT
<213> Artificial Sequence

<220>
<223> Description of Artificial Sequence: Synthetic
      polypeptide

<400> 26
Met Glu Thr Pro Ala Gln Leu Leu Phe Leu Leu Leu Leu Trp Leu Pro
1               5                   10                  15

Asp Thr Thr Gly Val Glu Glu Pro Gln Asn Leu Ile Lys Gln Asn Cys
            20                  25                  30

Glu Leu Phe Glu Gln Leu Gly Glu Tyr Lys Phe Gln Asn Ala Leu Leu
        35                  40                  45

Val Arg Tyr Thr Lys Lys Val Pro Gln Val Ser Thr Pro Thr Leu Val
    50                  55                  60

Glu Val Ser Arg Asn Leu Gly Lys Val Gly Ser Lys Cys Cys Lys His
65                  70                  75                  80

Pro Glu Ala Lys Arg Met Pro Cys Ala Glu Asp Tyr Leu Ser Val Val
                85                  90                  95

Leu Asn Gln Leu Cys Val Leu His Glu Lys Thr Pro Val Ser Asp Arg
            100                 105                 110

Val Thr Lys Cys Cys Thr Glu Ser Leu Val Asn Arg Arg Pro Cys Phe
        115                 120                 125

Ser Ala Leu Glu Val Asp Glu Thr Tyr Val Pro Lys Glu Phe Asn Ala
    130                 135                 140

Glu Thr Phe Thr Phe His Ala Asp Ile Cys Thr Leu Ser Glu Lys Glu
145                 150                 155                 160

Arg Gln Ile Lys Lys Gln Thr Ala Leu Val Glu Leu Val Lys His Lys
                165                 170                 175

Pro Lys Ala Thr Lys Glu Gln Leu Lys Ala Val Met Asp Asp Phe Ala
            180                 185                 190

Ala Phe Val Glu Lys Cys Cys Lys Ala Asp Asp Lys Glu Thr Cys Phe
        195                 200                 205

Ala Glu Glu Gly Lys Lys Leu Val Ala Ala Ser Arg Ala Ala Leu Gly
    210                 215                 220

Leu Gly Gly Gly Gly Ser Phe Cys Pro Gly Pro Val Thr Leu Cys Ser
225                 230                 235                 240

Asp Leu Glu Ser His Ser Thr Glu Ala Val Leu Gly Asp Ala Leu Val
                245                 250                 255

Asp Phe Ser Leu Lys Leu Tyr His Ala Phe Ser Ala Met Lys Lys Val
            260                 265                 270

Glu Thr Asn Met Ala Phe Ser Pro Phe Ser Ile Ala Ser Leu Leu Thr
        275                 280                 285

Gln Val Leu Leu Gly Ala Gly Glu Asn Thr Lys Thr Asn Leu Glu Ser
    290                 295                 300

Ile Leu Ser Tyr Pro Lys Asp Phe Thr Cys Val His Gln Ala Leu Lys
305                 310                 315                 320

Gly Phe Thr Thr Lys Gly Val Thr Ser Val Ser Gln Ile Phe His Ser
                325                 330                 335

Pro Asp Leu Ala Ile Arg Asp Thr Phe Val Asn Ala Ser Arg Thr Leu
            340                 345                 350

Tyr Ser Ser Ser Pro Arg Val Leu Ser Asn Asn Ser Asp Ala Asn Leu
        355                 360                 365

Glu Leu Ile Asn Thr Trp Val Ala Lys Asn Thr Asn Asn Lys Ile Ser
    370                 375                 380

Arg Leu Leu Asp Ser Leu Pro Ser Asp Thr Arg Leu Val Leu Leu Asn
385                 390                 395                 400

Ala Ile Tyr Leu Ser Ala Lys Trp Lys Thr Thr Phe Asp Pro Lys Lys
                405                 410                 415

Thr Arg Met Glu Pro Phe His Phe Lys Asn Ser Val Ile Lys Val Pro
            420                 425                 430

Met Met Asn Ser Lys Lys Tyr Pro Val Ala His Phe Ile Asp Gln Thr
        435                 440                 445

Leu Lys Ala Lys Val Gly Gln Leu Gln Leu Ser His Asn Leu Ser Leu
    450                 455                 460

Val Ile Leu Val Pro Gln Asn Leu Lys His Arg Leu Glu Asp Met Glu
465                 470                 475                 480

Gln Ala Leu Ser Pro Ser Val Phe Lys Ala Ile Met Glu Lys Leu Glu

485                 490                 495

Met Ser Lys Phe Gln Pro Thr Leu Leu Thr Leu Pro Arg Ile Lys Val
            500                 505                 510

Thr Thr Ser Gln Asp Met Leu Ser Ile Met Glu Lys Leu Glu Phe Phe
        515                 520                 525

Asp Phe Ser Tyr Asp Leu Asn Leu Cys Gly Leu Thr Glu Asp Pro Asp
    530                 535                 540

Leu Gln Val Ser Ala Met Gln His Gln Thr Val Leu Glu Leu Thr Glu
545                 550                 555                 560

Thr Gly Val Glu Ala Ala Ala Ala Ser Ala Ile Ser Val Ala Arg Thr
                565                 570                 575

Leu Leu Val Phe Glu Val Gln Gln Pro Phe Leu Phe Val Leu Trp Asp
            580                 585                 590

Gln Gln His Lys Phe Pro Val Phe Met Gly Arg Val Tyr Asp Pro Arg
        595                 600                 605

Ala

<210> 27
<211> 10
<212> PRT
<213> Artificial Sequence

<220>
<223> Description of Artificial Sequence: Synthetic
      peptide

<400> 27
Gly Gly Gly Gly Ser Gly Gly Gly Gly Ser
1               5                   10

<210> 28
<211> 1097
<212> PRT
<213> Artificial Sequence

<220>
<223> Description of Artificial Sequence: Synthetic
      polypeptide

<400> 28
Met Lys Trp Val Thr Phe Ile Ser Leu Leu Phe Leu Phe Ser Ser Ala
1               5                   10                  15

Tyr Ser Arg Gly Val Phe Arg Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Lys Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Arg Ala Ala Leu Gly
        595                 600                 605

Leu Gly Gly Gly Gly Ser Gly Gly Gly Gly Ser Asn Pro Asn Ala Thr
    610                 615                 620

Ser Ser Ser Ser Gln Asp Pro Glu Ser Leu Gln Asp Arg Gly Glu Gly
625                 630                 635                 640

Lys Val Ala Thr Thr Val Ile Ser Lys Met Leu Phe Val Glu Pro Ile
                645                 650                 655

Leu Glu Val Ser Ser Leu Pro Thr Thr Asn Ser Thr Thr Asn Ser Ala
            660                 665                 670

Thr Lys Ile Thr Ala Asn Thr Thr Asp Glu Pro Thr Thr Gln Pro Thr
        675                 680                 685

Thr Glu Pro Thr Thr Gln Pro Thr Ile Gln Pro Thr Gln Pro Thr Thr
    690                 695                 700

Gln Leu Pro Thr Asp Ser Pro Thr Gln Pro Thr Thr Gly Ser Phe Cys
705                 710                 715                 720

Pro Gly Pro Val Thr Leu Cys Ser Asp Leu Glu Ser His Ser Thr Glu
                725                 730                 735

Ala Val Leu Gly Asp Ala Leu Val Asp Phe Ser Leu Lys Leu Tyr His
            740                 745                 750

Ala Phe Ser Ala Met Lys Lys Val Glu Thr Asn Met Ala Phe Ser Pro
        755                 760                 765

Phe Ser Ile Ala Ser Leu Leu Thr Gln Val Leu Leu Gly Ala Gly Glu

770                 775                 780

Asn Thr Lys Thr Asn Leu Glu Ser Ile Leu Ser Tyr Pro Lys Asp Phe
785                 790                 795                 800

Thr Cys Val His Gln Ala Leu Lys Gly Phe Thr Thr Lys Gly Val Thr
                805                 810                 815

Ser Val Ser Gln Ile Phe His Ser Pro Asp Leu Ala Ile Arg Asp Thr
            820                 825                 830

Phe Val Asn Ala Ser Arg Thr Leu Tyr Ser Ser Ser Pro Arg Val Leu
        835                 840                 845

Ser Asn Asn Ser Asp Ala Asn Leu Glu Leu Ile Asn Thr Trp Val Ala
    850                 855                 860

Lys Asn Thr Asn Asn Lys Ile Ser Arg Leu Leu Asp Ser Leu Pro Ser
865                 870                 875                 880

Asp Thr Arg Leu Val Leu Leu Asn Ala Ile Tyr Leu Ser Ala Lys Trp
                885                 890                 895

Lys Thr Thr Phe Asp Pro Lys Lys Thr Arg Met Glu Pro Phe His Phe
            900                 905                 910

Lys Asn Ser Val Ile Lys Val Pro Met Met Asn Ser Lys Lys Tyr Pro
        915                 920                 925

Val Ala His Phe Ile Asp Gln Thr Leu Lys Ala Lys Val Gly Gln Leu
    930                 935                 940

Gln Leu Ser His Asn Leu Ser Leu Val Ile Leu Val Pro Gln Asn Leu
945                 950                 955                 960

Lys His Arg Leu Glu Asp Met Glu Gln Ala Leu Ser Pro Ser Val Phe
                965                 970                 975

Lys Ala Ile Met Glu Lys Leu Glu Met Ser Lys Phe Gln Pro Thr Leu
            980                 985                 990

Leu Thr Leu Pro Arg Ile Lys Val  Thr Thr Ser Gln Asp  Met Leu Ser
        995                 1000                1005

Ile Met  Glu Lys Leu Glu Phe  Phe Asp Phe Ser Tyr  Asp Leu Asn
    1010                1015                1020

Leu Cys Gly Leu Thr Glu Asp Pro Asp Leu Gln Val Ser Ala Met
    1025                1030                1035

Gln His Gln Thr Val Leu Glu Leu Thr Glu Thr Gly Val Glu Ala
    1040                1045                1050

Ala Ala Ala Ser Ala Ile Ser Val Ala Arg Thr Leu Leu Val Phe
    1055                1060                1065

Glu Val Gln Gln Pro Phe Leu Phe Val Leu Trp Asp Gln Gln His
    1070                1075                1080

Lys Phe Pro Val Phe Met Gly Arg Val Tyr Asp Pro Arg Ala
    1085                1090                1095

<210> 29
<211> 1000
<212> PRT
<213> Artificial Sequence

<220>
<223> Description of Artificial Sequence: Synthetic
      polypeptide

<400> 29
Met Lys Trp Val Thr Phe Ile Ser Leu Leu Phe Leu Phe Ser Ser Ala
1               5                   10                  15

Tyr Ser Arg Gly Val Phe Arg Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val

130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Lys Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Arg Ala Ala Leu Gly
        595                 600                 605

Leu Gly Gly Gly Gly Ser Gly Gly Gly Gly Ser Gly Ser Phe Cys Pro
    610                 615                 620

Gly Pro Val Thr Leu Cys Ser Asp Leu Glu Ser His Ser Thr Glu Ala
625                 630                 635                 640

Val Leu Gly Asp Ala Leu Val Asp Phe Ser Leu Lys Leu Tyr His Ala
                645                 650                 655

Phe Ser Ala Met Lys Lys Val Glu Thr Asn Met Ala Phe Ser Pro Phe
            660                 665                 670

Ser Ile Ala Ser Leu Leu Thr Gln Val Leu Leu Gly Ala Gly Glu Asn
        675                 680                 685

Thr Lys Thr Asn Leu Glu Ser Ile Leu Ser Tyr Pro Lys Asp Phe Thr
    690                 695                 700

Cys Val His Gln Ala Leu Lys Gly Phe Thr Thr Lys Gly Val Thr Ser
705                 710                 715                 720

Val Ser Gln Ile Phe His Ser Pro Asp Leu Ala Ile Arg Asp Thr Phe
                725                 730                 735

Val Asn Ala Ser Arg Thr Leu Tyr Ser Ser Ser Pro Arg Val Leu Ser
            740                 745                 750

Asn Asn Ser Asp Ala Asn Leu Glu Leu Ile Asn Thr Trp Val Ala Lys
        755                 760                 765

Asn Thr Asn Asn Lys Ile Ser Arg Leu Leu Asp Ser Leu Pro Ser Asp
    770                 775                 780

Thr Arg Leu Val Leu Leu Asn Ala Ile Tyr Leu Ser Ala Lys Trp Lys
785                 790                 795                 800

Thr Thr Phe Asp Pro Lys Lys Thr Arg Met Glu Pro Phe His Phe Lys
                805                 810                 815

Asn Ser Val Ile Lys Val Pro Met Met Asn Ser Lys Lys Tyr Pro Val
            820                 825                 830

Ala His Phe Ile Asp Gln Thr Leu Lys Ala Lys Val Gly Gln Leu Gln
        835                 840                 845

Leu Ser His Asn Leu Ser Leu Val Ile Leu Val Pro Gln Asn Leu Lys
    850                 855                 860

His Arg Leu Glu Asp Met Glu Gln Ala Leu Ser Pro Ser Val Phe Lys
865                 870                 875                 880

Ala Ile Met Glu Lys Leu Glu Met Ser Lys Phe Gln Pro Thr Leu Leu
                885                 890                 895

Thr Leu Pro Arg Ile Lys Val Thr Thr Ser Gln Asp Met Leu Ser Ile
            900                 905                 910

Met Glu Lys Leu Glu Phe Phe Asp Phe Ser Tyr Asp Leu Asn Leu Cys
        915                 920                 925

Gly Leu Thr Glu Asp Pro Asp Leu Gln Val Ser Ala Met Gln His Gln
    930                 935                 940

Thr Val Leu Glu Leu Thr Glu Thr Gly Val Glu Ala Ala Ala Ala Ser
945                 950                 955                 960

Ala Ile Ser Val Ala Arg Thr Leu Leu Val Phe Glu Val Gln Gln Pro
                965                 970                 975

Phe Leu Phe Val Leu Trp Asp Gln Gln His Lys Phe Pro Val Phe Met
            980                 985                 990

Gly Arg Val Tyr Asp Pro Arg Ala
        995                 1000

<210> 30
<211> 713
<212> PRT
<213> Artificial Sequence

<220>
<223> Description of Artificial Sequence: Synthetic
      polypeptide

<400> 30
Met Glu Thr Pro Ala Gln Leu Leu Phe Leu Leu Leu Leu Trp Leu Pro
1               5                   10                  15

Asp Thr Thr Gly Val Glu Glu Pro Gln Asn Leu Ile Lys Gln Asn Cys
            20                  25                  30

Glu Leu Phe Glu Gln Leu Gly Glu Tyr Lys Phe Gln Asn Ala Leu Leu
        35                  40                  45

Val Arg Tyr Thr Lys Lys Val Pro Gln Val Ser Thr Pro Thr Leu Val
    50                  55                  60

Glu Val Ser Arg Asn Leu Gly Lys Val Gly Ser Lys Cys Cys Lys His
65                  70                  75                  80

Pro Glu Ala Lys Arg Met Pro Cys Ala Glu Asp Tyr Leu Ser Val Val
                85                  90                  95

Leu Asn Gln Leu Cys Val Leu His Glu Lys Thr Pro Val Ser Asp Arg
            100                 105                 110

Val Thr Lys Cys Cys Thr Glu Ser Leu Val Asn Arg Arg Pro Cys Phe
        115                 120                 125

Ser Ala Leu Glu Val Asp Glu Thr Tyr Val Pro Lys Glu Phe Asn Ala
    130                 135                 140

Glu Thr Phe Thr Phe His Ala Asp Ile Cys Thr Leu Ser Glu Lys Glu
145                 150                 155                 160

Arg Gln Ile Lys Lys Gln Thr Ala Leu Val Glu Leu Val Lys His Lys
                165                 170                 175

Pro Lys Ala Thr Lys Glu Gln Leu Lys Ala Val Met Asp Asp Phe Ala
            180                 185                 190

Ala Phe Val Glu Lys Cys Cys Lys Ala Asp Asp Lys Glu Thr Cys Phe
        195                 200                 205

Ala Glu Glu Gly Lys Lys Leu Val Ala Ala Ser Arg Ala Ala Leu Gly
    210                 215                 220

Leu Gly Gly Gly Gly Ser Gly Gly Gly Gly Ser Asn Pro Asn Ala Thr
225                 230                 235                 240

Ser Ser Ser Ser Gln Asp Pro Glu Ser Leu Gln Asp Arg Gly Glu Gly
                245                 250                 255

Lys Val Ala Thr Thr Val Ile Ser Lys Met Leu Phe Val Glu Pro Ile
            260                 265                 270

Leu Glu Val Ser Ser Leu Pro Thr Thr Asn Ser Thr Thr Asn Ser Ala
        275                 280                 285

Thr Lys Ile Thr Ala Asn Thr Thr Asp Glu Pro Thr Thr Gln Pro Thr
    290                 295                 300

Thr Glu Pro Thr Thr Gln Pro Thr Ile Gln Pro Thr Gln Pro Thr Thr
305                 310                 315                 320

Gln Leu Pro Thr Asp Ser Pro Thr Gln Pro Thr Thr Gly Ser Phe Cys
                325                 330                 335

Pro Gly Pro Val Thr Leu Cys Ser Asp Leu Glu Ser His Ser Thr Glu
            340                 345                 350

Ala Val Leu Gly Asp Ala Leu Val Asp Phe Ser Leu Lys Leu Tyr His
        355                 360                 365

Ala Phe Ser Ala Met Lys Lys Val Glu Thr Asn Met Ala Phe Ser Pro
    370                 375                 380

Phe Ser Ile Ala Ser Leu Leu Thr Gln Val Leu Leu Gly Ala Gly Glu
385                 390                 395                 400

Asn Thr Lys Thr Asn Leu Glu Ser Ile Leu Ser Tyr Pro Lys Asp Phe
                405                 410                 415

Thr Cys Val His Gln Ala Leu Lys Gly Phe Thr Thr Lys Gly Val Thr
            420                 425                 430

Ser Val Ser Gln Ile Phe His Ser Pro Asp Leu Ala Ile Arg Asp Thr
        435                 440                 445

Phe Val Asn Ala Ser Arg Thr Leu Tyr Ser Ser Ser Pro Arg Val Leu
    450                 455                 460

Ser Asn Asn Ser Asp Ala Asn Leu Glu Leu Ile Asn Thr Trp Val Ala
465                 470                 475                 480

Lys Asn Thr Asn Asn Lys Ile Ser Arg Leu Leu Asp Ser Leu Pro Ser
                485                 490                 495

Asp Thr Arg Leu Val Leu Leu Asn Ala Ile Tyr Leu Ser Ala Lys Trp
            500                 505                 510

Lys Thr Thr Phe Asp Pro Lys Lys Thr Arg Met Glu Pro Phe His Phe
        515                 520                 525

Lys Asn Ser Val Ile Lys Val Pro Met Met Asn Ser Lys Lys Tyr Pro
    530                 535                 540

Val Ala His Phe Ile Asp Gln Thr Leu Lys Ala Lys Val Gly Gln Leu
545                 550                 555                 560

Gln Leu Ser His Asn Leu Ser Leu Val Ile Leu Val Pro Gln Asn Leu
                565                 570                 575

Lys His Arg Leu Glu Asp Met Glu Gln Ala Leu Ser Pro Ser Val Phe
            580                 585                 590

Lys Ala Ile Met Glu Lys Leu Glu Met Ser Lys Phe Gln Pro Thr Leu

595                 600                 605

Leu Thr Leu Pro Arg Ile Lys Val Thr Thr Ser Gln Asp Met Leu Ser
    610                 615                 620

Ile Met Glu Lys Leu Glu Phe Phe Asp Phe Ser Tyr Asp Leu Asn Leu
625                 630                 635                 640

Cys Gly Leu Thr Glu Asp Pro Asp Leu Gln Val Ser Ala Met Gln His
                645                 650                 655

Gln Thr Val Leu Glu Leu Thr Glu Thr Gly Val Glu Ala Ala Ala Ala
            660                 665                 670

Ser Ala Ile Ser Val Ala Arg Thr Leu Leu Val Phe Glu Val Gln Gln
        675                 680                 685

Pro Phe Leu Phe Val Leu Trp Asp Gln Gln His Lys Phe Pro Val Phe
    690                 695                 700

Met Gly Arg Val Tyr Asp Pro Arg Ala
705                 710

<210> 31
<211> 616
<212> PRT
<213> Artificial Sequence

<220>
<223> Description of Artificial Sequence: Synthetic
      polypeptide

<400> 31
Met Glu Thr Pro Ala Gln Leu Leu Phe Leu Leu Leu Leu Trp Leu Pro
1               5                   10                  15

Asp Thr Thr Gly Val Glu Glu Pro Gln Asn Leu Ile Lys Gln Asn Cys
            20                  25                  30

Glu Leu Phe Glu Gln Leu Gly Glu Tyr Lys Phe Gln Asn Ala Leu Leu
        35                  40                  45

Val Arg Tyr Thr Lys Lys Val Pro Gln Val Ser Thr Pro Thr Leu Val
    50                  55                  60

Glu Val Ser Arg Asn Leu Gly Lys Val Gly Ser Lys Cys Cys Lys His
65                  70                  75                  80

Pro Glu Ala Lys Arg Met Pro Cys Ala Glu Asp Tyr Leu Ser Val Val
                85                  90                  95

Leu Asn Gln Leu Cys Val Leu His Glu Lys Thr Pro Val Ser Asp Arg
            100                 105                 110

Val Thr Lys Cys Cys Thr Glu Ser Leu Val Asn Arg Arg Pro Cys Phe
        115                 120                 125

Ser Ala Leu Glu Val Asp Glu Thr Tyr Val Pro Lys Glu Phe Asn Ala
    130                 135                 140

Glu Thr Phe Thr Phe His Ala Asp Ile Cys Thr Leu Ser Glu Lys Glu
145                 150                 155                 160

Arg Gln Ile Lys Lys Gln Thr Ala Leu Val Glu Leu Val Lys His Lys
                165                 170                 175

Pro Lys Ala Thr Lys Glu Gln Leu Lys Ala Val Met Asp Asp Phe Ala
            180                 185                 190

Ala Phe Val Glu Lys Cys Cys Lys Ala Asp Asp Lys Glu Thr Cys Phe
        195                 200                 205

Ala Glu Glu Gly Lys Lys Leu Val Ala Ala Ser Arg Ala Ala Leu Gly
    210                 215                 220

Leu Gly Gly Gly Gly Ser Gly Gly Gly Gly Ser Gly Ser Phe Cys Pro
225                 230                 235                 240

Gly Pro Val Thr Leu Cys Ser Asp Leu Glu Ser His Ser Thr Glu Ala
                245                 250                 255

Val Leu Gly Asp Ala Leu Val Asp Phe Ser Leu Lys Leu Tyr His Ala
            260                 265                 270

Phe Ser Ala Met Lys Lys Val Glu Thr Asn Met Ala Phe Ser Pro Phe
        275                 280                 285

Ser Ile Ala Ser Leu Leu Thr Gln Val Leu Leu Gly Ala Gly Glu Asn
    290                 295                 300

Thr Lys Thr Asn Leu Glu Ser Ile Leu Ser Tyr Pro Lys Asp Phe Thr
305                 310                 315                 320

Cys Val His Gln Ala Leu Lys Gly Phe Thr Thr Lys Gly Val Thr Ser
                325                 330                 335

Val Ser Gln Ile Phe His Ser Pro Asp Leu Ala Ile Arg Asp Thr Phe

340                 345                 350

Val Asn Ala Ser Arg Thr Leu Tyr Ser Ser Ser Pro Arg Val Leu Ser
    355                 360                 365

Asn Asn Ser Asp Ala Asn Leu Glu Leu Ile Asn Thr Trp Val Ala Lys
    370                 375                 380

Asn Thr Asn Asn Lys Ile Ser Arg Leu Leu Asp Ser Leu Pro Ser Asp
385                 390                 395                 400

Thr Arg Leu Val Leu Leu Asn Ala Ile Tyr Leu Ser Ala Lys Trp Lys
                405                 410                 415

Thr Thr Phe Asp Pro Lys Lys Thr Arg Met Glu Pro Phe His Phe Lys
            420                 425                 430

Asn Ser Val Ile Lys Val Pro Met Met Asn Ser Lys Lys Tyr Pro Val
    435                 440                 445

Ala His Phe Ile Asp Gln Thr Leu Lys Ala Lys Val Gly Gln Leu Gln
    450                 455                 460

Leu Ser His Asn Leu Ser Leu Val Ile Leu Val Pro Gln Asn Leu Lys
465                 470                 475                 480

His Arg Leu Glu Asp Met Glu Gln Ala Leu Ser Pro Ser Val Phe Lys
                485                 490                 495

Ala Ile Met Glu Lys Leu Glu Met Ser Lys Phe Gln Pro Thr Leu Leu
            500                 505                 510

Thr Leu Pro Arg Ile Lys Val Thr Thr Ser Gln Asp Met Leu Ser Ile
    515                 520                 525

Met Glu Lys Leu Glu Phe Phe Asp Phe Ser Tyr Asp Leu Asn Leu Cys
    530                 535                 540

Gly Leu Thr Glu Asp Pro Asp Leu Gln Val Ser Ala Met Gln His Gln
545                 550                 555                 560

Thr Val Leu Glu Leu Thr Glu Thr Gly Val Glu Ala Ala Ala Ala Ser
                565                 570                 575

Ala Ile Ser Val Ala Arg Thr Leu Leu Val Phe Glu Val Gln Gln Pro
            580                 585                 590

Phe Leu Phe Val Leu Trp Asp Gln Gln His Lys Phe Pro Val Phe Met
        595                 600                 605

Gly Arg Val Tyr Asp Pro Arg Ala
    610                 615

<210> 32
<211> 707
<212> PRT
<213> Artificial Sequence

<220>
<223> Description of Artificial Sequence: Synthetic
      polypeptide

<400> 32
Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu

            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Asn Pro Asn Ala Thr Ser Ser Ser Ser Gln Asp
225                 230                 235                 240

Pro Glu Ser Leu Gln Asp Arg Gly Glu Gly Lys Val Ala Thr Thr Val
                245                 250                 255

Ile Ser Lys Met Leu Phe Val Glu Pro Ile Leu Glu Val Ser Ser Leu
            260                 265                 270

Pro Thr Thr Asn Ser Thr Thr Asn Ser Ala Thr Lys Ile Thr Ala Asn
        275                 280                 285

Thr Thr Asp Glu Pro Thr Thr Gln Pro Thr Thr Glu Pro Thr Thr Gln
    290                 295                 300

Pro Thr Ile Gln Pro Thr Gln Pro Thr Thr Gln Leu Pro Thr Asp Ser
305                 310                 315                 320

Pro Thr Gln Pro Thr Thr Gly Ser Phe Cys Pro Gly Pro Val Thr Leu
                325                 330                 335

Cys Ser Asp Leu Glu Ser His Ser Thr Glu Ala Val Leu Gly Asp Ala
            340                 345                 350

Leu Val Asp Phe Ser Leu Lys Leu Tyr His Ala Phe Ser Ala Met Lys
        355                 360                 365

Lys Val Glu Thr Asn Met Ala Phe Ser Pro Phe Ser Ile Ala Ser Leu
    370                 375                 380

Leu Thr Gln Val Leu Leu Gly Ala Gly Glu Asn Thr Lys Thr Asn Leu
385                 390                 395                 400

Glu Ser Ile Leu Ser Tyr Pro Lys Asp Phe Thr Cys Val His Gln Ala
                405                 410                 415

Leu Lys Gly Phe Thr Thr Lys Gly Val Thr Ser Val Ser Gln Ile Phe
            420                 425                 430

His Ser Pro Asp Leu Ala Ile Arg Asp Thr Phe Val Asn Ala Ser Arg
        435                 440                 445

Thr Leu Tyr Ser Ser Ser Pro Arg Val Leu Ser Asn Asn Ser Asp Ala
    450                 455                 460

Asn Leu Glu Leu Ile Asn Thr Trp Val Ala Lys Asn Thr Asn Asn Lys
465                 470                 475                 480

Ile Ser Arg Leu Leu Asp Ser Leu Pro Ser Asp Thr Arg Leu Val Leu
                485                 490                 495

Leu Asn Ala Ile Tyr Leu Ser Ala Lys Trp Lys Thr Thr Phe Asp Pro
            500                 505                 510

Lys Lys Thr Arg Met Glu Pro Phe His Phe Lys Asn Ser Val Ile Lys
        515                 520                 525

Val Pro Met Met Asn Ser Lys Lys Tyr Pro Val Ala His Phe Ile Asp
    530                 535                 540

Gln Thr Leu Lys Ala Lys Val Gly Gln Leu Gln Leu Ser His Asn Leu
545                 550                 555                 560

Ser Leu Val Ile Leu Val Pro Gln Asn Leu Lys His Arg Leu Glu Asp
                565                 570                 575

Met Glu Gln Ala Leu Ser Pro Ser Val Phe Lys Ala Ile Met Glu Lys
            580                 585                 590

Leu Glu Met Ser Lys Phe Gln Pro Thr Leu Leu Thr Leu Pro Arg Ile
        595                 600                 605

Lys Val Thr Thr Ser Gln Asp Met Leu Ser Ile Met Glu Lys Leu Glu
    610                 615                 620

Phe Phe Asp Phe Ser Tyr Asp Leu Asn Leu Cys Gly Leu Thr Glu Asp
625                 630                 635                 640

Pro Asp Leu Gln Val Ser Ala Met Gln His Gln Thr Val Leu Glu Leu
                645                 650                 655

Thr Glu Thr Gly Val Glu Ala Ala Ala Ala Ser Ala Ile Ser Val Ala
            660                 665                 670

Arg Thr Leu Leu Val Phe Glu Val Gln Gln Pro Phe Leu Phe Val Leu
        675                 680                 685

Trp Asp Gln Gln His Lys Phe Pro Val Phe Met Gly Arg Val Tyr Asp
    690                 695                 700

Pro Arg Ala
705

<210> 33
<211> 610
<212> PRT
<213> Artificial Sequence

<220>
<223> Description of Artificial Sequence: Synthetic
      polypeptide

<400> 33
Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
        180                 185                 190

Thr Val Asp Lys Ser Arg Trp Gln Glu Gly Asn Val Phe Ser Cys Ser
    195                 200                 205

Val Met His Glu Ala Leu His Asn His Tyr Thr Gln Lys Ser Leu Ser
210                 215                 220

Leu Ser Leu Gly Lys Gly Ser Phe Cys Pro Gly Pro Val Thr Leu Cys
225                 230                 235                 240

Ser Asp Leu Glu Ser His Ser Thr Glu Ala Val Leu Gly Asp Ala Leu
                245                 250                 255

Val Asp Phe Ser Leu Lys Leu Tyr His Ala Phe Ser Ala Met Lys Lys
            260                 265                 270

Val Glu Thr Asn Met Ala Phe Ser Pro Phe Ser Ile Ala Ser Leu Leu
        275                 280                 285

Thr Gln Val Leu Leu Gly Ala Gly Glu Asn Thr Lys Thr Asn Leu Glu
    290                 295                 300

Ser Ile Leu Ser Tyr Pro Lys Asp Phe Thr Cys Val His Gln Ala Leu
305                 310                 315                 320

Lys Gly Phe Thr Thr Lys Gly Val Thr Ser Val Ser Gln Ile Phe His
                325                 330                 335

Ser Pro Asp Leu Ala Ile Arg Asp Thr Phe Val Asn Ala Ser Arg Thr
            340                 345                 350

Leu Tyr Ser Ser Ser Pro Arg Val Leu Ser Asn Asn Ser Asp Ala Asn
        355                 360                 365

Leu Glu Leu Ile Asn Thr Trp Val Ala Lys Asn Thr Asn Asn Lys Ile
    370                 375                 380

Ser Arg Leu Leu Asp Ser Leu Pro Ser Asp Thr Arg Leu Val Leu Leu
385                 390                 395                 400

Asn Ala Ile Tyr Leu Ser Ala Lys Trp Lys Thr Thr Phe Asp Pro Lys
                405                 410                 415

Lys Thr Arg Met Glu Pro Phe His Phe Lys Asn Ser Val Ile Lys Val
            420                 425                 430

Pro Met Met Asn Ser Lys Lys Tyr Pro Val Ala His Phe Ile Asp Gln
        435                 440                 445

Thr Leu Lys Ala Lys Val Gly Gln Leu Gln Leu Ser His Asn Leu Ser
    450                 455                 460

Leu Val Ile Leu Val Pro Gln Asn Leu Lys His Arg Leu Glu Asp Met
465                 470                 475                 480

Glu Gln Ala Leu Ser Pro Ser Val Phe Lys Ala Ile Met Glu Lys Leu
                485                 490                 495

Glu Met Ser Lys Phe Gln Pro Thr Leu Leu Thr Leu Pro Arg Ile Lys
            500                 505                 510

Val Thr Thr Ser Gln Asp Met Leu Ser Ile Met Glu Lys Leu Glu Phe
        515                 520                 525

Phe Asp Phe Ser Tyr Asp Leu Asn Leu Cys Gly Leu Thr Glu Asp Pro
    530                 535                 540

Asp Leu Gln Val Ser Ala Met Gln His Gln Thr Val Leu Glu Leu Thr
545                 550                 555                 560

Glu Thr Gly Val Glu Ala Ala Ala Ala Ser Ala Ile Ser Val Ala Arg
                565                 570                 575

Thr Leu Leu Val Phe Glu Val Gln Gln Pro Phe Leu Phe Val Leu Trp
            580                 585                 590

Asp Gln Gln His Lys Phe Pro Val Phe Met Gly Arg Val Tyr Asp Pro
        595                 600                 605

Arg Ala
    610

<210> 34
<211> 21
<212> PRT
<213> Artificial Sequence

<220>
<223> Description of Artificial Sequence: Synthetic
      peptide

<400> 34
Gly Ala Pro Gly Gly Gly Gly Gly Ala Ala Ala Ala Ala Gly Gly Gly
1               5                   10                  15

Gly Gly Gly Ala Pro
            20

<210> 35
<211> 39
<212> PRT
<213> Artificial Sequence

<220>
<223> Description of Artificial Sequence: Synthetic
      polypeptide

<400> 35
Gly Ala Pro Gly Gly Gly Gly Gly Ala Ala Ala Ala Ala Gly Gly Gly
1               5                   10                  15

Gly Gly Gly Ala Pro Gly Gly Gly Gly Gly Ala Ala Ala Ala Ala Gly
            20                  25                  30

Gly Gly Gly Gly Gly Ala Pro
        35

<210> 36
<211> 57
<212> PRT
<213> Artificial Sequence

<220>
<223> Description of Artificial Sequence: Synthetic
      polypeptide

<400> 36
Gly Ala Pro Gly Gly Gly Gly Gly Ala Ala Ala Ala Ala Gly Gly Gly
1               5                   10                  15

Gly Gly Gly Ala Pro Gly Gly Gly Gly Gly Ala Ala Ala Ala Ala Gly
            20                  25                  30

Gly Gly Gly Gly Gly Ala Pro Gly Gly Gly Gly Gly Ala Ala Ala Ala
        35                  40                  45

Ala Gly Gly Gly Gly Gly Gly Ala Pro
    50                  55

<210> 37
<211> 9
<212> PRT
<213> Artificial Sequence

<220>
<223> Description of Artificial Sequence: Synthetic
      peptide

<400> 37
Ala Leu Glu Val Leu Phe Gln Gly Pro

1               5

<210> 38
<211> 5
<212> PRT
<213> Artificial Sequence

<220>
<223> Description of Artificial Sequence: Synthetic
      peptide

<400> 38
Gly Gly Gly Gly Ser
1               5